# EUROPEAN PATENT APPLICATION

(11) **EP 4 792 883 A2**
(43) Date of publication of application: **19.08.2026**
(21) Application number: 26179230.3
(22) Date of filing: 08.09.2023
(51) Int. Cl.: A61P 21/00

(54) **PYRIDAZINONE COMPOSITIONS FOR THE TREATMENT OF NEUROMUSCULAR CONDITIONS**

(30) Priority: 09.09.2022 US 202263375180 P; 12.09.2022 US 202263375366 P; 26.06.2023 US 202363510348 P; 01.09.2023 US 202363580334 P
(62) Document number(s) of the earlier application(s) in accordance with Art. 76 EPC: 23783258.9 / 4 583 879
(71) Applicant: EDGEWISE THERAPEUTICS, INC., Boulder, CO 80301 (US)
(72) Inventor: DONOVAN, Joanne, Boulder, 80301 (US); COLLINS, Samuel Anthony, Boulder, 80301 (US); SILVERMAN, Jeffrey Alan, Boulder, 80301 (US); RUSSELL, Alan James, Boulder, 80301 (US)
(74) Representative: J A Kemp LLP

(57) **Abstract**

Treatments of neuromuscular diseases, such as Duchenne Muscular Dystrophy (DMD), Becker Muscular Dystrophy (BMD), Limb-Girdle Muscular Dystrophy (LGMD), and McArdle's disease, with compositions comprising substituted pyridazinone compounds are described herein.

## Description

### CROSS-REFERENCE

This application claims the benefit of US Provisional Application Serial Number 63/375,180 filed on September 9, 2022; US Provisional Application Serial Number 63/375,366 filed on September 12, 2022; US Provisional Application Serial Number 63/510,348 filed on June 26, 2023; and US Provisional Application Serial Number 63/580,334 filed on September 1, 2023, the entirety of which is hereby incorporated by reference herein.

### BACKGROUND OF THE INVENTION

Skeletal muscle is the largest organ system in the human body, serving two primary purposes. The first is force production to enable muscle contraction, locomotion, and postural maintenance; the second is glucose, fatty acid, and amino acid metabolism. The contraction of skeletal muscle during every-day activity and exercise is naturally connected to muscle stress, breakdown and remodeling which is important for muscle adaptation. In individuals with neuromuscular conditions, such as Duchenne Muscular Dystrophy (DMD), Becker Muscular Dystrophy (BMD), Limb-girdle muscular dystrophies (LGMD), and McArdle's disease, muscle contractions lead to continued rounds of amplified muscle breakdown that the body struggles to repair. Eventually, as patients age, a pathophysiological process emerges that leads to excess inflammation, fibrosis, and fatty deposit accumulation in the muscle, portending a steep decline in physical function and contribution to mortality. One potential treatment to treating such neuromuscular conditions is to administer a compound that reduces skeletal muscle contractions. However, reductions in skeletal contractivity can also lead to muscle weakness and similiary reduce a patient's physical abilities. There remains a need for treatments that reduce muscle breakdown in patients with neuromuscular conditions while preserving or improving the patient's muscle strength.

### SUMMARY OF THE INVENTION

Described herein is a method of treating a neuromuscular condition, comprising administering to a subject in need thereof a dose of 1 mg to 40 mg per day of a compound of Formula (I): 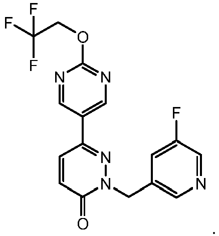 In some embodiments, the method comprises administering to the subject a dose of 15 mg to 40 mg per day of the compound of Formula (I). In some embodiments, the method comprises administering to the subject a dose of 15 mg, 20 mg, 25 mg, or 30 mg per day of the compound of Formula (I). In some embodiments, the method comprises administering to the subject a dose of 20 mg per day of the compound of Formula (I). In some embodiments, the method comprises administering to the subject a dose of 1 mg to 20 mg per day of the compound of Formula (I). In some embodiments, the method comprises administering to the subject a dose of 1 mg, 2 mg, 2.5 mg, 5 mg, 7.5 mg, 10 mg, 12.5 mg, 15 mg, or 20 mg per day of the compound of Formula (I). In some embodiments, the method comprises administering to the subject a dose of 1 mg per day of the compound of Formula (I). In some embodiments, the method comprises administering to the subject a dose of 2 mg per day of the compound of Formula (I). In some embodiments, the method comprises administering to the subject a dose of 2.5 mg per day of the compound of Formula (I). In some embodiments, the method comprises administering to the subject a dose of 5 mg per day of the compound of Formula (I). In some embodiments, the method comprises administering to the subject a dose of 7.5 mg per day of the compound of Formula (I). In some embodiments, the method comprises administering to the subject a dose of 10 mg per day of the compound of Formula (I). In some embodiments, the method comprises administering to the subject a dose of 15 mg per day of the compound of Formula (I). In some embodiments, the method comprises administering to the subject a dose of 20 mg per day of the compound of Formula (I). In some embodiments, the subject is over 18 years old. In some embodiments, the method comprises administering to the subject a dose of 10 mg per day of the compound of Formula (I). In some embodiments, the method comprises administering to the subject a dose of 15 mg per day of the compound of Formula (I). In some embodiments, the method comprises administering to the subject a dose of 20 mg per day of the compound of Formula (I). In some embodiments, the subject is 18 years or younger. In some embodiments, the method comprises administering to the subject a dose of 10 mg per day of the compound of Formula (I). In some embodiments, the method comprises administering to the subject a dose of 7.5 mg per day of the compound of Formula (I). In some embodiments, the method comprises administering to the subject a dose of 5 mg per day of the compound of Formula (I). In some embodiments, the method comprises administering to the subject a dose of 2.5 mg per day of the compound of Formula (I). In some embodiments, the method comprises administering to the subject a dose of 2 mg per day of the compound of Formula (I). In some embodiments, the method comprises administering to the subject a dose of 1 mg per day of the compound of Formula (I). In some embodiments, the method comprises administering of a first dose of said muscle fast myosin (Type II) inhibitor and one or more subsequent doses of said muscle fast myosin (Type II) inhibitor, and optionally wherein the one or more subsequent doses are higher than the first dose. In some embodiments, the method comprises administering maintains a North Star Ambulatory Assessment (NSAA) score or increases the NSAA score of the subject relative to a pre-treatment NSAA score. In some embodiments, the method maintains or improves one or more of the following functional measures of the subject: max elbow flexion strength relative to a pre-treatment max elbow flexion strength; max knee extension strength relative to a pre-treatment max knee extension strength; 10 meter walk/run velocity relative to a pre-treatment 10 meter walk/run velocity; 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value. In some embodiments, the method maintains a creatine kinase activity level or reduces the creatine kinase activity level of the subject relative to a pre-treatment creatine kinase activity level by 10% or more. In some embodiments, the method maintains a creatine kinase activity level or increases the creatine kinase activity level of the subject relative to a pre-treatment creatine kinase activity level by 10% or more. In some embodiments, the method maintains a Dual Energy x-Ray absorptiometry (DXA) % lean mass of the subject or increase the DXA % lean mass of the subject relative to a pre-treatment DXA % lean mass by 5% or more. In some embodiments, the method maintains a Fast Skeletal Muscle Troponin I (TNNI2) concentration of the subject or reduce the TNNI2 concentration of the subject relative to a pre-treatment TNNI2 concentration by 10% or more, and optionally wherein the TNNI2 concentration is inferred from a SOMAscan level. In some embodiments, said administering decreases protein concentration of one or more muscle injury biomarkers in the subject relative to a pre-treatment protein concentration of the one or more muscle injury biomarkers by 10% or more. In some embodiments, the one or more muscle injury biomarkers comprise ACTN2, MYOM2, MYBPC1, PDLIM3, MYL3, TNNI2, MYL11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof. In some embodiments, said administering decreases protein concentration of one or more pro-inflammatory proteins in the subject relative to a pre-treatment protein concentration of the one or more pro-inflammatory proteins by 10% or more. In some embodiments, the one or more pro-inflammatory proteins comprise CCL22, CCL5, CXCL10, FGG, IFN-γ, IL3, PPBP, PF4, or a combination thereof. In some embodiments, said administering increases protein concentration of one or more anti-inflammatory proteins in the subject relative to a pre-treatment protein concentration of the one or more anti-inflammatory proteins by 10% or more. In some embodiments, the one or more anti-inflammatory proteins comprise IL10, IL13, IL22, IL37, IL4, or a combination thereof. In some embodiments, the neuromuscular condition is selected from Becker Muscular Dystrophy, Duchenne Muscular Dystrophy, Limb-Girdle Muscular Dystrophy and McArdle Disease. In some embodiments, the neuromuscular condition is Becker Muscular Dystrophy. In some embodiments, the neuromuscular condition is Duchenne Muscular Dystrophy. In some embodiments, the administering comprises administering the compound of Formula (I) for 2 months or more, 3 months or more, 4 months or more, 5 months or more, 6 months or more, or 7 months or more. In some embodiments, the administering comprises administering the compound of Formula (I) for 4 months or more, 5 months or more, 6 months or more, or 7 months or more. In some embodiments, the administering comprises administering the compound of Formula (I) for 6 months or more. In some embodiments, prior to said administering the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

Described herein is a method of treating a neuromuscular condition, comprising administering to the subject a muscle fast myosin (Type II) inhibitor in an amount sufficient to maintain a North Star Ambulatory Assessment (NSAA) score or increase the NSAA score of the subject relative to a pre-treatment NSAA score.

Described herein is a method of treating a neuromuscular condition, comprising administering to a subject a muscle fast myosin (Type II)muscle fast myosin (Type II) inhibitor in an amount sufficient to maintain or improve one or more of the following functional measures of the subject: 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value.

Described herein is a method of treating a neuromuscular condition, comprising administering to the subject a muscle fast myosin (Type II)muscle fast myosin (Type II) inhibitor in an amount sufficient to maintain the creatine kinase activity level or reduce the creatine kinase activity level of the subject relative to a pre-treatment creatine kinase activity level by 10% or more.

Described herein is a method of treating a neuromuscular condition, comprising administering to the subject a muscle fast myosin (Type II)muscle fast myosin (Type II) inhibitor in an amount sufficient to maintain the serum creatinine level or increase the serum creatinine level of the subject relative to a pre-treatment serum creatinine level by 10% or more.

Described herein is a method of treating a neuromuscular condition, comprising administering to the subject a muscle fast myosin (Type II)muscle fast myosin (Type II) inhibitor in an amount sufficient to maintain the Dual Energy x-Ray absorptiometry (DXA) % lean mass of the subject or increase the DXA % lean mass of the subject relative to a pre-treatment DXA % lean mass by 5% or more.

Described herein is a method of treating a neuromuscular condition, comprising administering to the subject a muscle fast myosin (Type II)muscle fast myosin (Type II) inhibitor in an amount sufficient to maintain the Fast Skeletal Muscle Troponin I (TNNI2) concentration of the subject or reduce the TNNI2 concentration of the subject relative to a pre-treatment TNNI2 concentration by 10% or more.

Described herein is a method of inhibiting muscle fast myosin (Type II)muscle fast myosin (Type II) in a subject in need thereof, comprising selecting a subject with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, -0.5 to -3, or -1 to -4; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater; and administering a muscle fast myosin (Type II)muscle fast myosin (Type II) inhibitor to the subject.

Described herein is a unit dose for oral administration, comprising a compound of Formula (I) 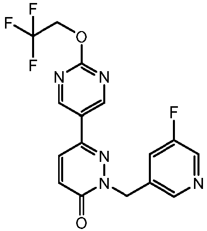 in an amount from about 1 mg to about 20 mg, and a pharmaceutically acceptable excipient.

Described herein is a kit, comprising an oral pharmaceutical composition and instructions for administration to a subject in need thereof, wherein the oral pharmaceutical composition comprises a compound of Formula (I): 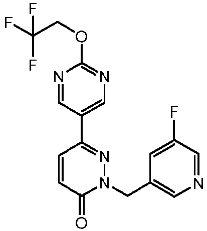 and a pharmaceutically acceptable excipient.

Described herein is a method of treating a neuromuscular condition, comprising administering to the subject a compound of Formula (I): 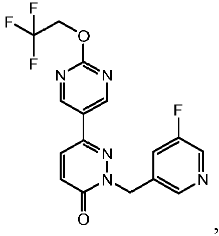 in an amount sufficient to maintain an AUC₂₄ of the subject of 1000 ng/mL or greater.

Described herein is a method of treating a neuromuscular condition comprising administering to a subject a muscle fast myosin (Type II)muscle fast myosin (Type II) inhibitor in an amount sufficient to maintain a myoglobin level or reduce a myoglobin level of the subject relative to a pre-treatment myoglobin level by 10% or more.

Described herein is a method of reducing a pain associated with a neuromuscular condition comprising administering to a subject in need thereof a muscle fast myosin (Type II)muscle fast myosin (Type II) inhibitor.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

Patent publications WO2020097265, WO2020097258, WO2020097266, WO2021231572, WO2021231572, WO2021231546, WO2021231630, WO2021231615 and PCT Application PCT/US2023/021739 are herein incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG.** 1 depicts results from a study of BMD patients showing the progression of the disease as measured by the NSAA.
FIG. 2 depicts the NSAA scores of BMD patients after four months of Compound 1 dosing.
FIG. 3 depicts baseline characteristics for BMD patients in comparison to age normative values.
**FIG.** 4 depicts plasma levels of Compound 1 and step count of BMD patients after four months of dosing. Mean AUC for the Compound 1 Plasma PK levels were 2,293 ng/mL, 1,454 ng/mL, and 2,299 ng/mL for the 20 mg, 10 mg, and 15 mg doses respectively.
**FIG.** 5 depicts measurements of creatine kinase activity and Fast Skeletal Troponin I (TNNI2) levels in BMD patients following the administration of Compound 1 in comparison to baseline measurements.
**FIG.** 6 depicts NSAA scores of patients at two and four months following the initial administration of Compound 1 in comparison to baseline measurements.
**FIG.** 7 depicts increases in functional measures such; walk/run velocity, stair climb velocity, and grip strength in BMD patients at two and four months following the initial administration of Compound 1 in comparison to baseline measurements.
**FIG.** 8 depicts a decrease in the self-reported pain score in BMD patients at two and four months following the initial administration of Compound 1.
**FIGs. 9A****,** **9B****,** and **9C** depict a North Star Ambulatory Assessment.

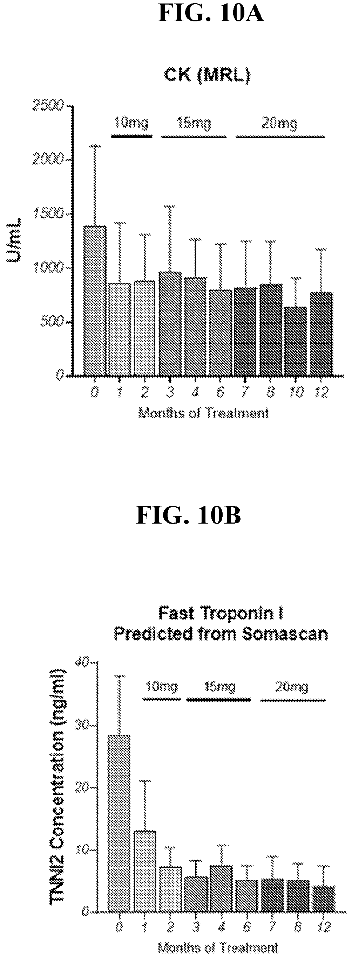

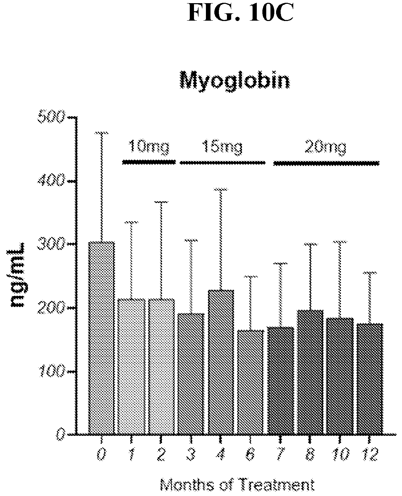

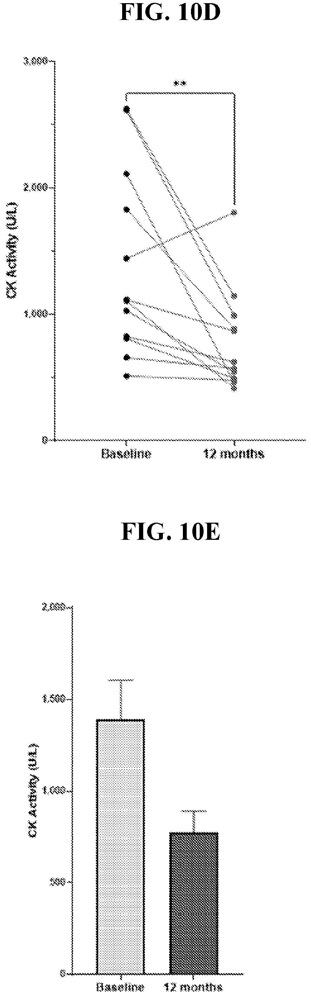

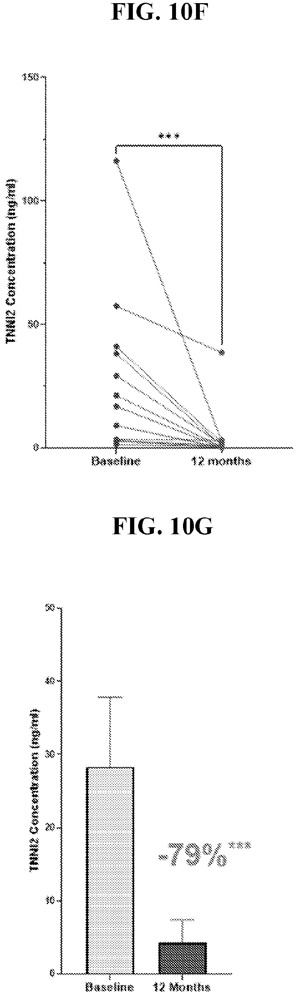

**FIGs. 10A-10G** depict the changes of several biomarkers after treatment of Compound 1. ** p<0.01 ; ***p<0.001. **FIG. 10A** depicts the average of creatine kinase (CK) level in different dosing regimens. All P< 0.05 except month 3. **FIG. 10B** depicts the average of fast skeletal muscle troponin I (TNNI2) level in different dosing regimens. All P< 0.05 except month 4. **FIG. 10C** depicts the average of myoglobin level in different dosing regimens. All P< 0.05 except month 2, 3, 4, 6, or 10. **FIG.10D** depicts the changes in CK activity in each patient from the 12-month treatment group. p = 0.0087. **FIG.10E** depicts the average of CK level between baseline and the 12-month treatment group. p = 0.0012. The percentage difference specified was against mean baseline; the bar graph shows means ± SEM. **FIG. 10F** depicts changes in TNNI2 activity for each patient in the 12-month treatment group. p = 0.022. The TNNI2 data were projected from SOMAscan. **FIG. 10G** depicts average of TNNI2 level between baseline and the 12-month treatment group. p <0.0001. The percentage difference specified was against mean baseline; the graph shows means ± SEM. The TNNI2 data were projected from SOMAscan.

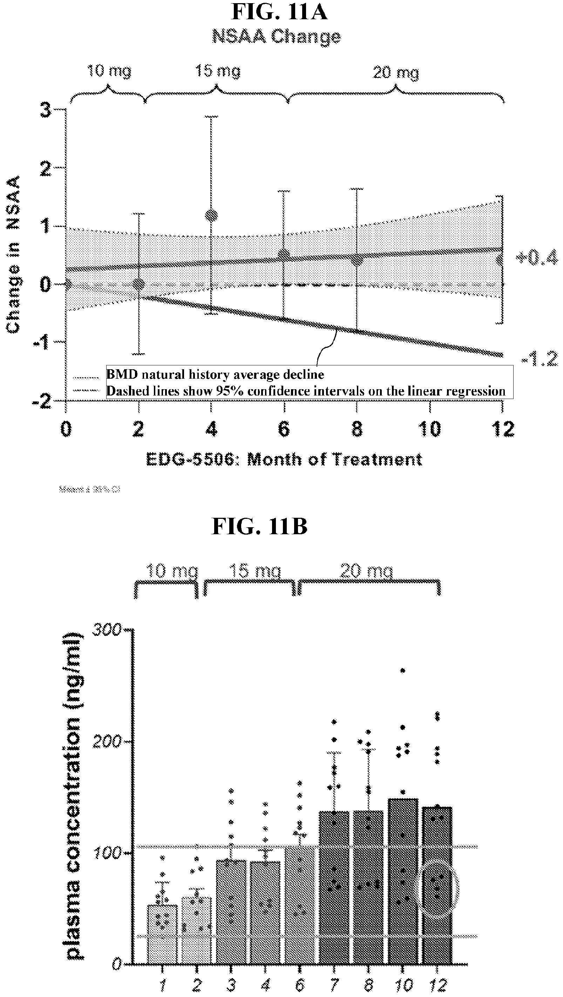

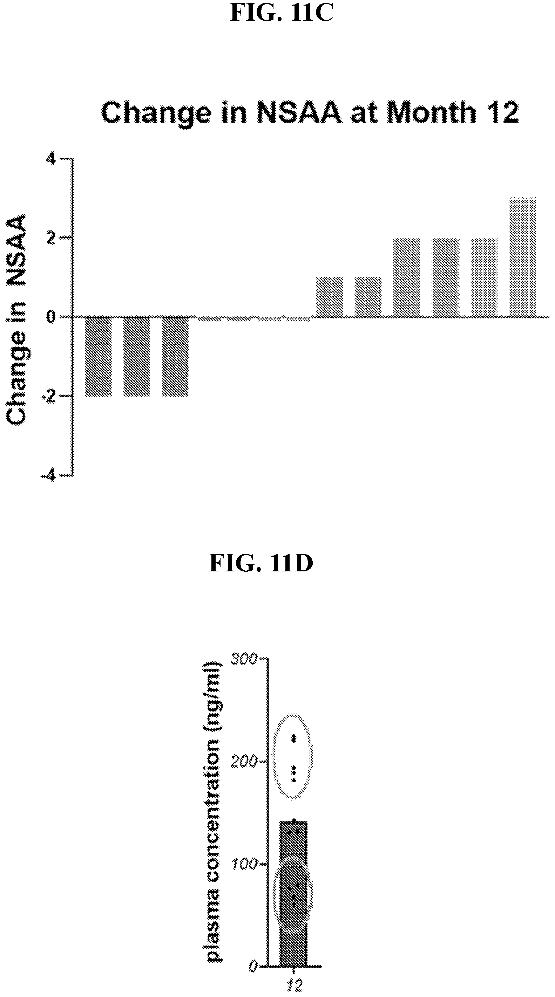

**FIGs. 11A-11D** depict the changes of NSAA scores and plasma concentrations of Compound 1 in different dosing regimens and the correlation thereof. **FIG. 11A** depicts the changes of NSAA scores in different dosing regimens. Dashed lines show means ± 95% CI. The call-out line showing a downward trend reflects natural history data, which were based on Luca Bello at al. MDA (2022) and van de Velde NM et al., Neurology (2021). **FIG. 11B** depicts the plasma concentrations of Compound 1 in different dosing regimens. **FIG.11C** depicts individual patients' NSAA responses from the 12-month group. The dash line represents the expected natural history decline in untreated patients. **FIG. 11D** depicts the corresponding plasma concentrations of Compound 1 from the 12-month group.

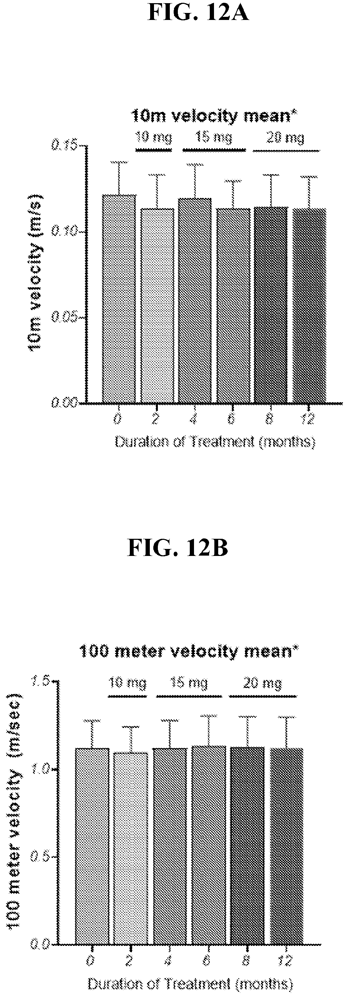

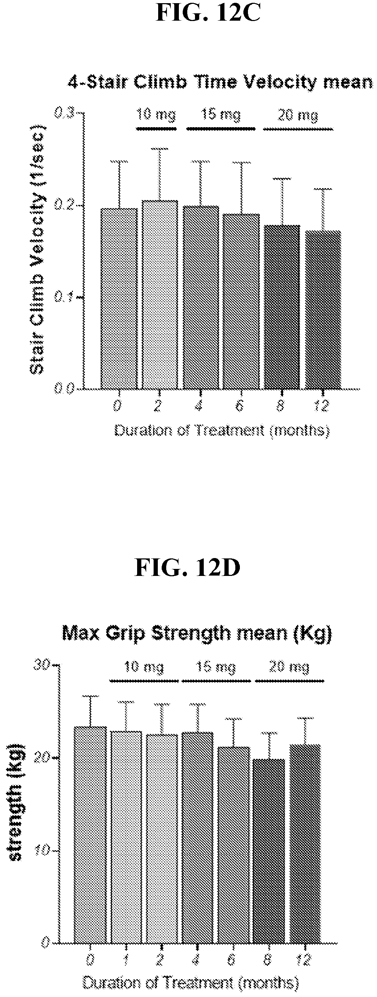

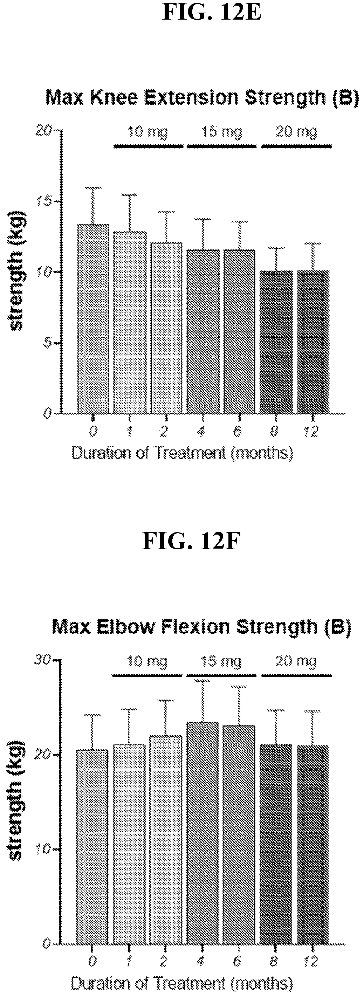

**FIGs. 12A-12F** depict the functional evaluation after the treatment of Compound 1. **FIG. 12A** depicts the average 10m velocity in different dosing regimens. All p-values NS with a 7% decrease at 12 months. Last observation carried forward (all N=12, except for 2 missing values, month 4 and 8). **FIG. 12B** depicts the average 100 meter velocity in different dosing regimens. All p-values NS with 0% change at 12 months. Last observation carried forward (all N=12, except for 2 missing values, month 4 and 8). **FIG. 12C** depicts the average 4-stair climb time velocity in different dosing regimens. All p-values NS except that p value for 12 months was 0.016, which was 13% decrease at 12 months. **FIG. 12D** depicts the average maximum grip strength in different dosing regimens. p-values <0,05 at 4, 6, 8 month and NS at 12 months. There was a 8% decrease at 12 months. **FIG. 12E** depicts the average maximum knee extension strength in different dosing regimens. P-values <0.05 at 4, 8, 12 month. There was a 25% decrease at 12 months. **FIG. 12F** depicts the maximum elbow flexion strength in different dosing regimens. All p-values NS, and there was no change at 12 months.
**FIG. 13** depicts self-reported pain scores after the treatment of Compound 1. The figure shows means ± SEM.
**FIG. 14A-14H** illustrate characterization of short- and long-term proteomic response to the fast skeletal myosin (e.g., Compound 1) in Becker muscular dystrophy (BMD).

### DETAILED DESCRIPTION OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Described herein, in some aspects, is a method for treating a neuromuscular condition, comprising administering to a subject in need thereof a dose of 1 mg to 40 mg per day of a compound of Formula (I): 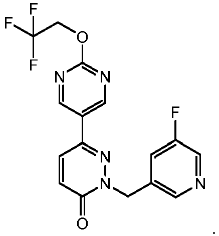 In some embodiments, the method comprises administering to the subject a dose of 15 mg to 40 mg per day of the compound of Formula (I). In some embodiments, the method comprises administering to the subject a dose of 1 mg, 2 mg, 2.5 mg, 5 mg, 7.5 mg, 10 mg, 12.5 mg, 15 mg, or 20 mg per day of the compound of Formula (I). In some embodiments, the method comprises administering to the subject a dose of 10 mg, 15 mg, or 20 mg per day of the compound of Formula (I), when the subject is over 18 years old. In some embodiments, the method comprises administering to the subject a dose of 10 mg, 7.5 mg, 5 mg, 2.5 mg, 2 mg, or 1 mg per day of the compound of Formula (I), when the subject is under 18 years old. In some embodiments, the administering comprises administering of a first dose of said muscle fast myosin (Type II)muscle fast myosin (Type II) inhibitor and one or more subsequent doses of said muscle fast myosin (Type II)muscle fast myosin (Type II) inhibitor, and optionally wherein the one or more subsequent doses are higher than the first dose. In some embodiments, said administering maintains a North Star Ambulatory Assessment (NSAA) score or increase the NSAA score of the subject relative to a pre-treatment NSAA score. In some embodiments, said administering maintains or improves one or more of the following functional measures of the subject: max elbow flexion strength relative to a pre-treatment max elbow flexion strength; max knee extension strength relative to a pre-treatment max knee extension strength;10 meter walk/run velocity relative to a pre-treatment 10 meter walk/run velocity; 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value. In some embodiments, said administering maintains a creatine kinase activity level or reduces the creatine kinase activity level of the subject relative to a pre-treatment creatine kinase activity level by 10% or more. In some embodiments, said administering maintains a creatine kinase activity level or increases the creatine kinase activity level of the subject relative to a pre-treatment creatine kinase activity level by 10% or more. In some embodiments, said administering maintains a Dual Energy x-Ray absorptiometry (DXA) % lean mass of the subject or increase the DXA % lean mass of the subject relative to a pre-treatment DXA % lean mass by 5% or more. In some embodiments, said administering maintains a Fast Skeletal Muscle Troponin I (TNNI2) concentration of the subject or reduce the TNNI2 concentration of the subject relative to a pre-treatment TNNI2 concentration by 10% or more, and optionally wherein the TNNI2 concentration is inferred from a SOMAscan level. In some embodiments, said administering decreases protein concentration of one or more muscle injury biomarkers in the subject relative to a pre-treatment protein concentration of the one or more muscle injury biomarkers by 10% or more. In some embodiments, the one or more muscle injury biomarkers comprise ACTN2, MYOM2, MYBPC1, PDLIM3, MYL3, TNNI2, MYL11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof. In some embodiments, said administering decreases protein concentration of one or more pro-inflammatory proteins in the subject relative to a pre-treatment protein concentration of the one or more pro-inflammatory proteins by 10% or more. In some embodiments, the one or more pro-inflammatory proteins comprise CCL22, CCL5, CXCL10, FGG, IFN-γ, IL3, PPBP, PF4, or a combination thereof. In some embodiments, said administering increases protein concentration of one or more anti-inflammatory proteins in the subject relative to a pre-treatment protein concentration of the one or more anti-inflammatory proteins by 10% or more. In some embodiments, the one or more anti-inflammatory proteins comprise IL10, IL13, IL22, IL37, IL4, or a combination thereof. In some embodiments, the neuromuscular condition is selected from Becker Muscular Dystrophy, Duchenne Muscular Dystrophy, Limb-Girdle Muscular Dystrophy and McArdle Disease. In some embodiments, the administering comprises administering the compound of Formula (I) for 2 months or more, 3 months or more, 4 months or more, 5 months or more, 6 months or more, or 7 months or more. In some embodiments, prior to said administering the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

In certain aspects, the disclosure provides methods and compositions for treating neuromuscular conditions through selective inhibition of fast-fiber skeletal muscle myosin. In particular, methods of the disclosure may be used in the treatment of DMD, BMD, LGMD, McArdle's disease, and other neuromuscular conditions. In some embodiments, the methods describe herein include compounds or salts previously described in publications WO2020097265, WO2020097258, WO2020097266, WO2021231572, WO2021231572, WO2021231546, WO2021231630, and WO2021231615.

Skeletal muscle is mainly composed of two types of fibers, slow-twitch muscle fiber (i.e., type I) and fast-twitch muscle fiber (i.e., type II). In each muscle, the two types of fibers are configured in a mosaic-like arrangement, with differences in fiber type composition in different muscles and at different points in growth and development. Slow-twitch muscle fibers have excellent aerobic energy production ability. Contraction rate of the slow-twitch muscle fiber is low but tolerance to fatigue is high. Slow-twitch muscle fibers typically have a higher concentration of mitochondria and myoglobin than do fast-twitch fibers and are surrounded by more capillaries than are fast-twitch fibers. Slow-twitch fibers contract at a slower rate due to lower myosin ATPase activity and produce less power compared to fast-twitch fibers, but they are able to maintain contractile function over longer-terms, such as in stabilization, postural control, and endurance exercises.

Fast twitch muscle fibers in humans are further divided into two main fiber types depending on the specific fast skeletal myosin they express (Type IIa, IIx/d). A third type of fast fiber (Type IIb) exists in other mammals but is rarely identified in human muscle. Fast-twitch muscle fibers have excellent anaerobic energy production ability and are able to generate high amounts of tension over a short period of time. Typically, fast-twitch muscle fibers have lower concentrations of mitochondria, myoglobin, and capillaries compared to slow-twitch fibers, and thus can fatigue more quickly. Fast-twitch muscles produce quicker force required for power and resistance activities.

The proportion of the type I and type II can vary in different individuals. For example, non-athletic individuals can have close to 50% of each muscle fiber types. Power athletes can have a higher ratio of fast-twitch fibers, e.g.,70-75% type II in sprinters. Endurance athletes can have a higher ratio of slow-twitch fibers, e.g., 70-80% in distance runners. The proportion of the type I and type II fibers can also vary depending on the age of an individual. The proportion of type II fibers, especially the type IIx, can decline as an individual ages, resulting in a loss in lean muscle mass.

The contractile action of skeletal muscle leads to muscle damage in subjects with neuromuscular disease, e.g., DMD, and this damage appears to be more prevalent in fast fibers. It has been observed that acute force drop after lengthening injury is greater in predominantly fast type II fiber muscles compared to predominantly slow type I fiber muscles in dystrophy mouse models. It has also been demonstrated that the degree of acute force drop and histological damage in dystrophy mouse models is proportional to peak force development during lengthening injury. Excessive contraction-induced injuries, which precede the inflammation and irreversible fibrosis that characterizes late-stage DMD pathology.

Inhibitors of skeletal muscle myosin that are not selective for the type II fibers may lead to excessive inhibition of skeletal muscle contraction including respiratory function and unwanted inhibition of cardiac activity as the heart shares several structural components (such as type I myosin) with type I skeletal muscle fibers. While not wishing to be bound by a particular mechanistic theory, this disclosure provides selective inhibitors of fast-fiber skeletal muscle myosin as a treatment option for Becker muscular dystrophy (BMD), Duchenne muscular dystrophy (DMD), Limb-girdle muscular dystrophies (LGMD), McArdle disease, and other neuromuscular conditions. The targeted inhibition of type II skeletal muscle myosin may reduce skeletal muscle contractions while minimizing the impact on a subject's daily activities.

When healthy muscle is subjected to excessive, unaccustomed exercise, it develops soreness and sustained reductions in strength and range of motion. Proteins also leak from injured muscle fibers into circulation, including creatine kinase (CK), lactate dehydrogenase and myoglobin. These biomarkers are not unique to either fast or slow fibers and so do not provide detail regarding differences in fiber responses to injury. Troponin I (TNNI) is a component of the troponin complex that controls initiation of contraction of muscle by calcium. It is distinct in that there is a different isoform for each type of striated muscle: TNNI1 in slow skeletal muscle, TNNI2 in fast skeletal muscle and TNNI3 in cardiac muscle. Selective enzyme-linked immunosorbent assays (ELISAs) have been used to demonstrate that TNNI2 but not TNNI1 is elevated in circulation after injurious exercise, even under extreme conditions.

DMD and BMD are caused by an absence (DMD) or truncation (BMD) of the dystrophin protein5. Dystrophin provides a structural link between the actin cytoskeleton and the basement membrane through the dystrophin-glycoprotein complex. When dystrophin is absent or truncated, contraction of muscle leads to heightened muscle stress and injury with normal use. While the sensitivity to injury is much higher in DMD muscle than in BMD or healthy muscle, fast fibers still appear to be more susceptible than slow fibers, with young DMD patients exhibiting histological evidence of disruption in fast fibers7 and early loss of type IIx fibers. In some embodiments, this disclosure provides selective inhibitors of fast-fiber skeletal muscle myosin as a treatment option for DMD, BMD, McArdle's disease, or Limb-girdle muscular dystrophies.

### Definitions

Unless defined otherwise, all technical and scientific terms used herein have the same meaning as is commonly understood by one of skill in the art to which this invention belongs.

As used in the specification and claims, the singular form "a", "an" and "the" includes plural references unless the context clearly dictates otherwise.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable excipient" or "pharmaceutically acceptable carrier" as used herein means a pharmaceutically acceptable material, composition, or vehicle, such as a liquid or solid filler, diluent, excipient, solvent, or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogenfree water; (17) isotonic saline; (18) Ringer's solution; (19) ethyl alcohol; (20) phosphate buffer solutions; and (21) other non-toxic compatible substances employed in pharmaceutical formulations.

As used herein, "treatment" or "treating" refers to an approach for obtaining beneficial or desired results with respect to a disease, disorder, or medical condition including but not limited to a therapeutic benefit and/or a prophylactic benefit. A therapeutic benefit can include, for example, the eradication or amelioration of the underlying disorder being treated. Also, a therapeutic benefit can include, for example, the eradication or amelioration of one or more of the physiological symptoms associated with the underlying disorder such that an improvement is observed in the subject, notwithstanding that the subject may still be afflicted with the underlying disorder. In certain embodiments, for prophylactic benefit, the compositions are administered to a subject at risk of developing a particular disease, or to a subject reporting one or more of the physiological symptoms of a disease, even though a diagnosis of this disease may not have been made. Treatment via administration of a compound described herein does not require the involvement of a medical professional.

The following is a discussion of compounds and salts thereof that may be used in the methods described herein. In certain embodiments, the compounds or salts of the disclosure inhibit muscle fast myosin (Type II)muscle fast myosin (Type II) and are referred to as muscle fast myosin (Type II)muscle fast myosin (Type II) inhibitors. In certain embodiments, the compounds, and salts for use in the methods and compositions of the disclosure are described in any of WO2020097265, WO2020097258, WO2020097266, WO2021231572, WO2021231572, WO2021231546, WO2021231630, and WO2021231615. In certain embodiments, the compound for use in the methods described herein is depicted as the compound of Formula (I): 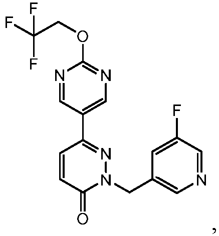 also referred to herein as Compound 1.

### Therapeutic Applications

Described herein, in some aspects, is a method for treating a neuromuscular condition, comprising administering to a subject in need thereof a dose of 1 mg to 40 mg per day of a compound of Formula (I): 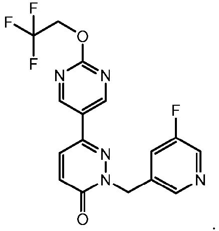 In some embodiments, the administering to the sibject comprises a dose of 15 mg to 40 mg per day of the compound of Formula (I). **In** some embodiments, the administering to the subject comprises a dose of 15 mg, 20 mg, 25 mg, or 30 mg per day of the compound of Formula (I). **In** some embodiments, the dose of the compound of Formula (I) comprises about 1 mg per day to about 30 mg per day. **In** some embodiments, the dose of the compound of Formula (I) comprises about 1 mg per day to about 2 mg per day, about 1 mg per day to about 2.5 mg per day, about 1 mg per day to about 3 mg per day, about 1 mg per day to about 5 mg per day, about 1 mg per day to about 7.5 mg per day, about 1 mg per day to about 10 mg per day, about 1 mg per day to about 12.5 mg per day, about 1 mg per day to about 15 mg per day, about 1 mg per day to about 20 mg per day, about 1 mg per day to about 25 mg per day, about 1 mg per day to about 30 mg per day, about 2 mg per day to about 2.5 mg per day, about 2 mg per day to about 3 mg per day, about 2 mg per day to about 5 mg per day, about 2 mg per day to about 7.5 mg per day, about 2 mg per day to about 10 mg per day, about 2 mg per day to about 12.5 mg per day, about 2 mg per day to about 15 mg per day, about 2 mg per day to about 20 mg per day, about 2 mg per day to about 25 mg per day, about 2 mg per day to about 30 mg per day, about 2.5 mg per day to about 3 mg per day, about 2.5 mg per day to about 5 mg per day, about 2.5 mg per day to about 7.5 mg per day, about 2.5 mg per day to about 10 mg per day, about 2.5 mg per day to about 12.5 mg per day, about 2.5 mg per day to about 15 mg per day, about 2.5 mg per day to about 20 mg per day, about 2.5 mg per day to about 25 mg per day, about 2.5 mg per day to about 30 mg per day, about 3 mg per day to about 5 mg per day, about 3 mg per day to about 7.5 mg per day, about 3 mg per day to about 10 mg per day, about 3 mg per day to about 12.5 mg per day, about 3 mg per day to about 15 mg per day, about 3 mg per day to about 20 mg per day, about 3 mg per day to about 25 mg per day, about 3 mg per day to about 30 mg per day, about 5 mg per day to about 7.5 mg per day, about 5 mg per day to about 10 mg per day, about 5 mg per day to about 12.5 mg per day, about 5 mg per day to about 15 mg per day, about 5 mg per day to about 20 mg per day, about 5 mg per day to about 25 mg per day, about 5 mg per day to about 30 mg per day, about 7.5 mg per day to about 10 mg per day, about 7.5 mg per day to about 12.5 mg per day, about 7.5 mg per day to about 15 mg per day, about 7.5 mg per day to about 20 mg per day, about 7.5 mg per day to about 25 mg per day, about 7.5 mg per day to about 30 mg per day, about 10 mg per day to about 12.5 mg per day, about 10 mg per day to about 15 mg per day, about 10 mg per day to about 20 mg per day, about 10 mg per day to about 25 mg per day, about 10 mg per day to about 30 mg per day, about 12.5 mg per day to about 15 mg per day, about 12.5 mg per day to about 20 mg per day, about 12.5 mg per day to about 25 mg per day, about 12.5 mg per day to about 30 mg per day, about 15 mg per day to about 20 mg per day, about 15 mg per day to about 25 mg per day, about 15 mg per day to about 30 mg per day, about 20 mg per day to about 25 mg per day, about 20 mg per day to about 30 mg per day, or about 25 mg per day to about 30 mg per day. In some embodiments, the dose of the compound of Formula (I) comprises about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day. In some embodiments, the dose of the compound of Formula (I) comprises at least about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, or about 25 mg per day. In some embodiments, the dose of the compound of Formula (I) comprises at most about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day.

In some embodiments, the dose of the compound of Formula (I) comprises at least about 1 mg per day to about 30 mg per day. In some embodiments, the dose of the compound of Formula (I) comprises at least about 1 mg per day to about 2 mg per day, about 1 mg per day to about 2.5 mg per day, about 1 mg per day to about 3 mg per day, about 1 mg per day to about 5 mg per day, about 1 mg per day to about 7.5 mg per day, about 1 mg per day to about 10 mg per day, about 1 mg per day to about 12.5 mg per day, about 1 mg per day to about 15 mg per day, about 1 mg per day to about 20 mg per day, about 1 mg per day to about 25 mg per day, about 1 mg per day to about 30 mg per day, about 2 mg per day to about 2.5 mg per day, about 2 mg per day to about 3 mg per day, about 2 mg per day to about 5 mg per day, about 2 mg per day to about 7.5 mg per day, about 2 mg per day to about 10 mg per day, about 2 mg per day to about 12.5 mg per day, about 2 mg per day to about 15 mg per day, about 2 mg per day to about 20 mg per day, about 2 mg per day to about 25 mg per day, about 2 mg per day to about 30 mg per day, about 2.5 mg per day to about 3 mg per day, about 2.5 mg per day to about 5 mg per day, about 2.5 mg per day to about 7.5 mg per day, about 2.5 mg per day to about 10 mg per day, about 2.5 mg per day to about 12.5 mg per day, about 2.5 mg per day to about 15 mg per day, about 2.5 mg per day to about 20 mg per day, about 2.5 mg per day to about 25 mg per day, about 2.5 mg per day to about 30 mg per day, about 3 mg per day to about 5 mg per day, about 3 mg per day to about 7.5 mg per day, about 3 mg per day to about 10 mg per day, about 3 mg per day to about 12.5 mg per day, about 3 mg per day to about 15 mg per day, about 3 mg per day to about 20 mg per day, about 3 mg per day to about 25 mg per day, about 3 mg per day to about 30 mg per day, about 5 mg per day to about 7.5 mg per day, about 5 mg per day to about 10 mg per day, about 5 mg per day to about 12.5 mg per day, about 5 mg per day to about 15 mg per day, about 5 mg per day to about 20 mg per day, about 5 mg per day to about 25 mg per day, about 5 mg per day to about 30 mg per day, about 7.5 mg per day to about 10 mg per day, about 7.5 mg per day to about 12.5 mg per day, about 7.5 mg per day to about 15 mg per day, about 7.5 mg per day to about 20 mg per day, about 7.5 mg per day to about 25 mg per day, about 7.5 mg per day to about 30 mg per day, about 10 mg per day to about 12.5 mg per day, about 10 mg per day to about 15 mg per day, about 10 mg per day to about 20 mg per day, about 10 mg per day to about 25 mg per day, about 10 mg per day to about 30 mg per day, about 12.5 mg per day to about 15 mg per day, about 12.5 mg per day to about 20 mg per day, about 12.5 mg per day to about 25 mg per day, about 12.5 mg per day to about 30 mg per day, about 15 mg per day to about 20 mg per day, about 15 mg per day to about 25 mg per day, about 15 mg per day to about 30 mg per day, about 20 mg per day to about 25 mg per day, about 20 mg per day to about 30 mg per day, or about 25 mg per day to about 30 mg per day. In some embodiments, the dose of the compound of Formula (I) comprises at least about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day. In some embodiments, the dose of the compound of Formula (I) comprises at least at least about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, or about 25 mg per day. In some embodiments, the dose of the compound of Formula (I) comprises at least at most about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day.

In some embodiments, the dose of the compound of Formula (I) comprises at most about 1 mg per day to about 30 mg per day. In some embodiments, the dose of the compound of Formula (I) comprises at most about 1 mg per day to about 2 mg per day, about 1 mg per day to about 2.5 mg per day, about 1 mg per day to about 3 mg per day, about 1 mg per day to about 5 mg per day, about 1 mg per day to about 7.5 mg per day, about 1 mg per day to about 10 mg per day, about 1 mg per day to about 12.5 mg per day, about 1 mg per day to about 15 mg per day, about 1 mg per day to about 20 mg per day, about 1 mg per day to about 25 mg per day, about 1 mg per day to about 30 mg per day, about 2 mg per day to about 2.5 mg per day, about 2 mg per day to about 3 mg per day, about 2 mg per day to about 5 mg per day, about 2 mg per day to about 7.5 mg per day, about 2 mg per day to about 10 mg per day, about 2 mg per day to about 12.5 mg per day, about 2 mg per day to about 15 mg per day, about 2 mg per day to about 20 mg per day, about 2 mg per day to about 25 mg per day, about 2 mg per day to about 30 mg per day, about 2.5 mg per day to about 3 mg per day, about 2.5 mg per day to about 5 mg per day, about 2.5 mg per day to about 7.5 mg per day, about 2.5 mg per day to about 10 mg per day, about 2.5 mg per day to about 12.5 mg per day, about 2.5 mg per day to about 15 mg per day, about 2.5 mg per day to about 20 mg per day, about 2.5 mg per day to about 25 mg per day, about 2.5 mg per day to about 30 mg per day, about 3 mg per day to about 5 mg per day, about 3 mg per day to about 7.5 mg per day, about 3 mg per day to about 10 mg per day, about 3 mg per day to about 12.5 mg per day, about 3 mg per day to about 15 mg per day, about 3 mg per day to about 20 mg per day, about 3 mg per day to about 25 mg per day, about 3 mg per day to about 30 mg per day, about 5 mg per day to about 7.5 mg per day, about 5 mg per day to about 10 mg per day, about 5 mg per day to about 12.5 mg per day, about 5 mg per day to about 15 mg per day, about 5 mg per day to about 20 mg per day, about 5 mg per day to about 25 mg per day, about 5 mg per day to about 30 mg per day, about 7.5 mg per day to about 10 mg per day, about 7.5 mg per day to about 12.5 mg per day, about 7.5 mg per day to about 15 mg per day, about 7.5 mg per day to about 20 mg per day, about 7.5 mg per day to about 25 mg per day, about 7.5 mg per day to about 30 mg per day, about 10 mg per day to about 12.5 mg per day, about 10 mg per day to about 15 mg per day, about 10 mg per day to about 20 mg per day, about 10 mg per day to about 25 mg per day, about 10 mg per day to about 30 mg per day, about 12.5 mg per day to about 15 mg per day, about 12.5 mg per day to about 20 mg per day, about 12.5 mg per day to about 25 mg per day, about 12.5 mg per day to about 30 mg per day, about 15 mg per day to about 20 mg per day, about 15 mg per day to about 25 mg per day, about 15 mg per day to about 30 mg per day, about 20 mg per day to about 25 mg per day, about 20 mg per day to about 30 mg per day, or about 25 mg per day to about 30 mg per day. In some embodiments, the dose of the compound of Formula (I) comprises at most about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day. In some embodiments, the dose of the compound of Formula (I) comprises at most at least about 1 mg per day , about 2 mg per day, about 2.5 mg per day, about 3 mg per day, about 5 mg per day, about 7.5 mg per day, about 10 mg per day, about 12.5 mg per day, about 15 mg per day, about 20 mg per day, or about 25 mg per day. In some embodiments, the dose of the compound of Formula (I) comprises at most at most about 2 mg per day, about 2.5 mg per day, about 3 mg per day, about 5 mg per day, about 7.5 mg per day, about 10 mg per day, about 12.5 mg per day, about 15 mg per day, about 20 mg per day, about 25 mg per day, or about 30 mg per day.

In some embodiments, the administering to the subject comprises a dose of 20 mg per day of the compound of Formula (I). In some embodiments, the administering to the subject comprises a dose of 1 mg to 20 mg per day of the compound of Formula (I). In some embodiments, the administering to the subject comprise a dose of 1 mg, 2 mg, 2.5 mg, 5 mg, 7.5 mg, 10 mg, 12.5 mg, 15 mg, or 20 mg per day of the compound of Formula (I). In some embodiments, the administering to the subject comprises a dose of 1 mg per day of the compound of Formula (I). In some embodiments, the administering to the subject comprises a dose of 2 mg per day of the compound of Formula (I). In some embodiments, the administering to the subject comprises a dose of 2.5 mg per day of the compound of Formula (I). In some embodiments, the administering to the subject comprises a dose of 5 mg per day of the compound of Formula (I). In some embodiments, the administering to the subject comprises a dose of 7.5 mg per day of the compound of Formula (I). In some embodiments, the administering to the subject comprises a dose of 10 mg per day of the compound of Formula (I). In some embodiments, the administering to the subject comprises a dose of 15 mg per day of the compound of Formula (I). In some embodiments, the administering to the subject comprises a dose of 20 mg per day of the compound of Formula (I).

In some embodiments, the subject being treated by the compound of Formula (I) is over 18 years old. In some embodiments, the administering to the subject comprises a dose of 10 mg per day of the compound of Formula (I). In some embodiments, the administering to the subject comprises a dose of 15 mg per day of the compound of Formula (I). In some embodiments, the administering to the subject comprises a dose of 20 mg per day of the compound of Formula (I). In some embodiments, the administering to the subject comprises a dose of 10 mg per day of the compound of Formula (I). In some embodiments, the administering to the subject comprises a dose of 7.5 mg per day of the compound of Formula (I). In some embodiments, the administering to the subject comprises a dose of 5 mg per day of the compound of Formula (I). In some embodiments, the administering to the subject comprises a dose of 2.5 mg per day of the compound of Formula (I). In some embodiments, the administering to the subject comprises a dose of 2 mg per day of the compound of Formula (I). In some embodiments, the administering to the subject comprises a dose of 1 mg per day of the compound of Formula (I). In some embodiments, the administering comprises administering of a first dose of said muscle fast myosin (Type II)muscle fast myosin (Type II) inhibitor and one or more subsequent doses of said muscle fast myosin (Type II)muscle fast myosin (Type II) inhibitor, and optionally wherein the one or more subsequent doses are higher than the first dose. In some embodiments, said administering maintains a North Star Ambulatory Assessment (NSAA) score or increase the NSAA score of the subject relative to a pre-treatment NSAA score. In some embodiments, said administering maintains or improves one or more of the following functional measures of the subject: max elbow flexion strength relative to a pre-treatment max elbow flexion strength; max knee extension strength relative to a pre-treatment max knee extension strength;10 meter walk/run velocity relative to a pre-treatment 10 meter walk/run velocity; 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value. In some embodiments, said administering maintains a creatine kinase activity level or reduces the creatine kinase activity level of the subject relative to a pre-treatment creatine kinase activity level by 10% or more. In some embodiments, said administering maintains a creatine kinase activity level or increases the creatine kinase activity level of the subject relative to a pre-treatment creatine kinase activity level by 10% or more. In some embodiments, said administering maintains a Dual Energy x-Ray absorptiometry (DXA) % lean mass of the subject or increase the DXA % lean mass of the subject relative to a pre-treatment DXA % lean mass by 5% or more. In some embodiments, said administering maintains a Fast Skeletal Muscle Troponin I (TNNI2) concentration of the subject or reduce the TNNI2 concentration of the subject relative to a pre-treatment TNNI2 concentration by 10% or more, and optionally wherein the TNNI2 concentration is inferred from a SOMAscan level. In some embodiments, said administering decreases protein concentration of one or more muscle injury biomarkers in the subject relative to a pre-treatment protein concentration of the one or more muscle injury biomarkers by 10% or more. In some embodiments, the one or more muscle injury biomarkers comprise ACTN2, MYOM2, MYBPC1, PDLIM3, MYL3, TNNI2, MYLI11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof. In some embodiments, said administering decreases protein concentration of one or more pro-inflammatory proteins in the subject relative to a pre-treatment protein concentration of the one or more pro-inflammatory proteins by 10% or more. In some embodiments, the one or more pro-inflammatory proteins comprise CCL22, CCL5, CXCL10, FGG, IFN-γ, IL3, PPBP, PF4, or a combination thereof. In some embodiments, said administering increases protein concentration of one or more anti-inflammatory proteins in the subject relative to a pre-treatment protein concentration of the one or more anti-inflammatory proteins by 10% or more. In some embodiments, the one or more anti-inflammatory proteins comprise IL10, IL13, IL22, IL37, IL4, or a combination thereof. In some embodiments, the neuromuscular condition is selected from Becker Muscular Dystrophy, Duchenne Muscular Dystrophy, Limb-Girdle Muscular Dystrophy and McArdle Disease. In some embodiments, the administering comprises administering the compound of Formula (I) for 2 months or more, 3 months or more, 4 months or more, 5 months or more, 6 months or more, or 7 months or more. In some embodiments, prior to said administering, the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

The methods described herein are useful for the treatment of neuromuscular conditions. In some embodiments, the methods comprise administering a composition comprising a muscle fast myosin (Type II)muscle fast myosin (Type II) inhibitor and one or more pharmaceutically acceptable carriers, diluents, excipients, stabilizers, dispersing agents, suspending agents, and/or thickening agents. In some embodiments, the muscle fast myosin (Type II)muscle fast myosin (Type II) inhibitor is a compound described in WO2020097265, WO2020097258, WO2020097266, WO2021231572, WO2021231572, WO2021231546, WO2021231630, WO2021231615 or PCT Application PCT/US2023/021739. In some embodiments, the muscle fast myosin (Type II)muscle fast myosin (Type II) inhibitor is described in WO2020097266. In some embodiments, the muscle myosin inhibitor is depicted as Formula (I): 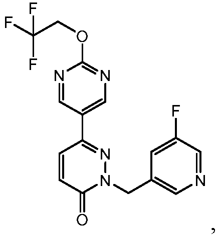 also referred to as Compound 1.

Methods of administration of Compound 1 or another muscle fast myosin (Type II)muscle fast myosin (Type II) inhibitor may be used for the treatment of neuromuscular conditions and movement disorders. Examples of neuromuscular conditions include but are not limited to Duchenne Muscular Dystrophy, Becker muscular dystrophy, myotonic dystrophy 1, myotonic dystrophy 2, facioscapulohumeral muscular dystrophy, oculopharyngeal muscular dystrophy, limb girdle muscular dystrophies, tendinitis, and carpal tunnel syndrome. Examples of movement disorders include but are not limited to muscle spasticity disorders, spasticity associated with multiple sclerosis, Parkinson's disease, Alzheimer's disease, or cerebral palsy, or injury or a traumatic event such as stroke, traumatic brain injury, spinal cord injury, hypoxia, meningitis, encephalitis, phenylketonuria, or amyotrophic lateral sclerosis. Also included are other conditions that may respond to the inhibition of skeletal myosin II, skeletal troponin C, skeletal troponin I, skeletal tropomyosin, skeletal troponin T, skeletal regulatory light chains, skeletal myosin binding protein C or skeletal actin. In some embodiments, neuromuscular conditions and movement disorders are selected from muscular dystrophies and myopathies. In some embodiments, muscular dystrophies are diseases that cause progressive weakness and loss of muscle mass where abnormal genes (mutations) interfere with the production of proteins needed to form healthy muscle. In some embodiments, muscular dystrophies are selected from Becker muscular dystrophy (BMD), Congenital muscular dystrophies (CMD), Duchenne muscular dystrophy (DMD), Emery-Dreifuss muscular dystrophy (EDMD), Facioscapulohumeral muscular dystrophy (FSHD), Limb-girdle muscular dystrophies (LGMD), Myotonic dystrophy (DM), and Oculopharyngeal muscular dystrophy (OPMD). In some embodiments, Congenital muscular dystrophies (CMD) is selected from Bethlem CMD, Fukuyama CMD, Muscle-eye-brain diseases (MEBs), Rigid spine syndromes, Ullrich CMD, and Walker-Warburg syndromes (WWS). In some embodiments, myopathies are diseases of muscle that are not caused by nerve disorders. Myopathies cause the muscles to become weak or shrunken (atrophied). In some embodiments, myopathies are selected from congenital myopathies, distal myopathies, endocrine myopathies, inflammatory myopathies, metabolic myopathies, myofibrillar myopathies (MFM), scapuloperoneal myopathy, and cardiomyopathies. In some embodiments, congenital myopathies are selected from cap myopathies, centronuclear myopathies, congenital myopathies with fiber type disproportion, core myopathies, central core disease, multiminicore myopathies, myosin storage myopathies, myotubular myopathy, and nemaline myopathies. In some embodiments, distal myopathies are selected from, GNE myopathy/Nonaka myopathy/hereditary inclusion-body myopathy (HIBM), Laing distal myopathy, Markesbery-Griggs late-onset distal myopathy, Miyoshi myopathy, Udd myopathy/tibial muscular dystrophy, VCP myopathy / IBMPFD, vocal cord and pharyngeal distal myopathy, and welander distal myopathy. In some embodiments, endocrine myopathies are selected from, hyperthyroid myopathy, and hypothyroid myopathy. In some embodiments, inflammatory myopathies are selected from, dermatomyositis, inclusion-body myositis, and polymyositis. In some embodiments, metabolic myopathies are selected from, von Gierke's disease, Anderson disease, Fanconi-Bickel syndrome, aldolase A deficiency, acid maltase deficiency (Pompe disease), carnitine deficiency, carnitine palmitoyltransferase deficiency, debrancher enzyme deficiency (Cori disease, Forbes disease), lactate dehydrogenase deficiency, myoadenylate deaminase deficiency, phosphofructokinase deficiency (Tarui disease), phosphoglycerate kinase deficiency, phosphoglycerate mutase deficiency (e.g., glycogen storage disease X), and phosphorylase deficiency (McArdle disease). In some embodiments, cardiomyopathies are selected from intrinsic cardiomyopathies and extrinsic cardiomyopathies. In some embodiments, intrinsic cardiomyopathies are selected from genetic myopathies and acquired myopathies. In some embodiments, genetic myopathies are selected from Hypertrophic cardiomyopathy, arrhythmogenic right ventricular cardiomyopathy (ARVC), LV noncompaction, ion channelopathies, dilated cardiomyopathy (DCM), and restrictive cardiomyopathy (RCM). In some embodiments, acquired myopathies are selected from stress cardiomyopathy, myocarditis, eosinophilic myocarditis, and ischemic cardiomyopathy. In some embodiments, extrinsic cardiomyopathies are selected from metabolic cardiomyopathies, endomyocardial cardiomyopathies, endocrine cardiomyopathies, and cardiofacial cardiomyopathies. In some embodiments, metabolic cardiomyopathies are selected from Fabry's disease and hemochromatosis. In some embodiments, endomyocardial cardiomyopathies are selected from endomyocardial fibrosis and Hypereosinophilic syndrome. In some embodiments, endocrine cardiomyopathies are selected from diabetes mellitus, hyperthyroidism, and acromegaly. In some embodiments, the Cardiofacial cardiomyopathy is Noonan syndrome.

In some aspects, methods of treating neuromuscular conditions or movement disorders may comprise administering Compound 1 or a salt thereof to inhibit skeletal muscle contraction. In some embodiments, Compound 1 or a salt thereof does not significantly inhibit cardiac muscle contraction. In some embodiments, cardiac muscle contraction is inhibited by 20% or less. In some embodiments, cardiac muscle contraction is inhibited by 15% or less. In some embodiments, cardiac muscle contraction is inhibited by 10% or less. In some embodiments, cardiac muscle contraction is inhibited by 9% or less. In some embodiments, cardiac muscle contraction is inhibited by 8% or less. In some embodiments, cardiac muscle contraction is inhibited by 7% or less. In some embodiments, cardiac muscle contraction is inhibited by 6% or less. In some embodiments, cardiac muscle contraction is inhibited by 5% or less. In some embodiments, cardiac muscle contraction is inhibited by 4% or less. In some embodiments, cardiac muscle contraction is inhibited by 3% or less. In some embodiments, cardiac muscle contraction is inhibited by 2% or less. In some embodiments, cardiac muscle contraction is inhibited by 1% or less.

A subject's activities of daily life (ADL) or habitual physical activity may be monitored prior to and following the treatment with Compound 1 or a salt thereof. ADL or habitual physical activity is subject-dependent and may range from simple walking to extensive exercise depending on the subject's ability and routine. Treatment options and dosages of the skeletal muscle contraction inhibitors discussed herein may be personalized to a subject such that the ADL and habitual physical activity remains unchanged.

In some aspects, methods of treating neuromuscular conditions or movement disorders may comprise administering Compound 1 or a salt thereof to inhibit skeletal muscle contraction. Compound 1 or a salt thereof may be given in an amount relative to the amount needed to reduce skeletal muscle contraction by 50%. Compound 1 or a salt thereof may be administered in an amount less than the amount needed to reduce skeletal muscle contraction by 50% relative to pre-treatment skeletal muscle contraction capacity of the subject. Compound 1 or a salt thereof may be administered in an amount that reduces skeletal muscle contraction by 5% to 45% relative to pre-treatment skeletal muscle contraction capacity of said subject. In some cases, Compound 1 or a salt thereof may be administered in an amount that reduces skeletal muscle contraction by less than 10%, less than 15%, less than 20%, less than 25%, less than 30%, less than 35%, less than 40%, less than 45% or even less than 50% relative to pre-treatment skeletal muscle contraction capacity of said subject. In certain embodiments, Compound 1 or a salt thereof may be administered in an amount that reduces skeletal muscle contraction from 1% to 50% relative to pre-treatment skeletal muscle contraction capacity of said subject.

In some aspects, methods of treating neuromuscular conditions or movement disorders may comprise administering Compound 1 or a salt thereof to inhibit type I skeletal muscle contraction. The inhibitor of type I skeletal muscle contraction may be given in an amount relative to the amount needed to reduce type I skeletal muscle contraction by 20%. The inhibitor of type I skeletal muscle contraction may be administered in an amount less than the amount needed to reduce type I skeletal muscle contraction by 20% relative to pre-treatment type I skeletal muscle contraction capacity of the subject. The inhibitor of type I skeletal muscle contraction may be administered in an amount that reduces type I skeletal muscle contraction by 0.01% to 20% relative to pre-treatment type I skeletal muscle contraction capacity of said subject. In some cases, the inhibitor may be administered in an amount that reduces type I skeletal muscle contraction by less than 0.01%, less than 0.1%, less than 0.5%, less than 1%, less than 5%, less than 10%, less than 15% or less than 20% relative to pre-treatment type I skeletal muscle contraction capacity of said subject. In certain embodiments, the inhibitor may be administered in an amount that reduces type I skeletal muscle contraction from 0.01% to 20% relative to pre-treatment type I skeletal muscle contraction capacity of said subject.

In some aspects, methods of treating neuromuscular conditions or movement disorders may comprise administering Compound 1 or a salt thereof to inhibit type II skeletal muscle contraction. The inhibitor of type II skeletal muscle contraction may be given in an amount relative to the amount needed to reduce type II skeletal muscle contraction by 90%. The inhibitor of type II skeletal muscle contraction may be administered in an amount less than the amount needed to reduce type II skeletal muscle contraction by 90% relative to pre-treatment type II skeletal muscle contraction capacity of the subject. The inhibitor of type II skeletal muscle contraction may be administered in an amount that reduces type II skeletal muscle contraction by 5% to 75% relative to pre-treatment type II skeletal muscle contraction capacity of said subject. In some cases, the inhibitor may be administered in an amount that reduces type II skeletal muscle contraction by less than 10%, less than 15%, less than 20%, less than 25%, less than 30%, less than 35%, less than 40%, less than 45%, less than 50%, less than 55%, less than 60%, less than 65%, less than 70%, less than 75%, less than 80%, less than 85% or even less than 90% relative to pre-treatment type II skeletal muscle contraction capacity of said subject. In certain embodiments, the inhibitor may be administered in an amount that reduces type II skeletal muscle contraction by from 1% to 50% relative to pre-treatment type II skeletal muscle contraction capacity of said subject.

In some aspects, methods of treating contraction-induced injury in skeletal muscle fiber may comprise administering Compound 1 or a salt thereof to inhibit skeletal muscle contraction and/or skeletal muscle fast myosin (Type II)muscle fast myosin (Type II). In certain embodiments, the inhibitor does not appreciably inhibit cardiac muscle contraction.

In some aspects, methods of treating metabolic myopathies, e.g. McArdle's syndrome, may comprise administering Compound 1 or a salt thereof.

In certain embodiments, the contraction-induced injury in skeletal muscle fiber is from involuntary skeletal muscle contraction. The involuntary skeletal muscle contraction may be associated with a neuromuscular condition or spasticity-associated condition. In certain embodiments, the contraction-induced injury in skeletal muscle fiber may be from voluntary skeletal muscle contraction, e.g., physical exercise.

In certain embodiments, the administration of Compound 1 or a salt thereof to a subject modulates one or more biomarkers associated with muscle contraction. Examples of biomarkers include but are not limited to creatinine, creatine kinase (CK), Troponin T (TnT), Troponin C (TnC), Troponin I (TnI), pyruvate kinase (PK), lactate dehydrogenase (LDH), myoglobin, isoforms of TnI (such as cardiac, slow skeletal, fast skeletal muscles) and inflammatory markers (IL-1, IL-6, IL-4, TNF-α). Biomarkers may also include measures of muscle inflammation for example, edema. The level of biomarkers described herein may increase after the administration of the inhibitor relative to pre-treatment level of the biomarkers. Alternatively, the level of biomarkers may decrease after the administration of the inhibitor relative to pre-treatment level of the biomarkers. The modulation of one or more biomarkers with an inhibitor described herein may indicate treatment of a neuromuscular condition such as those described herein.

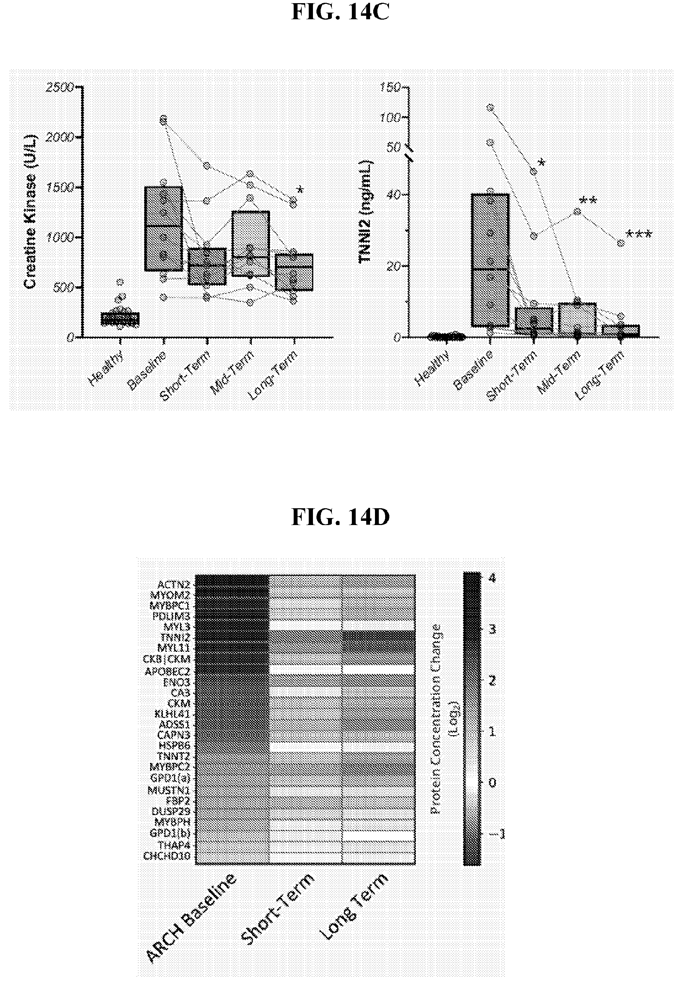

In certain embodiments, the administration of Compound 1 or a salt thereof to a subject decreases one or more muscle injury biomarkers (e.g., one or more of the muscle injury proteins shown in FIG. 14D). Examples of the one or more muscle injury biomarkers can include ACTN2, MYOM2, MYBPC1, PDLIM3, MYL3, TNNI2, MYL11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof. In certain embodiments, the administration of Compound 1 or a salt thereof to a subject decreases one or more muscle injury biomarkers during short-term treatment (e.g., 1-2 months of treatment with Compound 1). In certain embodiments, the administration of Compound 1 or a salt thereof to a subject decreases one or more muscle injury biomarkers during mid-term treatment (e.g., 3-4 months of treatment with Compound 1). In certain embodiments, the administration of Compound 1 or a salt thereof to a subject decreases one or more muscle injury biomarkers during long-term treatment (e.g., 6-12 months of treatment with Compound 1).

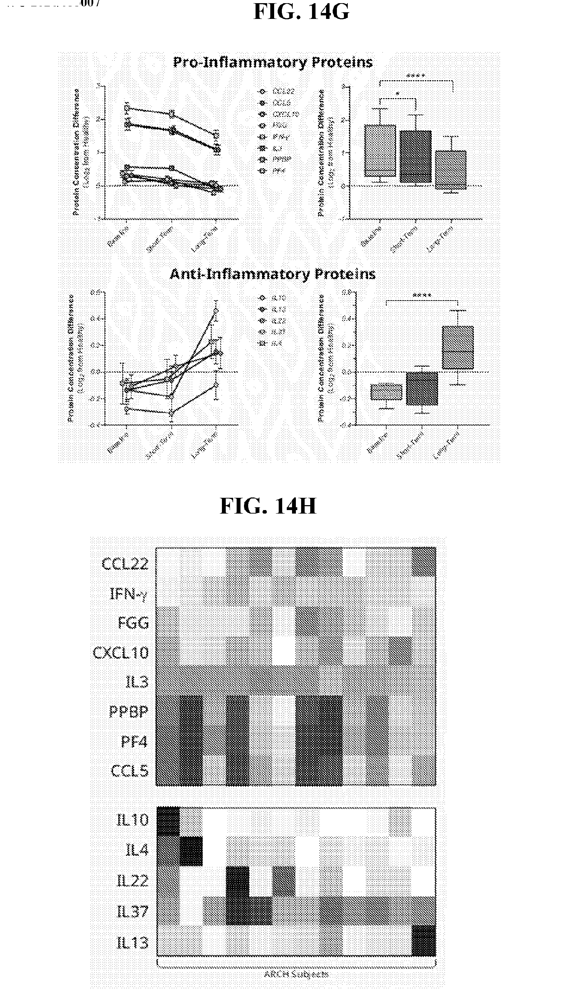

In certain embodiments, the administration of Compound 1 or a salt thereof to a subject decreases one or more pro-inflammatory proteins (e.g., one or more of the pro-inflammatory proteins shown in FIG. 14G or FIG. 14H). Examples of the one or more pro-inflammatory proteins can include CCL22, CCL5, CXCL10, FGG, IFN-γ, IL3, PPBP, PF4, or a combination thereof. In certain embodiments, the administration of Compound 1 or a salt thereof to a subject decreases one or more pro-inflammatory proteins during short-term treatment (e.g., 1-2 months of treatment with Compound 1). In certain embodiments, the administration of Compound 1 or a salt thereof to a subject decreases one or more pro-inflammatory proteins during mid-term treatment (e.g., 3-4 months of treatment with Compound 1). In certain embodiments, the administration of Compound 1 or a salt thereof to a subject decreases one or more pro-inflammatory proteins during long-term treatment (e.g., 6-12 months of treatment with Compound 1).

In certain embodiments, the administration of Compound 1 or a salt thereof to a subject increases one or more anti-inflammatory proteins (e.g., one or more of the anti-inflammatory proteins shown in FIG. 14G or FIG. 14H). Examples of the one or more anti-inflammatory proteins can include IL10, IL13, IL22, IL37, IL4, or a combination thereof. In certain embodiments, the administration of Compound 1 or a salt thereof to a subject increases one or more anti-inflammatory proteins during short-term treatment (e.g., 1-2 months of treatment with Compound 1). In certain embodiments, the administration of Compound 1 or a salt thereof to a subject increases one or more anti-inflammatory proteins during mid-term treatment (e.g., 3-4 months of treatment with Compound 1). In certain embodiments, the administration of Compound 1 or a salt thereof to a subject increases one or more anti-inflammatory proteins during long-term treatment (e.g., 6-12 months of treatment with Compound 1).

Levels of CK in a subject increase when the subject is active as compared to when the subject is inactive (e.g., sleeping) and therefore CK is a potential metric for evaluating skeletal muscle breakdown caused by skeletal muscle contraction. In certain embodiments, Compound 1 or a salt thereof may be administered to a subject prior to mild, moderate, or strenuous activity to reduce or prevent skeletal muscle breakdown from the activity. Moderate to strenuous activity may be dependent on a subject's abilities and may include physical exercise that increases the heart rate by at least 20% or more, such as about 50% or more relative to the subject's resting heart rate. Examples of moderate to strenuous activity include walking, running, weightlifting, biking, swimming, hiking, etc.

In some embodiments, a muscle fast myosin (Type II) inhibitor, such as Compound 1, is administered to the subject in an amount sufficient to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, the creatine kinase activity level of the subject is evaluated following the first dose of said muscle fast myosin (Type II) inhibitor to the subject. In some embodiments, the evaluation is performed 1 month or more, 2 months or more, 3 months or more, 4 months or more, 6 months or more, 8 months or more, 12 months or more, 24 months or more, 18 months or more, or 24 months or more. In some embodiments, the creatine kinase activity level of the subject is evaluated following the first dose of said muscle fast myosin (Type II) inhibitor to the subject. In some embodiments, the evaluation is performed 1 month or more, 2 months or more, 3 months or more, 4 months or more, 6 months or more, 8 months or more, 12 months or more, 24 months or more, 18 months or more, or 24 months or more following the first dose of said muscle fast myosin (Type II) inhibitor to the subject. In some embodiments, said administering comprises administering of a first dose of said muscle fast myosin (Type II) inhibitor and one or more subsequent doses of said muscle fast myosin (Type II) inhibitor. In some embodiments, the one or more subsequent doses are higher than the first dose. In some embodiments, the one or more subsequent doses are lower than the first dose. In some embodiments, the one or more subsequent doses are determined based on the level of creatine kinase activity evaluated following the first dose of said muscle fast myosin (Type II) inhibitor to the subject. In some embodiments, the one or more subsequent doses are determined based on the level of creatine kinase activity evaluated following a previous dose of said muscle fast myosin (Type II) inhibitor to the subject. In some embodiments, the pre-treatment creatine kinase activity level is evaluated within 1 month prior to the first dose of said muscle fast myosin (Type II) inhibitor to the subject.

In certain embodiments, a muscle fast myosin (Type II) inhibitor, such as Compound 1 or a salt thereof, is administered prior to, during, or after moderate or strenuous activity to reduce or prevent skeletal muscle breakdown from the activity. Compound 1 or a salt thereof may reduce the subject's level of CK relative to the untreated subject performing the same activity. The level of CK may be measured in the peripheral blood of the subject during or after the activity. The administration of an inhibitor described herein may reduce the level of CK by 5% to 90% in an active subject relative to the untreated subject performing the same activity, thereby reducing, or preventing skeletal muscle breakdown from the activity. The administration of an inhibitor described herein may modulate the level of CK by about 5% to about 90% relative to the untreated subject performing the same activity, thereby reducing, or preventing skeletal muscle breakdown from the activity. The administration of an inhibitor described herein may reduce the level of CK by at least about 5% relative to the untreated subject performing the same activity thereby reducing or preventing skeletal muscle breakdown from the activity. The administration of an inhibitor described herein may modulate the level of CK by at most about 90% relative to the untreated subject performing the same activity. The administration of an inhibitor described herein may reduce the level of CK by about 5% to about 15%, about 5% to about 25%, about 5% to about 35%, about 5% to about 45%, about 5% to about 55%, about 5% to about 65%, about 5% to about 75%, about 5% to about 85%, about 5% to about 90%, about 15% to about 25%, about 15% to about 35%, about 15% to about 45%, about 15% to about 55%, about 15% to about 65%, about 15% to about 75%, about 15% to about 85%, about 15% to about 90%, about 25% to about 35%, about 25% to about 45%, about 25% to about 55%, about 25% to about 65%, about 25% to about 75%, about 25% to about 85%, about 25% to about 90%, about 35% to about 45%, about 35% to about 55%, about 35% to about 65%, about 35% to about 75%, about 35% to about 85%, about 35% to about 90%, about 45% to about 55%, about 45% to about 65%, about 45% to about 75%, about 45% to about 85%, about 45% to about 90%, about 55% to about 65%, about 55% to about 75%, about 55% to about 85%, about 55% to about 90%, about 65% to about 75%, about 65% to about 85%, about 65% to about 90%, about 75% to about 85%, about 75% to about 90%, or about 85% to about 90% relative to the untreated subject performing the same activity, thereby reducing or preventing skeletal muscle breakdown from the activity. The administration of an inhibitor described herein may modulate the level of CK by about 5%, about 15%, about 25%, about 35%, about 45%, about 55%, about 65%, about 75%, about 85%, or about 90% relative to the untreated subject performing the same activity, thereby reducing, or preventing skeletal muscle breakdown from the activity.

The treatment of muscle fast myosin (Type II) inhibitor, such as Compound 1, that lasts for varying durations may reduce the creatine kinase activity level of the subject. In some embodiments, a muscle fast myosin (Type II) inhibitor, such as Compound 1, is administered to the subject for 1 month to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 2 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 3 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 4 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 5 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 6 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 7 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 8 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 9 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 10 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 11 months to maintain the creatine kinase activity level or reduce the creatine kinase activity level of the subject relative to a pre-treatment creatine kinase activity level by 10% or more, 20% or more, 30% or more, 40% or more, or 50% or more. In some embodiments, a muscle fast myosin (Type II) inhibitor, such as Compound 1, is administered to the subject for 12 months to maintain the creatine kinase activity level or reduce the creatine kinase activity level of the subject relative to a pre-treatment creatine kinase activity level by 10% or more, 20% or more, 30% or more, 40% or more, or 50% or more.

In certain embodiments, a muscle fast myosin (Type II) inhibitor, such as Compound 1 or a salt thereof, is administered in an amount sufficient to maintain or increase the lean mass of a subject as determined by Dual Energy x-Ray absorptiometry (DXA) %. In some embodiments, the lean mass of the subject is increased by 5% or more, 10% or more, 15% or more, 20% or more, 30% or more, 40% or more, or 50% or more. In some embodiments, the lean mass of the subject is evaluated following the first dose of said muscle fast myosin (Type II) inhibitor to the subject. In some embodiments, the evaluation is performed 1 month or more, 2 months or more, 3 months or more, 4 months or more, 6 months or more, 8 months or more, 12 months or more, 24 months or more, 18 months or more, or 24 months or more following the first dose of said muscle fast myosin (Type II) inhibitor to the subject. In some embodiments, said administering comprises administering of a first dose of said muscle fast myosin (Type II) inhibitor and one or more subsequent doses of said muscle fast myosin (Type II) inhibitor. In some embodiments, the one or more subsequent doses are higher than the first dose. In some embodiments, the one or more subsequent doses are lower than the first dose. In some embodiments, the one or more subsequent doses are determined based on the level of lean mass evaluated following the first dose of said muscle fast myosin (Type II) inhibitor to the subject. In some embodiments, the one or more subsequent doses are determined based on the level of lean mass evaluated following one or more previous doses of said muscle fast myosin (Type II) inhibitor to the subject. In some embodiments, the pre-treatment lean mass level is evaluated within 1 month prior to the first dose of said muscle fast myosin (Type II) inhibitor to the subject.

The administration of Compound 1 or a salt thereof to a subject may modulate the levels of circulating fast skeletal muscle Troponin I (fS-TnI or TNNI2). The level of fS-TnI may be measured in the peripheral blood. The level of fS-TnI in the peripheral blood may increase after the administration of the inhibitor relative to pre-treatment level of fS-TnI for the subject. Alternatively, the level of fS-TnI in the peripheral blood may decrease after the administration of the inhibitor relative to pre-treatment level of fS-TnI for the subject. The administration of an inhibitor described herein may modulate the level of fS-TnI by 5% to 90% relative to pre-treatment level of fS-TnI for the subject. In some cases, the level of fS-TnI may be modulated by at least about 5% relative to pre-treatment level of fS-TnI of the subject. In some cases, the level of fS-TnI may be modulated by at most about 90% relative to pre-treatment level of fS-TnI of the subject. In some cases, the level of fS-TnI may be modulated by about 5% to about 15%, about 5% to about 25%, about 5% to about 35%, about 5% to about 45%, about 5% to about 55%, about 5% to about 65%, about 5% to about 75%, about 5% to about 85%, about 5% to about 90%, about 15% to about 25%, about 15% to about 35%, about 15% to about 45%, about 15% to about 55%, about 15% to about 65%, about 15% to about 75%, about 15% to about 85%, about 15% to about 90%, about 25% to about 35%, about 25% to about 45%, about 25% to about 55%, about 25% to about 65%, about 25% to about 75%, about 25% to about 85%, about 25% to about 90%, about 35% to about 45%, about 35% to about 55%, about 35% to about 65%, about 35% to about 75%, about 35% to about 85%, about 35% to about 90%, about 45% to about 55%, about 45% to about 65%, about 45% to about 75%, about 45% to about 85%, about 45% to about 90%, about 55% to about 65%, about 55% to about 75%, about 55% to about 85%, about 55% to about 90%, about 65% to about 75%, about 65% to about 85%, about 65% to about 90%, about 75% to about 85%, about 75% to about 90%, or about 85% to about 90% relative to pre-treatment level of fS-TnI of the subject. In some cases, the level of fS-TnI may be modulated by about 5%, about 15%, about 25%, about 35%, about 45%, about 55%, about 65%, about 75%, about 85%, or about 90% relative to pre-treatment level of fS-TnI of the subject.

Isoforms of troponin may be measured in a subject prior to and following the administration Compound 1 or a salt thereof. Inhibition of skeletal muscle contraction may not inhibit some isoforms of troponin, such as cardiac troponin I (cTnI) or slow skeletal troponin I (ssTnI). In some cases, the inhibition of skeletal muscle contraction may not appreciably inhibit cTnI or ssTnI. As used herein with regard to cTnI or ssTnI, the phrase not appreciably refers to the cTnI or ssTnI reduced by less than 10%, less than 8%, less than 6%, less than 4%, less than 2%, less than 1%, less than 0.5% or even less than 0.1% relative to the cTnI or ssTnI prior to the administration of the inhibitor.

In certain embodiments, a muscle fast myosin (Type II) inhibitor, such as Compound 1 or a salt thereof, is administered in an amount sufficient maintain or reduce the TNNI2 concentration of the subject relative to a pre-treatment TNNI2 concentration. In some embodiments, the TNNI2 of the subject is reduced by 5% or more, 10%, 15%, 20%, 30%, 40%, 50%, 60%, 70%, 80%, 90% or more. In some embodiments, the TNNI2 of the subject is evaluated following the first dose of said muscle fast myosin (Type II) inhibitor to the subject. In some embodiments, the evaluation is performed 1 month or more, 2 months or more, 3 months or more, 4 months or more, 6 months or more, 8 months or more, 12 months or more, 24 months or more, 18 months or more, or 24 months or more following the first dose of said muscle fast myosin (Type II) inhibitor to the subject. In some embodiments, said administering comprises administering of a first dose of said muscle fast myosin (Type II) inhibitor and one or more subsequent doses of said muscle fast myosin (Type II) inhibitor. In some embodiments, the one or more subsequent doses are higher than the first dose. In some embodiments, the one or more subsequent doses are lower than the first dose. In some embodiments, the one or more subsequent doses are determined based on the level of TNNI2 evaluated following the first dose of said muscle fast myosin (Type II) inhibitor to the subject. In some embodiments, the one or more subsequent doses are determined based on the level of TNNI2 evaluated following one or more previous doses of said muscle fast myosin (Type II) inhibitor to the subject. In some embodiments, the pre-treatment TNNI2 level is evaluated within 1 month prior to the first dose of said muscle fast myosin (Type II) inhibitor to the subject.

The treatment of muscle fast myosin (Type II) inhibitor, such as Compound 1, that lasts for varying durations may reduce the TNNI2 level of the subject. In some embodiments, a muscle fast myosin (Type II) inhibitor, such as Compound 1, is administered to the subject for 1 month to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 2 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 3 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 4 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 5 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 6 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 7 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 8 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 9 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 10 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 11 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more. In some embodiments, a muscle fast myosin (Type II) inhibitor, such as Compound 1, is administered to the subject for 12 months to maintain the TNNI2 level or reduce the TNNI2 level of the subject relative to a pre-treatment TNNI2 level by 10% or more, 20% or more, 30% or more, 40% or more, 50% or more, 60% or more, 70% or more, 80% or more, or 90% or more.

Elevated levels of serum myoglobin may be found in patients with several types of neuromuscular conditions, so myoglobin may be a potential metric for evaluating progress of the neuromuscular conditions. In some embodiments, a muscle fast myosin (Type II) inhibitor, such as Compound 1, is administered to the subject in an amount sufficient to maintain the myoglobin level or reduce the myoglobin level of the subject relative to a pre-treatment myoglobin level by 5% or more, by 10% or more, by 15% or more, 20% or more, by 25% or more, 30% or more, by 35% or more, 40% or more, by 45% or more, or 50% or more. In some embodiments, a muscle fast myosin (Type II) inhibitor, such as Compound 1, is administered to the subject in an amount sufficient to maintain the myoglobin level or reduce the myoglobin level of the subject relative to a pre-treatment myoglobin level by about 5% to about 15%, about 5% to about 25%, about 5% to about 35%, about 5% to about 45%, about 5% to about 55%, about 5% to about 65%, about 5% to about 75%, about 5% to about 85%, about 5% to about 90%, about 15% to about 25%, about 15% to about 35%, about 15% to about 45%, about 15% to about 55%, about 15% to about 65%, about 15% to about 75%, about 15% to about 85%, about 15% to about 90%, about 25% to about 35%, about 25% to about 45%, about 25% to about 55%, about 25% to about 65%, about 25% to about 75%, about 25% to about 85%, about 25% to about 90%, about 35% to about 45%, about 35% to about 55%, about 35% to about 65%, about 35% to about 75%, about 35% to about 85%, about 35% to about 90%, about 45% to about 55%, about 45% to about 65%, about 45% to about 75%, about 45% to about 85%, about 45% to about 90%, about 55% to about 65%, about 55% to about 75%, about 55% to about 85%, about 55% to about 90%, about 65% to about 75%, about 65% to about 85%, about 65% to about 90%, about 75% to about 85%, about 75% to about 90%, or about 85% to about 90%. In some embodiments, a muscle fast myosin (Type II) inhibitor, such as Compound 1, is administered to the subject in an amount sufficient to maintain the myoglobin level or reduce the myoglobin level of the subject relative to a pre-treatment myoglobin level by about 5%, about 15%, about 25%, about 35%, about 45%, about 55%, about 65%, about 75%, about 85%, or about 90%.

In some embodiments, the myoglobin level of the subject is evaluated following the first dose of said muscle fast myosin (Type II) inhibitor to the subject. In some embodiments, the evaluation is performed 1 month or more, 2 months or more, 3 months or more, 4 months or more, 6 months or more, 8 months or more, 12 months or more, 24 months or more, 18 months or more, or 24 months or more following the first dose of said muscle fast myosin (Type II) inhibitor to the subject. In some embodiments, said administering comprises administering of a first dose of said muscle fast myosin (Type II) inhibitor and one or more subsequent doses of said muscle fast myosin (Type II) inhibitor. In some embodiments, the one or more subsequent doses are higher than the first dose. In some embodiments, the one or more subsequent doses are lower than the first dose. In some embodiments, the one or more subsequent doses are determined based on the level of myoglobin evaluated following the first dose of said muscle fast myosin (Type II) inhibitor to the subject. In some embodiments, the one or more subsequent doses are determined based on the level of myoglobin evaluated following one or more previous dose of said muscle fast myosin (Type II) inhibitor to the subject. In some embodiments, the pre-treatment myoglobin level is evaluated within 1 month prior to the first dose of said muscle fast myosin (Type II) inhibitor to the subject.

In certain embodiments, a muscle fast myosin (Type II) inhibitor, such as Compound 1 or a salt thereof, is administered prior to, during, or after moderate or strenuous activity to reduce or prevent skeletal muscle breakdown from the activity. Compound 1 or a salt thereof may reduce the subject's level of myoglobin relative to the untreated subject performing the same activity. The level of myoglobin may be measured in the peripheral blood of the subject during or after the activity. The administration of an inhibitor described herein may reduce the level of myoglobin by 5% to 90% in an active subject relative to the untreated subject performing the same activity, thereby reducing, or preventing skeletal muscle breakdown from the activity. The administration of an inhibitor described herein may modulate the level of myoglobin by about 5% to about 90% relative to the untreated subject performing the same activity, thereby reducing, or preventing skeletal muscle breakdown from the activity. The administration of an inhibitor described herein may reduce the level of myoglobin by at least about 5% relative to the untreated subject performing the same activity thereby reducing or preventing skeletal muscle breakdown from the activity. The administration of an inhibitor described herein may modulate the level of myoglobin by at most about 90% relative to the untreated subject performing the same activity. The administration of an inhibitor described herein may reduce the level of myoglobin by about 5% to about 15%, about 5% to about 25%, about 5% to about 35%, about 5% to about 45%, about 5% to about 55%, about 5% to about 65%, about 5% to about 75%, about 5% to about 85%, about 5% to about 90%, about 15% to about 25%, about 15% to about 35%, about 15% to about 45%, about 15% to about 55%, about 15% to about 65%, about 15% to about 75%, about 15% to about 85%, about 15% to about 90%, about 25% to about 35%, about 25% to about 45%, about 25% to about 55%, about 25% to about 65%, about 25% to about 75%, about 25% to about 85%, about 25% to about 90%, about 35% to about 45%, about 35% to about 55%, about 35% to about 65%, about 35% to about 75%, about 35% to about 85%, about 35% to about 90%, about 45% to about 55%, about 45% to about 65%, about 45% to about 75%, about 45% to about 85%, about 45% to about 90%, about 55% to about 65%, about 55% to about 75%, about 55% to about 85%, about 55% to about 90%, about 65% to about 75%, about 65% to about 85%, about 65% to about 90%, about 75% to about 85%, about 75% to about 90%, or about 85% to about 90% relative to the untreated subject performing the same activity, thereby reducing or preventing skeletal muscle breakdown from the activity. The administration of an inhibitor described herein may modulate the level of myoglobin by about 5%, about 15%, about 25%, about 35%, about 45%, about 55%, about 65%, about 75%, about 85%, or about 90% relative to the untreated subject performing the same activity, thereby reducing, or preventing skeletal muscle breakdown from the activity.

In certain embodiments, a muscle fast myosin (Type II) inhibitor, such as Compound 1 or a salt thereof, is administered in an amount sufficient maintain or reduce the TNNI2 concentration of the subject relative to a pre-treatment TNNI2 concentration. In some embodiments, the TNNI2 concentration is reduced by 120 ng/mL or more, 100 ng/mL or more, 80 ng/mL or more, 65 ng/mL or more, 50 ng/mL or more, 45 ng/mL or more, 40 ng/mL or more, 35 ng/mL or more, 30 ng/mL or more, 25 ng/mL or more, 20 ng/mL or more, 15 ng/mL or more, 10 ng/mL or more, 5 ng/mL or more. In some embodiments, the TNNI2 concentration is reduced by between about 5 to 120 ng/mL, between about 10 to 120 ng/mL, between about 20 to 100 ng/mL, 5 to 50 ng/mL, between about 5 to 45 ng/mL, between about 5 to 40 ng/mL, between about 5 to 35 ng/mL, between about 5 to 30 ng/mL, between about 5 to 25 ng/mL, between about 5 to 20 ng/mL, between about 5 to 15 ng/mL, between about 5 to 10 ng/mL, between about 10 to 40 ng/mL, between about 20 to 40 ng/mL, between about 25 to 35 ng/mL, between about 15 to 35 ng/mL, between about 10 to 20 ng/mL, between about 40 to 50 ng/mL, or between about 20 to 35 ng/mL.

In certain embodiments, a muscle fast myosin (Type II) inhibitor, such as Compound 1 or a salt thereof, is administered in an amount sufficient maintain or reduce the TNNI2 concentration of the subject to about 1 ng/mL to about 50 ng/mL. In certain embodiments, a muscle fast myosin (Type II) inhibitor, such as Compound 1 or a salt thereof, is administered in an amount sufficient maintain or reduce the TNNI2 concentration of the subject to about 1 ng/mL to about 3 ng/mL, about 1 ng/mL to about 5 ng/mL, about 1 ng/mL to about 8 ng/mL, about 1 ng/mL to about 10 ng/mL, about 1 ng/mL to about 15 ng/mL, about 1 ng/mL to about 20 ng/mL, about 1 ng/mL to about 25 ng/mL, about 1 ng/mL to about 30 ng/mL, about 1 ng/mL to about 35 ng/mL, about 1 ng/mL to about 40 ng/mL, about 1 ng/mL to about 50 ng/mL, about 3 ng/mL to about 5 ng/mL, about 3 ng/mL to about 8 ng/mL, about 3 ng/mL to about 10 ng/mL, about 3 ng/mL to about 15 ng/mL, about 3 ng/mL to about 20 ng/mL, about 3 ng/mL to about 25 ng/mL, about 3 ng/mL to about 30 ng/mL, about 3 ng/mL to about 35 ng/mL, about 3 ng/mL to about 40 ng/mL, about 3 ng/mL to about 50 ng/mL, about 5 ng/mL to about 8 ng/mL, about 5 ng/mL to about 10 ng/mL, about 5 ng/mL to about 15 ng/mL, about 5 ng/mL to about 20 ng/mL, about 5 ng/mL to about 25 ng/mL, about 5 ng/mL to about 30 ng/mL, about 5 ng/mL to about 35 ng/mL, about 5 ng/mL to about 40 ng/mL, about 5 ng/mL to about 50 ng/mL, about 8 ng/mL to about 10 ng/mL, about 8 ng/mL to about 15 ng/mL, about 8 ng/mL to about 20 ng/mL, about 8 ng/mL to about 25 ng/mL, about 8 ng/mL to about 30 ng/mL, about 8 ng/mL to about 35 ng/mL, about 8 ng/mL to about 40 ng/mL, about 8 ng/mL to about 50 ng/mL, about 10 ng/mL to about 15 ng/mL, about 10 ng/mL to about 20 ng/mL, about 10 ng/mL to about 25 ng/mL, about 10 ng/mL to about 30 ng/mL, about 10 ng/mL to about 35 ng/mL, about 10 ng/mL to about 40 ng/mL, about 10 ng/mL to about 50 ng/mL, about 15 ng/mL to about 20 ng/mL, about 15 ng/mL to about 25 ng/mL, about 15 ng/mL to about 30 ng/mL, about 15 ng/mL to about 35 ng/mL, about 15 ng/mL to about 40 ng/mL, about 15 ng/mL to about 50 ng/mL, about 20 ng/mL to about 25 ng/mL, about 20 ng/mL to about 30 ng/mL, about 20 ng/mL to about 35 ng/mL, about 20 ng/mL to about 40 ng/mL, about 20 ng/mL to about 50 ng/mL, about 25 ng/mL to about 30 ng/mL, about 25 ng/mL to about 35 ng/mL, about 25 ng/mL to about 40 ng/mL, about 25 ng/mL to about 50 ng/mL, about 30 ng/mL to about 35 ng/mL, about 30 ng/mL to about 40 ng/mL, about 30 ng/mL to about 50 ng/mL, about 35 ng/mL to about 40 ng/mL, about 35 ng/mL to about 50 ng/mL, or about 40 ng/mL to about 50 ng/mL. In certain embodiments, a muscle fast myosin (Type II) inhibitor, such as Compound 1 or a salt thereof, is administered in an amount sufficient maintain or reduce the TNNI2 concentration of the subject to about 1 ng/mL, about 3 ng/mL, about 5 ng/mL, about 8 ng/mL, about 10 ng/mL, about 15 ng/mL, about 20 ng/mL, about 25 ng/mL, about 30 ng/mL, about 35 ng/mL, about 40 ng/mL, or about 50 ng/mL. In certain embodiments, a muscle fast myosin (Type II) inhibitor, such as Compound 1 or a salt thereof, is administered in an amount sufficient maintain or reduce the TNNI2 concentration of the subject to at least about 1 ng/mL, about 3 ng/mL, about 5 ng/mL, about 8 ng/mL, about 10 ng/mL, about 15 ng/mL, about 20 ng/mL, about 25 ng/mL, about 30 ng/mL, about 35 ng/mL, or about 40 ng/mL. In certain embodiments, a muscle fast myosin (Type II) inhibitor, such as Compound 1 or a salt thereof, is administered in an amount sufficient maintain or reduce the TNNI2 concentration of the subject to at most about 3 ng/mL, about 5 ng/mL, about 8 ng/mL, about 10 ng/mL, about 15 ng/mL, about 20 ng/mL, about 25 ng/mL, about 30 ng/mL, about 35 ng/mL, about 40 ng/mL, or about 50 ng/mL.

In some embodiments, a muscle fast myosin (Type II) inhibitor, such as Compound 1, is administered to the subject in an amount sufficient to reduce serum creatine kinase (CK) levels by 10 U/L or more, 20 U/L or more, 30 U/L or more, 40 U/L or more, 50 U/L or more, 60 U/L or more, 70 U/L or more, 80 U/L or more, 90 U/L or more, 100 U/L or more, 200 U/L or more, 300 U/L or more, 400 U/L or more, 500 U/L or more, 600 U/L or more, 700 U/L or more, 800 U/L or more, 900 U/L or more, 1000 U/L or more, 1100 U/L or more, 1200 U/L or more, 1300 U/L or more, 1400 U/L or more, 1500 U/L or more. In some embodiments, CK levels are reduced by between about 500 U/L and 1500 U/L, about 800 U/L and 1400 U/L, about 1000 U/L and 1300 U/L, or about 1100 U/L and 1300 U/L.

In certain embodiments, a muscle fast myosin (Type II) inhibitor, such as Compound 1 or a salt thereof, is administered in an amount sufficient maintain or reduce the serum creatine kinase (CK) levels of the subject to about 400 U/L to about 1,500 U/L. In certain embodiments, a muscle fast myosin (Type II) inhibitor, such as Compound 1 or a salt thereof, is administered in an amount sufficient maintain or reduce the serum creatine kinase (CK) levels of the subject to about 400 U/L to about 500 U/L, about 400 U/L to about 600 U/L, about 400 U/L to about 700 U/L, about 400 U/L to about 800 U/L, about 400 U/L to about 900 U/L, about 400 U/L to about 1,000 U/L, about 400 U/L to about 1,100 U/L, about 400 U/L to about 1,200 U/L, about 400 U/L to about 1,300 U/L, about 400 U/L to about 1,400 U/L, about 400 U/L to about 1,500 U/L, about 500 U/L to about 600 U/L, about 500 U/L to about 700 U/L, about 500 U/L to about 800 U/L, about 500 U/L to about 900 U/L, about 500 U/L to about 1,000 U/L, about 500 U/L to about 1,100 U/L, about 500 U/L to about 1,200 U/L, about 500 U/L to about 1,300 U/L, about 500 U/L to about 1,400 U/L, about 500 U/L to about 1,500 U/L, about 600 U/L to about 700 U/L, about 600 U/L to about 800 U/L, about 600 U/L to about 900 U/L, about 600 U/L to about 1,000 U/L, about 600 U/L to about 1,100 U/L, about 600 U/L to about 1,200 U/L, about 600 U/L to about 1,300 U/L, about 600 U/L to about 1,400 U/L, about 600 U/L to about 1,500 U/L, about 700 U/L to about 800 U/L, about 700 U/L to about 900 U/L, about 700 U/L to about 1,000 U/L, about 700 U/L to about 1,100 U/L, about 700 U/L to about 1,200 U/L, about 700 U/L to about 1,300 U/L, about 700 U/L to about 1,400 U/L, about 700 U/L to about 1,500 U/L, about 800 U/L to about 900 U/L, about 800 U/L to about 1,000 U/L, about 800 U/L to about 1,100 U/L, about 800 U/L to about 1,200 U/L, about 800 U/L to about 1,300 U/L, about 800 U/L to about 1,400 U/L, about 800 U/L to about 1,500 U/L, about 900 U/L to about 1,000 U/L, about 900 U/L to about 1,100 U/L, about 900 U/L to about 1,200 U/L, about 900 U/L to about 1,300 U/L, about 900 U/L to about 1,400 U/L, about 900 U/L to about 1,500 U/L, about 1,000 U/L to about 1,100 U/L, about 1,000 U/L to about 1,200 U/L, about 1,000 U/L to about 1,300 U/L, about 1,000 U/L to about 1,400 U/L, about 1,000 U/L to about 1,500 U/L, about 1,100 U/L to about 1,200 U/L, about 1,100 U/L to about 1,300 U/L, about 1,100 U/L to about 1,400 U/L, about 1,100 U/L to about 1,500 U/L, about 1,200 U/L to about 1,300 U/L, about 1,200 U/L to about 1,400 U/L, about 1,200 U/L to about 1,500 U/L, about 1,300 U/L to about 1,400 U/L, about 1,300 U/L to about 1,500 U/L, or about 1,400 U/L to about 1,500 U/L. In certain embodiments, a muscle fast myosin (Type II) inhibitor, such as Compound 1 or a salt thereof, is administered in an amount sufficient maintain or reduce the serum creatine kinase (CK) levels of the subject to about 400 U/L, about 500 U/L, about 600 U/L, about 700 U/L, about 800 U/L, about 900 U/L, about 1,000 U/L, about 1,100 U/L, about 1,200 U/L, about 1,300 U/L, about 1,400 U/L, or about 1,500 U/L. In certain embodiments, a muscle fast myosin (Type II) inhibitor, such as Compound 1 or a salt thereof, is administered in an amount sufficient maintain or reduce the serum creatine kinase (CK) levels of the subject to at least about 400 U/L, about 500 U/L, about 600 U/L, about 700 U/L, about 800 U/L, about 900 U/L, about 1,000 U/L, about 1,100 U/L, about 1,200 U/L, about 1,300 U/L, or about 1,400 U/L. In certain embodiments, a muscle fast myosin (Type II) inhibitor, such as Compound 1 or a salt thereof, is administered in an amount sufficient maintain or reduce the serum creatine kinase (CK) levels of the subject to at most about 500 U/L, about 600 U/L, about 700 U/L, about 800 U/L, about 900 U/L, about 1,000 U/L, about 1,100 U/L, about 1,200 U/L, about 1,300 U/L, about 1,400 U/L, or about 1,500 U/L.

In some embodiments, a muscle fast myosin (Type II) inhibitor, such as Compound 1, is administered to the subject in an amount sufficient to reduce myoglobin levels by 10 ng/mL or more, 20 ng/mL or more, 30 ng/mL or more, 40 ng/mL or more, 50 ng/mL or more, 60 ng/mL or more, 70 ng/mL or more, 80 ng/mL or more, 90 ng/mL or more, 100 ng/mL or more, 200 ng/mL or more, 300 ng/mL or more, 400 ng/mL or more, 500 ng/mL or more, 600 ng/mL or more, 700 ng/mL or more, 800 ng/mL or more, 900 ng/mL or more, 1000 ng/mL or more.

In certain embodiments, a muscle fast myosin (Type II) inhibitor, such as Compound 1 or a salt thereof, is administered in an amount sufficient maintain or reduce myoglobin levels of the subject to about 100 U/L to about 400 U/L. In certain embodiments, a muscle fast myosin (Type II) inhibitor, such as Compound 1 or a salt thereof, is administered in an amount sufficient maintain or reduce myoglobin levels of the subject to about 100 U/L to about 150 U/L, about 100 U/L to about 200 U/L, about 100 U/L to about 250 U/L, about 100 U/L to about 300 U/L, about 100 U/L to about 350 U/L, about 100 U/L to about 400 U/L, about 150 U/L to about 200 U/L, about 150 U/L to about 250 U/L, about 150 U/L to about 300 U/L, about 150 U/L to about 350 U/L, about 150 U/L to about 400 U/L, about 200 U/L to about 250 U/L, about 200 U/L to about 300 U/L, about 200 U/L to about 350 U/L, about 200 U/L to about 400 U/L, about 250 U/L to about 300 U/L, about 250 U/L to about 350 U/L, about 250 U/L to about 400 U/L, about 300 U/L to about 350 U/L, about 300 U/L to about 400 U/L, or about 350 U/L to about 400 U/L. In certain embodiments, a muscle fast myosin (Type II) inhibitor, such as Compound 1 or a salt thereof, is administered in an amount sufficient maintain or reduce myoglobin levels of the subject to about 100 U/L, about 150 U/L, about 200 U/L, about 250 U/L, about 300 U/L, about 350 U/L, or about 400 U/L. In certain embodiments, a muscle fast myosin (Type II) inhibitor, such as Compound 1 or a salt thereof, is administered in an amount sufficient maintain or reduce myoglobin levels of the subject to at least about 100 U/L, about 150 U/L, about 200 U/L, about 250 U/L, about 300 U/L, or about 350 U/L. In certain embodiments, a muscle fast myosin (Type II) inhibitor, such as Compound 1 or a salt thereof, is administered in an amount sufficient maintain or reduce myoglobin levels of the subject to at most about 150 U/L, about 200 U/L, about 250 U/L, about 300 U/L, about 350 U/L, or about 400 U/L.

In some embodiments, a muscle fast myosin (Type II) inhibitor, such as Compound 1, is administered to the subject in an amount sufficient to increase serum creatinine levels by 1 mg/dL or more, 0.8 mg/dL or more, 0.6 mg/dL or more, 0.5 mg/dL or more, 0.4 mg/dL or more, 0.3 mg/dL or more, 0.2 mg/dL or more, or 0.1 mg/dL or more.

The administration of Compound 1 or a salt thereof may reduce involuntary muscle contractions. Involuntary muscle contractions may be reduced by 20% to 90% relative to involuntary muscle contractions prior to the administration of the inhibitor. In some cases, involuntary muscle contractions may be reduced by at least about 20% relative to pre-treatment involuntary muscle contractions. In some cases, involuntary muscle contractions may be reduced by at most about 90% relative to pre-treatment involuntary muscle contractions. In some cases, involuntary muscle contractions may be reduced by about 20% to about 25%, about 20% to about 30%, about 20% to about 40%, about 20% to about 50%, about 20% to about 70%, about 20% to about 75%, about 20% to about 80%, about 20% to about 85%, about 20% to about 90%, about 25% to about 30%, about 25% to about 40%, about 25% to about 50%, about 25% to about 70%, about 25% to about 75%, about 25% to about 80%, about 25% to about 85%, about 25% to about 90%, about 30% to about 40%, about 30% to about 50%, about 30% to about 70%, about 30% to about 75%, about 30% to about 80%, about 30% to about 85%, about 30% to about 90%, about 40% to about 50%, about 40% to about 70%, about 40% to about 75%, about 40% to about 80%, about 40% to about 85%, about 40% to about 90%, about 50% to about 70%, about 50% to about 75%, about 50% to about 80%, about 50% to about 85%, about 50% to about 90%, about 70% to about 75%, about 70% to about 80%, about 70% to about 85%, about 70% to about 90%, about 75% to about 80%, about 75% to about 85%, about 75% to about 90%, about 80% to about 85%, about 80% to about 90%, or about 85% to about 90% relative to pre-treatment involuntary muscle contractions. In some cases, involuntary muscle contractions may be reduced by about 20%, about 25%, about 30%, about 40%, about 50%, about 70%, about 75%, about 80%, about 85%, or about 90% relative to pre-treatment involuntary muscle contractions.

Compound 1 or a salt thereof may be used to improve activities of daily living (ADL) or habitual physical activity in a subject as mature, functional undamaged muscle may be restored. Examples of ADL or habitual activities include but are not limited to stair climb, time to get up, timed chair rise, habitual walk speed, North Star Ambulatory Assessment (NSAA), incremental/endurance shuttle walk and 6 minute walk distance tests. ADL or habitual physical activity levels or capacity may be measured prior to and following the administration of a skeletal muscle inhibitor. Inhibition of skeletal muscle contraction may not affect ADL or habitual physical activity. In some cases, the inhibition of skeletal muscle contraction may not appreciably affect ADL or habitual physical activity. As used herein with regard to ADL or habitual physical activity, the phrase not appreciably refers to the level of ADL or habitual activity reduced by less than 20%, less than 15%, less than 10%, less than 8%, less than 6%, less than 4%, less than 2%, less than 1%, less than 0.5% or even less than 0.1% relative to the ADL or habitual activity prior to the administration of the inhibitor.

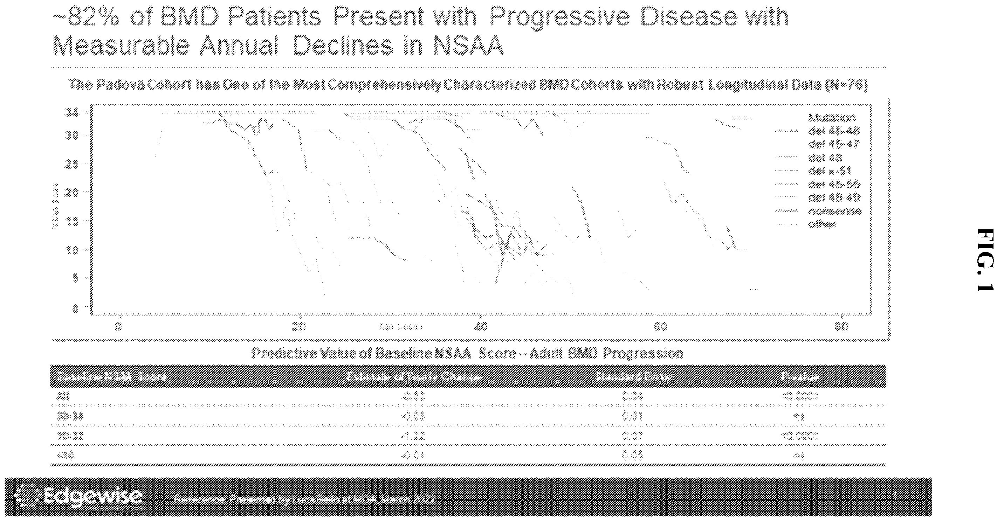

In some embodiments, the administration of Compound 1 or a salt thereof affect ADL or habitual physical activity of the subject. In some embodiments, the North Star Ambulatory Assessment is used to evaluate the progression of a neuromuscular disease in the subject over time. For example, FIG. 1 shows the NSAA scores of human subjects diagnosed with Becker Muscular Dystrophy over time. On average there is an estimated average yearly decrease of - 1.22 for patients 10 to 32 years if age. In some embodiments, administration of Compound 1 or a salt thereof slows the rate at which the neuromuscular disease progresses as determined by NSAA scores. In some embodiments, administration of Compound 1 or a salt increases the average yearly NSAA score to greater than -1.22. In some embodiments, administration of Compound 1 or a salt increases the average yearly NSAA score to greater than -1. In some embodiments, administration of Compound 1 or a salt increases the average yearly NSAA score to greater than -0.5. In some embodiments, administration of Compound 1 or a salt increases the average yearly NSAA score to greater than -0.4. In some embodiments, administration of Compound 1 or a salt increases the average yearly NSAA score to greater than -0.3. In some embodiments, administration of Compound 1 or a salt increases the average yearly NSAA score to greater than -0.2. In some embodiments, administration of Compound 1 or a salt increases the average yearly NSAA score to greater than -0.1. In some embodiments, administration of Compound 1 or a salt increases the average yearly NSAA score to greater than 0. In some embodiments, administration of Compound 1 or a salt increases the average yearly NSAA score to greater than 0.1. In some embodiments, administration of Compound 1 or a salt increases the average yearly NSAA score to greater than 0.2. In some embodiments, administration of Compound 1 or a salt increases the average yearly NSAA score to greater than 0.3. In some embodiments, administration of Compound 1 or a salt increases the average yearly NSAA score to greater than 0.4. In some embodiments, administration of Compound 1 or a salt increases the average yearly NSAA score to greater than 0.5. In some embodiments, administration of Compound 1 or a salt increases the average yearly NSAA score to greater than 1.0. In some embodiments, administration of Compound 1 or a salt increases the average yearly NSAA score to greater than 2.0. In some embodiments, administration of Compound 1 or a salt increases the average yearly NSAA score to greater than 3.0. In some embodiments, administration of Compound 1 or a salt increases the average yearly NSAA score to greater than 4.0. In some embodiments, administration of Compound 1 or a salt increases the average yearly NSAA score to greater than 5.0. In some embodiments, administration of Compound 1 or a salt increases the average yearly NSAA score to greater than 10.0. In some embodiments, administration of Compound 1 or a salt increases the average monthly NSAA score to at least -0.1, 0, 0.1, 0.2, 0.3, 0.4, 0.5, 0.6, 0.7, 0.8, 0.9, 1.0, 2.0, 3.0, 4.0, 5.0, or 10.0.

In some embodiments, administration of a muscle fast myosin (Type II) inhibitor increases the NSAA score of the patient to greater than about -1.2 to about 10. In some embodiments, administration of a muscle fast myosin (Type II) inhibitor increases the NSAA score of the patient to greater than about -1.2 to 2. In some embodiments, administration of a muscle fast myosin (Type II) inhibitor increases the NSAA score of the patient to greater than about -1.2 to 1.5. In some embodiments, administration of a muscle fast myosin (Type II) inhibitor increases the NSAA score of the patient to greater than about -1.2 to 1. In some embodiments, administration of a muscle fast myosin (Type II) inhibitor increases the NSAA score of the patient to greater than about -1.2 to 0.5. In some embodiments, administration of a muscle fast myosin (Type II) inhibitor increases the NSAA score of the patient to greater than about -1.2 to 0. In some embodiments, administration of a muscle fast myosin (Type II) inhibitor increases the NSAA score of the patient to greater than about -1.2 to -0.5. In some embodiments, administration of a muscle fast myosin (Type II) inhibitor increases the NSAA score of the patient to greater than about -1. to 2. In some embodiments, administration of a muscle fast myosin (Type II) inhibitor increases the NSAA score of the patient to greater than about -0.5 to 2. In some embodiments, administration of a muscle fast myosin (Type II) inhibitor increases the NSAA score of the patient to greater than about 0 to 5. In some embodiments, administration of a muscle fast myosin (Type II) inhibitor increases the NSAA score of the patient to greater than about -1. to 5. In some embodiments, administration of a muscle fast myosin (Type II) inhibitor increases the NSAA score of the patient to greater than about -0.5 to 4. In some embodiments, administration of a muscle fast myosin (Type II) inhibitor increases the NSAA score of the patient to greater than about 0 to 3. In some embodiments, administration of a muscle fast myosin (Type II) inhibitor increases the NSAA score of the patient to greater than about 2 to 10. In some embodiments, administration of a muscle fast myosin (Type II) inhibitor increases the NSAA score of the patient to greater than about -1.2, about -1, about -0.75, about -0.5, about - 0.25, about 0, about 0.25, about 0.5, about 0.75, about 1, about 1.5, or about 2, about 3, about 4, about 5, about 6, about 7, about 8, or about 10.

In some embodiments, the NSAA score of a patient increases following the administration of a muscle myosin inhibitor, such as Compound 1. In some embodiments, the muscle fast myosin (Type II) inhibitor is administered for about 1 month to about 24 months. In some embodiments, the NSAA score of a patient increases following the administration of a muscle myosin inhibitor, such as Compound 1. In some embodiments, the muscle fast myosin (Type II) inhibitor is administered for about 2 month to about 12 months. In some embodiments, the NSAA score of a patient increases following the administration of a muscle myosin inhibitor, such as Compound 1. In some embodiments, the muscle fast myosin (Type II) inhibitor is administered for about 4 month to about 12 months. In some embodiments, the NSAA score of a patient increases following the administration of a muscle myosin inhibitor, such as Compound 1. In some embodiments, the muscle fast myosin (Type II) inhibitor is administered for about 6 month to about 18 months. In some embodiments, the NSAA score of a patient increases following the administration of a muscle myosin inhibitor, such as Compound 1. In some embodiments, the muscle fast myosin (Type II) inhibitor is administered for about 2 month to about 6 months. In some embodiments, the muscle fast myosin (Type II) inhibitor is administered for about 1 month, about 2 months, about 3 months, about 4 months, about 5 months, about 6 months, about 7 months, about 8 months, about 10 months, about 12 months, about 18 months, or about 24 months. In some embodiments, the muscle fast myosin (Type II) inhibitor is administered for at least about 1 month, about 2 months, about 3 months, about 4 months, about 5 months, about 6 months, about 7 months, about 8 months, about 10 months, about 12 months, or about 18 months. In some embodiments, the muscle fast myosin (Type II) inhibitor is administered for at most about 2 months, about 3 months, about 4 months, about 5 months, about 6 months, about 7 months, about 8 months, about 10 months, about 12 months, about 18 months, or about 24 months.

Administering to a subject a muscle fast myosin (Type II) inhibitor, such as Compound 1, in an amount sufficient to maintain or improve one or more functional measures of the subject. In some embodiments, functional measures are selected from 100 meter timed test velocity relative to a pre-treatment 100 meter timed test velocity; 4-stair climb time velocity relative to a pre-treatment 4-stair climb time velocity; 10 meter walk/run velocity relative to a pre-treatment 10 meter walk/run velocity; max elbow flexion strength relative to a pre-treatment max elbow flexion strength; max knee extension strength relative to a pre-treatment max knee extension strength; and max-grip strength relative to a pre-treatment max-grip strength value. In some embodiments, functional measures include evaluation of the patients ability to: perform Gower's maneuver, walk, stand up from a chair, stand on one leg, climb a box with a leg step, descend a box with a leg step, sit down, rise from the floor, lift head, stand on heels, jump with two feet, hop with on foot, or run.

In some embodiments, the muscle fast myosin (Type II) inhibitor is administered in an amount sufficient to maintain the one or more functional measures of the subject relative to the pre-treatment value. In some embodiments, the muscle fast myosin (Type II) inhibitor is administered in an amount sufficient to improve the one or more functional measures of the subject relative to the pre-treatment value. In some embodiments, the one or more functional measures is maintained to within 20%, 30%, 40%, or 50% of the pre-treatment value. In some embodiments, the one or more functional measures are maintained for 2 months or more, for 4 months or more, for 6 months or more, for 8 months or more, for 12 months or more, for 18 months or more, for 24 months or more, or for 36 months or more.

Skeletal muscle contraction or force in a subject may be measured prior to and following the administration of Compound 1 or a salt thereof. Such measurements may be performed to generate a dose response curve for Compound 1 or a salt thereof. Dosage of Compound 1 or a salt thereof may be adjusted by about 5% to 50% relative to a dose that reduces type II skeletal muscle contraction by 90%. In some cases, dosage of the skeletal muscle contraction inhibitor may be adjusted by at least about 5% relative to a dose that reduces type II skeletal muscle contraction by 90%. In some cases, dosage of the skeletal muscle contraction inhibitor may be adjusted by at most about 50% relative to a dose that reduces type II skeletal muscle contraction by 90%. In some cases, dosage of the skeletal muscle contraction inhibitor may be adjusted by about 5 % to about 10 %, about 5 % to about 15 %, about 5 % to about 20 %, about 5 % to about 25 %, about 5 % to about 30 %, about 5 % to about 35 %, about 5 % to about 40 %, about 5 % to about 50 %, about 10 % to about 15 %, about 10 % to about 20 %, about 10 % to about 25 %, about 10 % to about 30 %, about 10 % to about 35 %, about 10 % to about 40 %, about 10 % to about 50 %, about 15 % to about 20 %, about 15 % to about 25 %, about 15 % to about 30 %, about 15 % to about 35 %, about 15 % to about 40 %, about 15 % to about 50 %, about 20 % to about 25 %, about 20 % to about 30 %, about 20 % to about 35 %, about 20 % to about 40 %, about 20 % to about 50 %, about 25 % to about 30 %, about 25 % to about 35 %, about 25 % to about 40 %, about 25 % to about 50 %, about 30 % to about 35 %, about 30 % to about 40 %, about 30 % to about 50 %, about 35 % to about 40 %, about 35 % to about 50 %, or about 40 % to about 50 % relative to a dose that reduces type II skeletal muscle contraction by 90%. In some cases, dosage of the skeletal muscle contraction inhibitor may be adjusted by about 10%, about 12%, about 15%, about 18%, about 20%, about 25%, about 30%, about 35%, about 40%, about 45% or about 50% relative to a dose that reduces type II skeletal muscle contraction by 90%. Skeletal muscle contraction may be measured by a muscle force test after nerve stimulation using surface electrodes (e.g., foot plantar flexion after peroneal nerve stimulation in the leg), isolated limb assay, heart rate monitor or an activity monitor or equivalents thereof prior to and following the administration of a skeletal muscle contraction inhibitor.

Tidal volume in lung in a subject may be measured prior to and following the administration of Compound 1 or a salt thereof. Administration may not inhibit tidal volume in a lung. In some cases, administration may not appreciably inhibit tidal volume in a lung. In certain embodiments with regard to tidal lung volume in a lung, the phrase not appreciably refers to the tidal volume in a lung reduced by less than 10%, less than 8%, less than 6%, less than 4%, less than 2%, less than 1%, less than 0.5% or less than 0.1% relative to the tidal volume in a lung prior to the administration of the inhibitor. Tidal volume in a lung in a subject may be measured using forced volume in one second test (FEV1) or forced vital capacity test (FVC) or equivalent tests thereof.

Smooth muscle contraction in a subject may be measured prior to and following the administration of a skeletal muscle contraction inhibitor. Inhibition of skeletal muscle contraction may not inhibit smooth muscle contraction. In some cases, the inhibition of skeletal muscle contraction may not appreciably inhibit smooth muscle contraction. As used herein with regard to smooth muscle contraction, the phrase not appreciably refers to the smooth muscle contraction reduced by less than 10%, less than 8%, less than 6%, less than 4%, less than 2%, less than 1%, less than 0.5% or even less than 0.1% relative to the smooth muscle contraction prior to the administration of the inhibitor. Smooth muscle contraction in a subject may be evaluated by measuring a subject's blood pressure.

Neuromuscular coupling in a subject may be measured prior to and following the administration of Compound 1 or a salt thereof. Inhibition of skeletal muscle contraction, with an inhibitor described herein, may not impair nerve conduction, neurotransmitter release or electrical depolarization of skeletal muscle in a subject. In some cases, the inhibition of skeletal muscle contraction may not appreciably impair neuromuscular coupling in a subject. As used herein with regard to neuromuscular coupling, the phrase not appreciably refers to a level of neuromuscular coupling in the subject reduced by less than 10%, less than 8%, less than 6%, less than 4%, less than 2%, less than 1%, less than 0.5% or less than 0.1% relative to the level of neuromuscular coupling in the subject prior to the administration of the inhibitor. Neuromuscular coupling in a subject may be evaluated by measuring nerve induced electrical depolarization of skeletal muscle by the recording of electrical activity produced by skeletal muscles after electrical or voluntary stimulation with electromyography (EMG) using surface or needle electrodes.

Pain may be associated with the neuromuscular condition disclosed herein. Therefore, disclosed here is a method of reducing a pain associated with a neuromuscular condition, comprising administering to a subject in need thereof a muscle fast myosin (Type II) inhibitor. In some embodiments, the pain is diffused. In some embodiments, the pain is in a spine of the subject. In some embodiments, the pain in in calves of the subject. In some embodiments, the pain is associated with Becker muscular dystrophy (BMD), Duchenne muscular dystrophy (DMD), Limb-girdle muscular dystrophies (LGMD), McArdle disease, and other neuromuscular conditions.

In some embodiments, a muscle fast myosin (Type II) inhibitor is administered to a subject exhibiting a dystrophin gene mutation selected from del 45-48, del 45-47, del 48, del x-51, del 45-55, and del 48-49.

In some embodiments, Compound 1 is administered to a subject in a sufficient quantity to maintain a plasma PK value of 500 ng/mL or higher, 1,000 ng/mL or higher, 1,200 ng/mL or higher, 1,400 ng/mL or higher, 1,800 ng/mL or higher, 2,000 ng/mL or higher, 2,200 ng/mL or higher, 2,300 ng/mL or higher, 2,400 ng/mL or higher, 2,500 ng/mL or higher, 2,800 ng/mL or higher, 3,000 ng/mL or higher, or 4,000 ng/mL or higher. In some embodiments, Compound 1 is administered to a subject in a sufficient quantity to maintain a plasma PK value of between about 500 ng/mL and 4,000 ng/mL, between about 500 ng/mL and 4,000 ng/mL, between about 1,000 ng/mL and 3,000 ng/mL, between about 1,300 ng/mL and 2,800 ng/mL, between about 1,400 ng/mL and 2,600 ng/mL, between about 2,000 ng/mL and 2,600 ng/mL, or between about 2,200 ng/mL and 2,400 ng/mL.

In some embodiments, Compound 1 is administered to a subject in a sufficient quantity to maintain a plasma concentration of about 10 ng/mL to about 200 ng/mL. In some embodiments, Compound 1 is administered to a subject in a sufficient quantity to maintain a plasma concentration of about 10 ng/mL to about 20 ng/mL, about 10 ng/mL to about 30 ng/mL, about 10 ng/mL to about 40 ng/mL, about 10 ng/mL to about 50 ng/mL, about 10 ng/mL to about 60 ng/mL, about 10 ng/mL to about 70 ng/mL, about 10 ng/mL to about 80 ng/mL, about 10 ng/mL to about 90 ng/mL, about 10 ng/mL to about 100 ng/mL, about 10 ng/mL to about 150 ng/mL, about 10 ng/mL to about 200 ng/mL, about 20 ng/mL to about 30 ng/mL, about 20 ng/mL to about 40 ng/mL, about 20 ng/mL to about 50 ng/mL, about 20 ng/mL to about 60 ng/mL, about 20 ng/mL to about 70 ng/mL, about 20 ng/mL to about 80 ng/mL, about 20 ng/mL to about 90 ng/mL, about 20 ng/mL to about 100 ng/mL, about 20 ng/mL to about 150 ng/mL, about 20 ng/mL to about 200 ng/mL, about 30 ng/mL to about 40 ng/mL, about 30 ng/mL to about 50 ng/mL, about 30 ng/mL to about 60 ng/mL, about 30 ng/mL to about 70 ng/mL, about 30 ng/mL to about 80 ng/mL, about 30 ng/mL to about 90 ng/mL, about 30 ng/mL to about 100 ng/mL, about 30 ng/mL to about 150 ng/mL, about 30 ng/mL to about 200 ng/mL, about 40 ng/mL to about 50 ng/mL, about 40 ng/mL to about 60 ng/mL, about 40 ng/mL to about 70 ng/mL, about 40 ng/mL to about 80 ng/mL, about 40 ng/mL to about 90 ng/mL, about 40 ng/mL to about 100 ng/mL, about 40 ng/mL to about 150 ng/mL, about 40 ng/mL to about 200 ng/mL, about 50 ng/mL to about 60 ng/mL, about 50 ng/mL to about 70 ng/mL, about 50 ng/mL to about 80 ng/mL, about 50 ng/mL to about 90 ng/mL, about 50 ng/mL to about 100 ng/mL, about 50 ng/mL to about 150 ng/mL, about 50 ng/mL to about 200 ng/mL, about 60 ng/mL to about 70 ng/mL, about 60 ng/mL to about 80 ng/mL, about 60 ng/mL to about 90 ng/mL, about 60 ng/mL to about 100 ng/mL, about 60 ng/mL to about 150 ng/mL, about 60 ng/mL to about 200 ng/mL, about 70 ng/mL to about 80 ng/mL, about 70 ng/mL to about 90 ng/mL, about 70 ng/mL to about 100 ng/mL, about 70 ng/mL to about 150 ng/mL, about 70 ng/mL to about 200 ng/mL, about 80 ng/mL to about 90 ng/mL, about 80 ng/mL to about 100 ng/mL, about 80 ng/mL to about 150 ng/mL, about 80 ng/mL to about 200 ng/mL, about 90 ng/mL to about 100 ng/mL, about 90 ng/mL to about 150 ng/mL, about 90 ng/mL to about 200 ng/mL, about 100 ng/mL to about 150 ng/mL, about 100 ng/mL to about 200 ng/mL, or about 150 ng/mL to about 200 ng/mL. In some embodiments, Compound 1 is administered to a subject in a sufficient quantity to maintain a plasma concentration of about 10 ng/mL, about 20 ng/mL, about 30 ng/mL, about 40 ng/mL, about 50 ng/mL, about 60 ng/mL, about 70 ng/mL, about 80 ng/mL, about 90 ng/mL, about 100 ng/mL, about 150 ng/mL, or about 200 ng/mL. In some embodiments, Compound 1 is administered to a subject in a sufficient quantity to maintain a plasma concentration of at least about 10 ng/mL, about 20 ng/mL, about 30 ng/mL, about 40 ng/mL, about 50 ng/mL, about 60 ng/mL, about 70 ng/mL, about 80 ng/mL, about 90 ng/mL, about 100 ng/mL, or about 150 ng/mL. In some embodiments, Compound 1 is administered to a subject in a sufficient quantity to maintain a plasma concentration of at most about 20 ng/mL, about 30 ng/mL, about 40 ng/mL, about 50 ng/mL, about 60 ng/mL, about 70 ng/mL, about 80 ng/mL, about 90 ng/mL, about 100 ng/mL, about 150 ng/mL, or about 200 ng/mL.

The muscle fast myosin (Type II) inhibitor may be administered for varying duration. In some embodiments, the muscle fast myosin (Type II) inhibitor is administered for about 1, 2, 3, 4, 5, 6, 7, 8, 9, 10, 11, or 12 months. In some embodiments, the muscle fast myosin (Type II) inhibitor is administered for more than 1 month, more than 2 months, more than 3 months, more than 4 months, more than 5 months, more than 6 months, more than 7 months, more than 8 months, more than 9 months, more than 10 months, more than 11 months, or more than 12 months.

In some embodiments, Compound 1 is administered. In some embodiments, Compound 1 is administered in a daily dose of about 1 mg to about 10 mg. In some embodiments, Compound 1 is administered in a daily dose of about 1 mg to about 2 mg, about 1 mg to about 2.5 mg, about 1 mg to about 3 mg, about 1 mg to about 4 mg, about 1 mg to about 5 mg, about 1 mg to about 6 mg, about 1 mg to about 7 mg, about 1 mg to about 7.5 mg, about 1 mg to about 8 mg, about 1 mg to about 9 mg, about 1 mg to about 10 mg, about 2 mg to about 2.5 mg, about 2 mg to about 3 mg, about 2 mg to about 4 mg, about 2 mg to about 5 mg, about 2 mg to about 6 mg, about 2 mg to about 7 mg, about 2 mg to about 7.5 mg, about 2 mg to about 8 mg, about 2 mg to about 9 mg, about 2 mg to about 10 mg, about 2.5 mg to about 3 mg, about 2.5 mg to about 4 mg, about 2.5 mg to about 5 mg, about 2.5 mg to about 6 mg, about 2.5 mg to about 7 mg, about 2.5 mg to about 7.5 mg, about 2.5 mg to about 8 mg, about 2.5 mg to about 9 mg, about 2.5 mg to about 10 mg, about 3 mg to about 4 mg, about 3 mg to about 5 mg, about 3 mg to about 6 mg, about 3 mg to about 7 mg, about 3 mg to about 7.5 mg, about 3 mg to about 8 mg, about 3 mg to about 9 mg, about 3 mg to about 10 mg, about 4 mg to about 5 mg, about 4 mg to about 6 mg, about 4 mg to about 7 mg, about 4 mg to about 7.5 mg, about 4 mg to about 8 mg, about 4 mg to about 9 mg, about 4 mg to about 10 mg, about 5 mg to about 6 mg, about 5 mg to about 7 mg, about 5 mg to about 7.5 mg, about 5 mg to about 8 mg, about 5 mg to about 9 mg, about 5 mg to about 10 mg, about 6 mg to about 7 mg, about 6 mg to about 7.5 mg, about 6 mg to about 8 mg, about 6 mg to about 9 mg, about 6 mg to about 10 mg, about 7 mg to about 7.5 mg, about 7 mg to about 8 mg, about 7 mg to about 9 mg, about 7 mg to about 10 mg, about 7.5 mg to about 8 mg, about 7.5 mg to about 9 mg, about 7.5 mg to about 10 mg, about 8 mg to about 9 mg, about 8 mg to about 10 mg, or about 9 mg to about 10 mg. In some embodiments, Compound 1 is administered in a daily dose of about 1 mg, about 2 mg, about 2.5 mg, about 3 mg, about 4 mg, about 5 mg, about 6 mg, about 7 mg, about 7.5 mg, about 8 mg, about 9 mg, or about 10 mg. In some embodiments, Compound 1 is administered in a daily dose of at least about 1 mg, about 2 mg, about 2.5 mg, about 3 mg, about 4 mg, about 5 mg, about 6 mg, about 7 mg, about 7.5 mg, about 8 mg, or about 9 mg. In some embodiments, Compound 1 is administered in a daily dose of at most about 2 mg, about 2.5 mg, about 3 mg, about 4 mg, about 5 mg, about 6 mg, about 7 mg, about 7.5 mg, about 8 mg, about 9 mg, or about 10 mg. In some embodiments, Compound 1 is administered in a daily dose of about 2.5 mg to about 20 mg. In some embodiments, Compound 1 is administered in a daily dose of about 2.5 mg to about 5 mg, about 2.5 mg to about 7.5 mg, about 2.5 mg to about 10 mg, about 2.5 mg to about 12.5 mg, about 2.5 mg to about 15 mg, about 2.5 mg to about 17.5 mg, about 2.5 mg to about 20 mg, about 5 mg to about 7.5 mg, about 5 mg to about 10 mg, about 5 mg to about 12.5 mg, about 5 mg to about 15 mg, about 5 mg to about 17.5 mg, about 5 mg to about 20 mg, about 7.5 mg to about 10 mg, about 7.5 mg to about 12.5 mg, about 7.5 mg to about 15 mg, about 7.5 mg to about 17.5 mg, about 7.5 mg to about 20 mg, about 10 mg to about 12.5 mg, about 10 mg to about 15 mg, about 10 mg to about 17.5 mg, about 10 mg to about 20 mg, about 12.5 mg to about 15 mg, about 12.5 mg to about 17.5 mg, about 12.5 mg to about 20 mg, about 15 mg to about 17.5 mg, about 15 mg to about 20 mg, or about 17.5 mg to about 20 mg. In some embodiments, Compound 1 is administered in a daily dose of about 2.5 mg, about 5 mg, about 7.5 mg, about 10 mg, about 12.5 mg, about 15 mg, about 17.5 mg, or about 20 mg. In some embodiments, Compound 1 is administered in a daily dose of at least about 2.5 mg, about 5 mg, about 7.5 mg, about 10 mg, about 12.5 mg, about 15 mg, or about 17.5 mg. In some embodiments, Compound 1 is administered in a daily dose of at most about 5 mg, about 7.5 mg, about 10 mg, about 12.5 mg, about 15 mg, about 17.5 mg, or about 20 mg.

In some embodiments, the muscle fast myosin (Type II) inhibitor is administered at a constant dosage throughout the whole treatment. In some embodiments, the muscle fast myosin (Type II) inhibitor is administered at a constant dosage throughout about 1, 2, 3, 4, 5, 6, 7, 8, 9, 10, 11, or 12 months. In some embodiments, the muscle fast myosin (Type II) inhibitor is administered at a constant dosage for more than 1 month, more than 2 months, more than 3 months, more than 4 months, more than 5 months, more than 6 months, more than 7 months, more than 8 months, more than 9 months, more than 10 months, more than 11 months, or more than 12 months. In some embodiments, the constant dosage is more than 1mg/day, more than 2.5mg/day, more than 5mg/day, more than 10mg/day, more than 15mg/day, more than 20mg/day, more than 25mg/day, more than 30mg/day, more than 35mg/day, or more than 40mg/day. In some embodiments, the constant dosage is less than 1mg/day, less than 2.5mg/day, less than 5mg/day, less than 10mg/day, less than 15mg/day, less than 20mg/day, less than 25mg/day, less than 30mg/day, less than 35mg/day, less than 40mg/day, less than 45mg/day, less than 50mg/day, less than 55mg/day, or less than 60mg/day. In some embodiments, the constant dosage is about 1mg/day, 2mg/day, 2.5mg/day, 3mg/day, 4mg/day, 5mg/day, 6mg/day, 7mg/day, 7.5mg/day, 8mg/day, 9mg/day, 10mg/day, 11mg/day, 12mg/day, 12.5mg/day, 13mg/day, 14mg/day, 15mg/day, 16mg/day, 17mg/day, 17,5mg/day, 18mg/day, 19mg/day,or 20mg/day. In some preferred embodiments, the constant dosage is about 10mg/day.

In some embodiments, the muscle fast myosin (Type II) inhibitor is administered at a varying dosage throughout the whole treatment. In some embodiments, the muscle fast myosin (Type II) inhibitor is administered at a varying dosage throughout about 1, 2, 3, 4, 5, 6, 7, 8, 9, 10, 11, or 12 months. In some embodiments, the muscle fast myosin (Type II) inhibitor is administered at a varying dosage for more than 1 month, more than 2 months, more than 3 months, more than 4 months, more than 5 months, more than 6 months, more than 7 months, more than 8 months, more than 9 months, more than 10 months, more than 11 months, or more than 12 months. In specific embodiments, the muscle fast myosin (Type II) inhibitor is administered at a deescalating dosage throughout the treatment. In specific embodiments, the muscle fast myosin (Type II) inhibitor is administered at an escalating dosage throughout the treatment. In some embodiments, the muscle fast myosin (Type II) inhibitor is administered at xmg/day, followed by (x+5)mg/day, further followed by (x+5 times 2)mg/day, further followed by (x+5 times 3)mg/day, further followed by (x+5 times 4)mg/day, lastly followed by (x+5 times y)mg/day where x can be a number between 1-100, 5-100, 5-50, 5-20, or 5-10, and y can be a number between 1-10. In some embodiments, the muscle fast myosin (Type II) inhibitor is administered at xmg/day, followed by (x+5)mg/day, and further followed by (x+10)mg/day, where x can be between 1-100, 5-100, 5-50, 5-20, or 5-10. In some embodiments, the muscle fast myosin (Type II) inhibitor is administered at 10mg/day, followed by 15mg/day, and further followed by 20mg/day.

In some embodiments where the muscle fast myosin (Type II) inhibitor is administered at a varying dosage throughout the treatment, the varying dosage lasts for about 1, 2, 3, 4, 5, 6, 7, 8, 9, 10, 11, or 12 months. In some specific embodiments, the muscle fast myosin (Type II) inhibitor is administered at 10mg/day for 1^{st} and 2^{nd} month, followed by 15mg/day from 3^{rd} month to 6^{th} month, and further followed by 20mg/day from 7^{th} month to 12^{th} month.

The muscle fast myosin (Type II) inhibitor may be administered at a varying dosage which is determined in real time based on one or more biomarkers and/or the plasma concentration of the muscle fast myosin (Type II) inhibitor in the subject. In some embodiments, the varying dosage is determined by whether it is sufficient to reduce one or more biomarkers (e.g., creatine kinase, lactate dehydrogenase, myoglobin, TNNI2). In some embodiments, the muscle fast myosin (Type II) inhibitor is administered at a varying dosage to maintain a plasma concentration of said muscle fast myosin (Type II) inhibitor in the subject in an optimal range. In some embodiments, the muscle fast myosin (Type II) inhibitor is administered at a varying dosage to maintain a plasma concentration of said muscle fast myosin (Type II) inhibitor in the subject to be between 5ng/ml to 200ng/ml, between 10ng/ml to 200ng/ml, between 10ng/ml to 150ng/ml, between 10ng/ml to 140ng/ml, between 10ng/ml to 130ng/ml, between 10ng/ml to 120ng/ml, between 10ng/ml to 110ng/ml, between 10ng/ml to 100ng/ml, between 10ng/ml to 80ng/ml, between 20ng/ml to 70ng/ml, between 40ng/ml to 60ng/ml, between 40ng/ml to 100ng/ml, between 20ng/ml to 200ng/ml, between 20ng/ml to 150ng/ml, between 20ng/ml to 140ng/ml, between 20ng/ml to 130ng/ml, between 20ng/ml to 120ng/ml, between 20ng/ml to 110ng/ml, between 20ng/ml to 100ng/ml, between 30ng/ml to 200ng/ml, between 30ng/ml to 150ng/ml, between 30ng/ml to 140ng/ml, between 30ng/ml to 130ng/ml, between 30ng/ml to 120ng/ml, between 30ng/ml to 110ng/ml, between 30ng/ml to 100ng/ml, between 40ng/ml to 200ng/ml, between 40ng/ml to 150ng/ml, between 40ng/ml to 140ng/ml, between 40ng/ml to 130ng/ml, between 40ng/ml to 120ng/ml, between 40ng/ml to 110ng/ml, or between 40ng/ml to 100ng/ml. In some embodiments, the muscle fast myosin (Type II) inhibitor is administered at a varying dosage to maintain a plasma concentration of said muscle fast myosin (Type II) inhibitor in the subject to be between 10ng/ml to 100ng/ml, between 10ng/ml to 100ng/ml, between 10ng/ml to 100ng/ml, 20ng/ml to 100ng/ml, or 30ng/ml to 100ng/ml. In some embodiments, the muscle fast myosin (Type II) inhibitor is administered at a varying dosage to maintain a plasma concentration of said muscle fast myosin (Type II) inhibitor in the subject to be more than 10ng/ml, more than 15ng/ml, more than 20ng/ml, more than 25ng/ml, more than 30ng/ml, more than 35ng/ml, or more than 40ng/ml. In some embodiments, the muscle fast myosin (Type II) inhibitor is administered at a varying dosage to maintain a plasma concentration of said muscle fast myosin (Type II) inhibitor in the subject to be less than 70ng/ml, less than 60ng/ml, less than 50ng/ml, less than 40ng/ml, or less than 30ng/ml. In some embodiments, the muscle fast myosin (Type II) inhibitor is administered at a varying dosage to maintain a plasma concentration of said muscle fast myosin (Type II) inhibitor in the subject to be between 15ng/ml to 70ng/ml, between 15ng/ml to 60ng/ml, between 15ng/ml to 50ng/ml, between 15ng/ml to 40ng/ml, between 15ng/ml to 30ng/ml, between 15ng/ml to 20ng/ml, between 20ng/ml to 70ng/ml, between 25ng/ml to 70ng/ml, between 30ng/ml to 70ng/ml, between 35ng/ml to 70ng/ml, between 40ng/ml to 70ng/ml, between 45ng/ml to 70ng/ml, between 50ng/ml to 70ng/ml, between 55ng/ml to 70ng/ml, between 60ng/ml to 70ng/ml, or between 65ng/ml to 70ng/ml.

In some embodiments, the muscle fast myosin (Type II) inhibitor is enriched in muscles. Accordingly, in some embodiments, the muscle fast myosin (Type II) inhibitor is administered at a varying dosage to maintain a muscle concentration of said muscle fast myosin (Type II) inhibitor in the subject to be more than 500 ng/g, more than 600 ng/g, more than 700 ng/g, more than 800 ng/g, more than 900 ng/g, more than 1000 ng/g, more than 1100 ng/g, more than 1200 ng/g, more than 1300 ng/g, more than 1400 ng/g, more than 1500 ng/g, more than 2000 ng/g, or more than 3000 ng/g. In some embodiments, the muscle fast myosin (Type II) inhibitor is administered at a varying dosage to maintain a muscle concentration of said muscle fast myosin (Type II) inhibitor in the subject to be less than 10000 ng/g, less than 9000 ng/g, less than 8000 ng/g, less than 7000 ng/g, less than 6000 ng/g, less than 5000 ng/g, less than 4000 ng/g, or less than 3000 ng/g. In some embodiments, the muscle fast myosin (Type II) inhibitor is administered at a varying dosage to maintain a muscle concentration of said muscle fast myosin (Type II) inhibitor in the subject to be between 1000 ng/g to 4000 ng/g, between 1000 ng/g to 3000 ng/g, between 1000 ng/g to 2000 ng/g, between 2000 ng/g to 4000 ng/g, between 2000 ng/g to 4000 ng/g, or between 3000 ng/g to 4000 ng/g.

The compositions and methods described herein may be considered useful as pharmaceutical compositions for administration to a subject in need thereof. Pharmaceutical compositions may comprise a muscle fast myosin (Type II) inhibitor and one or more pharmaceutically acceptable carriers, diluents, excipients, stabilizers, dispersing agents, suspending agents, and/or thickening agents. In some embodiments, the muscle fast myosin (Type II) inhibitor is a compound or salt thereof described in WO2020097265, WO2020097258, WO2020097266, WO2021231572, WO2021231572, WO2021231546, WO2021231630, and WO2021231615. In some embodiments, the muscle fast myosin (Type II) inhibitor is described in WO2020097266. In some embodiments, the muscle myosin inhibitor is the compound of Formula (I): 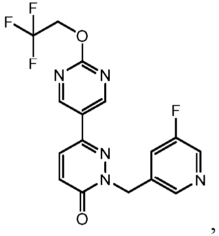 also referred to as Compound 1.

Pharmaceutical compositions comprising a muscle fast myosin (Type II) inhibitor may be formulated using one or more physiologically-acceptable carriers comprising excipients and auxiliaries. Formulation may be modified depending upon the route of administration chosen. Pharmaceutical compositions comprising a compound, salt or conjugate may be manufactured, for example, by lyophilizing the compound, salt or conjugate, mixing, dissolving, emulsifying, encapsulating, or entrapping the conjugate. The pharmaceutical compositions may also include the compounds, salts or conjugates in a free-base form or pharmaceutically-acceptable salt form.

Pharmaceutical compositions comprising a muscle fast myosin (Type II) inhibitor may include formulating any of the compounds, salts, or conjugates with one or more inert, pharmaceutically-acceptable excipients or carriers to form a solid, semi-solid, or liquid composition. Solid compositions may include, for example, powders, tablets, dispersible granules, and capsules, and in some aspects, the solid compositions further contain nontoxic, auxiliary substances, for example wetting or emulsifying agents, pH buffering agents, and other pharmaceutically-acceptable additives. Alternatively, the compounds, salts or conjugates may be lyophilized or in powder form for re-constitution with a suitable vehicle, e.g., sterile pyrogen-free water, before use.

Pharmaceutical compositions comprising a muscle fast myosin (Type II) inhibitor may comprise at least one active ingredient (e.g., a compound, salt or conjugate and other agents). The active ingredients may be entrapped in microcapsules prepared, for example, by coacervation techniques or by interfacial polymerization (e.g., hydroxymethylcellulose or gelatin microcapsules and poly-(methylmethacylate) microcapsules, respectively), in colloidal drug-delivery systems (e.g., liposomes, albumin microspheres, microemulsions, nano-particles and nanocapsules) or in macroemulsions.

The compositions and formulations may be sterilized. Sterilization may be accomplished by filtration through sterile filtration.

Pharmaceutical compositions comprising a muscle fast myosin (Type II) inhibitor may be formulated for administration as an injection. Non-limiting examples of formulations for injection may include a sterile suspension, solution, or emulsion in oily or aqueous vehicles. Suitable oily vehicles may include, but are not limited to, lipophilic solvents or vehicles such as fatty oils or synthetic fatty acid esters, or liposomes. Aqueous injection suspensions may contain substances which increase the viscosity of the suspension. The suspension may also contain suitable stabilizers. Injections may be formulated for bolus injection or continuous infusion. Alternatively, the compositions may be lyophilized or in powder form for reconstitution with a suitable vehicle, e.g., sterile pyrogen-free water, before use.

Pharmaceutical compositions comprising a muscle fast myosin (Type II) inhibitor may be formulated in a unit dosage injectable form (e.g., solution, suspension, emulsion) in association with a pharmaceutically acceptable parenteral vehicle. Such vehicles may be inherently nontoxic, and non-therapeutic. Vehicles may be water, saline, Ringer's solution, dextrose solution, and 5% human serum albumin. Non-aqueous vehicles such as fixed oils and ethyl oleate may also be used. Liposomes may be used as carriers. The vehicle may contain minor amounts of additives such as substances that enhance isotonicity and chemical stability (e.g., buffers and preservatives).

Pharmaceutical compositions comprising a muscle fast myosin (Type II) inhibitor may be formulated for oral delivery to a subject in need. In one embodiment a composition is formulated so as to deliver one or more pharmaceutically active agents to a subject through a mucosa layer in the mouth or esophagus. In another embodiment the composition is formulated to deliver one or more pharmaceutically active agents to a subject through a mucosa layer in the stomach and/or intestines.

In one embodiment compositions comprising a muscle fast myosin (Type II) inhibitor may include modified release dosage forms. Suitable modified release dosage vehicles include, but are not limited to, hydrophilic or hydrophobic matrix devices, water-soluble separating layer coatings, enteric coatings, osmotic devices, multi-particulate devices, and combinations thereof. The compositions may also comprise non-release controlling excipients.

In another embodiment compositions comprising a muscle fast myosin (Type II) inhibitor may be provided in enteric coated dosage forms. These enteric coated dosage forms can also comprise non-release controlling excipients. In one embodiment the compositions are in the form of enteric-coated granules, as controlled-release capsules for oral administration. The compositions can further comprise cellulose, disodium hydrogen phosphate, hydroxypropyl cellulose, pyridazine, lactose, mannitol, or sodium lauryl sulfate. In another embodiment the compositions are in the form of enteric-coated pellets, as controlled-release capsules for oral administration. The compositions can further comprise glycerol monostearate 40-50, hydroxypropyl cellulose, pyridazine, magnesium stearate, methacrylic acid copolymer type C, polysorbate 80, sugar spheres, talc, or triethyl citrate.

In another embodiment the compositions comprising a muscle fast myosin (Type II) inhibitor are enteric-coated controlled-release tablets for oral administration. The compositions can further comprise carnauba wax, crospovidone, diacetylated monoglycerides, ethylcellulose, hydroxypropyl cellulose, pyridazine phthalate, magnesium stearate, mannitol, sodium hydroxide, sodium stearyl fumarate, talc, titanium dioxide, or yellow ferric oxide.

Sustained release compositions comprising a muscle fast myosin (Type II) inhibitor may also be prepared. Examples of sustained-release preparations may include semipermeable matrices of solid hydrophobic polymers that may contain the compound, salt or conjugate, and these matrices may be in the form of shaped articles (e.g., films or microcapsules). Examples of sustained-release matrices may include polyesters, hydrogels (e.g., poly(2-hydroxyethylmethacrylate), or poly(vinyl alcohol)), polylactides, copolymers of L-glutamic acid and γ ethyl-L-glutamate, non-degradable ethylene-vinyl acetate, degradable lactic acid-glycolic acid copolymers such as the LUPRON DEPO^{™} (i.e., injectable microspheres composed of lactic acid-glycolic acid copolymer and leuprolide acetate), and poly-D-(-)-3-hydroxybutyric acid.

Pharmaceutical compositions comprising a muscle fast myosin (Type II) inhibitor may be prepared for storage by mixing a compound, salt or conjugate with a pharmaceutically acceptable carrier, excipient, and/or a stabilizer. This formulation may be a lyophilized formulation or an aqueous solution. Acceptable carriers, excipients, and/or stabilizers may be nontoxic to recipients at the dosages and concentrations used. Acceptable carriers, excipients, and/or stabilizers may include buffers such as phosphate, citrate, and other organic acids; antioxidants including ascorbic acid and methionine; preservatives, polypeptides; proteins, such as serum albumin or gelatin; hydrophilic polymers; amino acids; monosaccharides, disaccharides, and other carbohydrates including glucose, mannose, or dextrin; chelating agents such as EDTA; sugars such as sucrose, mannitol, trehalose or sorbitol; salt-forming counter-ions such as sodium; metal complexes; and/or non-ionic surfactants or polyethylene glycol.

Pharmaceutical compositions comprising a muscle fast myosin (Type II) inhibitor can further comprise calcium stearate, crospovidone, hydroxypropyl methylcellulose, iron oxide, mannitol, methacrylic acid copolymer, polysorbate 80, povidone, propylene glycol, sodium carbonate, sodium lauryl sulfate, titanium dioxide, and triethyl citrate.

Pharmaceutical compositions comprising a muscle fast myosin (Type II) inhibitor can be provided in a dosage form that has at least one component that can facilitate the immediate release of an active agent, and at least one component that can facilitate the controlled release of an active agent. In a further embodiment the dosage form can be capable of giving a discontinuous release of the compound in the form of at least two consecutive pulses separated in time from 0.1 up to 24 hours. The compositions can comprise one or more release controlling and non-release controlling excipients, such as those excipients suitable for a disruptable semipermeable membrane and as swellable substances.

Pharmaceutical compositions comprising a muscle fast myosin (Type II) inhibitor are provided in a dosage form for oral administration to a subject, which comprise one or more pharmaceutically acceptable excipients or carriers, enclosed in an intermediate reactive layer comprising a gastric juice-resistant polymeric layered material partially neutralized with alkali and having cation exchange capacity and a gastric juice-resistant outer layer.

Pharmaceutical compositions comprising a muscle fast myosin (Type II) inhibitor provided herein can be in unit-dosage forms or multiple-dosage forms. Unit-dosage forms, as used herein, refer to physically discrete units suitable for administration to human or non-human animal subjects and packaged individually. Each unit-dose can contain a predetermined quantity of an active ingredient(s) sufficient to produce the desired therapeutic effect, in association with the required pharmaceutical carriers or excipients. Examples of unit-dosage forms include, but are not limited to, ampoules, syringes, and individually packaged tablets and capsules. In some embodiments, unit-dosage forms may be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment the multiple dosage forms comprise different pharmaceutically active agents.

Pharmaceutical compositions comprising a muscle fast myosin (Type II) inhibitor may also be formulated as a modified release dosage form, including immediate-, delayed-, extended-, prolonged-, sustained-, pulsatile-, controlled-, extended, accelerated- and fast-, targeted-, programmed-release, and gastric retention dosage forms. These dosage forms can be prepared according to known methods and techniques (see, Remington: The Science and Practice of Pharmacy, supra; Modified-Release Drug Delivery Technology, Rathbone et al., Eds., Drugs and the Pharmaceutical Science, Marcel Dekker, Inc.: New York, N.Y., 2002; Vol. 126, which are herein incorporated by reference in their entirety).

### EMBODIMENTS

Embodiment 1. A method of treating a neuromuscular condition, comprising administering to a subject a muscle fast myosin (Type II) inhibitor in an amount sufficient to maintain a North Star Ambulatory Assessment (NSAA) score or increase the NSAA score of the subject relative to a pre-treatment NSAA score.

Embodiment 2. The method of Embodiment 1, comprising administering the muscle fast myosin (Type II) inhibitor in an amount sufficient to maintain the NSAA score of the subject relative to the pre-treatment NSAA score.

Embodiment 3. The method of Embodiment 2, comprising administering the muscle fast myosin (Type II) inhibitor in an amount sufficient to maintain the NSAA score of the subject within 20% of the pre-treatment NSAA score for a period of 2 months or more, 4 months or more, or 6 months or more.

Embodiment 4. The method of Embodiment 1, comprising administering the muscle fast myosin (Type II) inhibitor in an amount sufficient to increase the NSAA score of the subject by 0.3 points or more relative to the pre-treatment NSAA score.

Embodiment 5. The method of Embodiment 4, comprising administering the muscle fast myosin (Type II) inhibitor in an amount sufficient to increase the NSAA score of the subject by 1 point or more relative to the pre-treatment NSAA score.

Embodiment 6. The method of any one of Embodiments 1 to 5, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 7. The method of Embodiment 6, wherein the NSAA score of the subject is evaluated 2 months or more, 4 months or more, or 6 months or more following the first dose of said muscle fast myosin (Type II) inhibitor to the subject.

Embodiment 8. The method of Embodiment 6 or 7, wherein the pre-treatment NSAA score is evaluated within 1 month prior to the first dose of said muscle fast myosin (Type II) inhibitor to the subject.

Embodiment 9. A method of treating a neuromuscular condition, comprising administering to a subject a muscle fast myosin (Type II) inhibitor in an amount sufficient to maintain or improve one or more of the following functional measures of the subject: max elbow flexion strength relative to a pre-treatment max elbow flexion strength; max knee extension strength relative to a pre-treatment max knee extension strength; 10 meter walk/run velocity relative to a pre-treatment 10 meter walk/run velocity; 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value.

Embodiment 10100 meter timed test velocity 100 meter timed test velocity4-stair climb time velocity4-stair climb time velocity. The method of Embodiment 9, comprising administering the muscle fast myosin (Type II) inhibitor in an amount sufficient to maintain the one or more functional measures of the subject relative to the pre-treatment value.

Embodiment 11. The method of Embodiment 10, comprising administering the muscle fast myosin (Type II) inhibitor in an amount sufficient to maintain the one or more functional measures of the subject within 20% of the pre-treatment value for a period of 2 months or more, 4 months or more, or 6 months or more.

Embodiment 12. The method of Embodiment 9, comprising administering the muscle fast myosin (Type II) inhibitor in an amount sufficient to increase the one or more functional measures of the subject by 20% or more relative to the pre-treatment value.

Embodiment 13. The method of Embodiment 12, comprising administering the muscle fast myosin (Type II) inhibitor in an amount sufficient to increase the one or more functional measures of the subject by 30% or more relative to the pre-treatment value.

Embodiment 14. The method of any one of Embodiments 9 to 13, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 15. The method of Embodiment 14, wherein one or more functional measures of the subject is evaluated 2 months or more, 4 months or more, or 6 months or more following the first dose of said muscle fast myosin (Type II) inhibitor to the subject.

Embodiment 16. The method of Embodiment 14 or 15, wherein the pre-treatment value is evaluated within 1 month prior to the first dose of said muscle fast myosin (Type II) inhibitor to the subject.

Embodiment 17. A method of treating a neuromuscular condition, comprising administering to a subject a muscle fast myosin (Type II) inhibitor in an amount sufficient to maintain a creatine kinase activity level or reduce the creatine kinase activity level of the subject relative to a pre-treatment creatine kinase activity level by 10% or more.

Embodiment 18. The method of Embodiment 17, comprising administering the muscle fast myosin (Type II) inhibitor in an amount sufficient to maintain the creatine kinase activity of the subject relative to the pre-treatment creatine kinase activity level.

Embodiment 19. The method of Embodiment 18, comprising administering the muscle fast myosin (Type II) inhibitor in an amount sufficient to maintain the creatine kinase activity level of the subject within 10% of the pre-treatment creatine kinase activity level.

Embodiment 20. The method of Embodiment 17, comprising administering the muscle fast myosin (Type II) inhibitor in an amount sufficient to reduce the creatine kinase activity level of the subject relative to a pre-treatment creatine kinase activity level by 15% or more, 20 % or more, 25% or more, 30% or more, or 35% or more.

Embodiment 21. The method of any one of Embodiments 17 to 20, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 22. The method of Embodiment 21, wherein the creatine kinase activity level of the subject is evaluated 2 months or more, 4 months or more, or 6 months or more following the first dose of said muscle fast myosin (Type II) inhibitor to the subject.

Embodiment 23. The method of Embodiment 21 or 22, wherein the pre-treatment creatine kinase activity level is evaluated within 1 month prior to the first dose of said muscle fast myosin (Type II) inhibitor to the subject.

Embodiment 24. A method of treating a neuromuscular condition, comprising administering to the subject a muscle fast myosin (Type II) inhibitor in an amount sufficient to maintain the serum creatinine level or increase the serum creatinine level of the subject relative to a pre-treatment serum creatinine level by 10% or more.

Embodiment 25. The method of Embodiment 24, comprising administering the muscle fast myosin (Type II) inhibitor in an amount sufficient to maintain the serum creatinine level of the subject relative to the pre-treatment serum creatinine level.

Embodiment 26. The method of Embodiment 25, comprising administering the muscle fast myosin (Type II) inhibitor in an amount sufficient to maintain the serum creatinine level of the subject within 10% of the pre-treatment serum creatinine level for a period of 2 months or more, 4 months or more, or 6 months or more.

Embodiment 27. The method of Embodiment 24, comprising administering the muscle fast myosin (Type II) inhibitor in an amount sufficient to increase the serum creatinine level of the subject by 10% or more or 20% or more relative to the pre-treatment serum creatinine level.

Embodiment 28. The method of any one of Embodiments 24 to 27, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 29. The method of Embodiment 28, wherein the serum creatinine level of the subject is evaluated 2 months or more, 4 months or more, or 6 months or more following the first dose of said muscle fast myosin (Type II) inhibitor to the subject.

Embodiment 30. The method of Embodiment 28 or 29, wherein the pre-treatment serum creatinine level is evaluated within 1 month prior to the first dose of said muscle fast myosin (Type II) inhibitor to the subject.

Embodiment 31. A method of treating a neuromuscular condition, comprising administering to the subject a muscle fast myosin (Type II) inhibitor in an amount sufficient to maintain the Dual Energy x-Ray absorptiometry (DXA) % lean mass of the subject or increase the DXA % lean mass of the subject relative to a pre-treatment DXA % lean mass by 5% or more.

Embodiment 32. The method of Embodiment 31, comprising administering the muscle fast myosin (Type II) inhibitor in an amount sufficient to maintain the DXA % lean mass of the subject relative to the pre-treatment DXA % lean mass.

Embodiment 33. The method of Embodiment 32, comprising administering the muscle fast myosin (Type II) inhibitor in an amount sufficient to maintain the DXA % lean mass of the subject within 5% of the pre-treatment DXA % lean mass for a period of 2 months or more, 4 months or more, or 6 months or more.

Embodiment 34. The method of Embodiment 31, comprising administering the muscle fast myosin (Type II) inhibitor in an amount sufficient to increase the DXA % lean mass of the subject by 5% or more, 10% or more or 15% or more relative to a pre-treatment DXA % lean mass.

Embodiment 35. The method of any one of Embodiments 31 to 34, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 36. The method of Embodiment 35, wherein the DXA % lean mass of the subject is evaluated 2 months or more, 4 months or more, or 6 months or more following the first dose of said muscle fast myosin (Type II) inhibitor to the subject.

Embodiment 37. The method of Embodiment 35 or 36, wherein the pre-treatment DXA % lean mass is evaluated within 1 month prior to the first dose of said muscle fast myosin (Type II) inhibitor to the subject.

Embodiment 38. A method of treating a neuromuscular condition, comprising administering to the subject a muscle fast myosin (Type II) inhibitor in an amount sufficient to maintain a Fast Skeletal Muscle Troponin I (TNNI2) concentration of the subject or reduce the TNNI2 concentration of the subject relative to a pre-treatment TNNI2 concentration by 10% or more, and optionally wherein the TNNI2 concentration is inferred from a SOMAscan level.

Embodiment 39. The method of Embodiment 38, comprising administering the muscle fast myosin (Type II) inhibitor in an amount sufficient to maintain the TNNI2 concentration of the subject relative to the pre-treatment TNNI2 concentration.

Embodiment 40. The method of Embodiment 39, comprising administering the muscle fast myosin (Type II) inhibitor in an amount sufficient to maintain the TNNI2 concentration of the subject within 10% of the pre-treatment creatine kinase activity level.

Embodiment 41. The method of Embodiment 38, comprising administering the muscle fast myosin (Type II) inhibitor in an amount sufficient to reduce the TNNI2 concentration of the subject relative to a pre-treatment TNNI2 concentration by 15% or more, 20 % or more, 25% or more, 30% or more, 40% or more, 50% or more, 60% or more, or 70% or more.

Embodiment 42. The method of any one of Embodiments 38 to 41, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 43. The method of Embodiment 42, wherein the TNNI2 concentration of the subject is evaluated 2 months or more, 4 months or more, or 6 months or more following the first dose of said muscle fast myosin (Type II) inhibitor to the subject.

Embodiment 44. The method of Embodiment 42 or 43, wherein the TNNI2 concentration is evaluated within 1 month prior to the first dose of said muscle fast myosin (Type II) inhibitor to the subject.

Embodiment 45. The method of any one of the preceding Embodiments, wherein the muscle fast myosin (Type II) inhibitor is a compound of Formula (I):

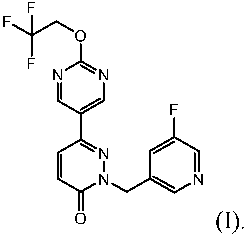

Embodiment 46. The method of any one of the preceding Embodiments, wherein administering comprises administering the muscle fast myosin (Type II) inhibitor once a day, twice a day, three times a day, once every two days, once every three days, once every four days, once every five days, once every six days or once a week.

Embodiment 47. The method of Embodiment 46, wherein administering comprises administering the muscle fast myosin (Type II) inhibitor once a day.

Embodiment 48. The method of any one of Embodiments 1 to 47, wherein the administering comprises administering the muscle fast myosin (Type II) inhibitor to the subject for 2 months or more, 3 months or more, 4 months or more, 5 months or more, 6 months or more, or 7 months or more.

Embodiment 49. The method of Embodiment 48, wherein the administering comprises administering the muscle fast myosin (Type II) inhibitor to the subject for 4 months or more, 5 months or more, 6 months or more, or 7 months or more.

Embodiment 50. The method of any of the preceding Embodiments, wherein the neuromuscular condition is selected from Becker Muscular Dystrophy, Duchenne Muscular Dystrophy, Limb-Girdle Muscular Dystrophy and McArdle Disease.

Embodiment 51. The method of Embodiment 50, wherein the neuromuscular condition is Becker Muscular Dystrophy.

Embodiment 52. The method of Embodiment 50, wherein the neuromuscular condition is Duchenne Muscular Dystrophy.

Embodiment 53. The method of any one of the preceding Embodiments, wherein the subject is 18 years or younger.

Embodiment 54. The method of any one of Embodiments 1 to 52, wherein the subject is over 18 years old.

Embodiment 55. The method of any of the preceding Embodiments, wherein prior to said administering the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

Embodiment 56. A method of inhibiting muscle fast myosin (Type II) in a subject in need thereof, comprising selecting a subject with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, -0.5 to -3, or - 1 to -4; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater; and administering a muscle fast myosin (Type II) inhibitor to the subject.
muscle fast myosin (Type II)Embodiment 57. The method of Embodiment 56, wherein the dystrophin gene mutation selected from del 45-48, del 45-47, del 48, del x-51, del 45-55, and del 48-49.

Embodiment 58. The method of Embodiment 56 or 57, wherein the muscle fast myosin (Type II) inhibitor is a compound of Formula (I):

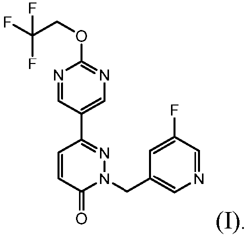

Embodiment 59. The method of any one of the preceding Embodiments, wherein administering comprises administering the muscle fast myosin (Type II) inhibitor once a day, twice a day, three times a day, once every two days, once every three days, once every four days, once every five days, once every six days or once a week.

Embodiment 60. The method of Embodiment 59, wherein administering comprises administering the muscle fast myosin (Type II) inhibitor once a day.

Embodiment 61. The method of any one of Embodiments 56 to 60, wherein the administering comprises administering the muscle fast myosin (Type II) inhibitor for 2 months or more, 3 months or more, 4 months or more, 5 months or more, 6 months or more, or 7 months or more.

Embodiment 62. The method of Embodiment 61, wherein the administering comprises administering the muscle fast myosin (Type II) inhibitor for 4 months or more, 5 months or more, 6 months or more, or 7 months or more.

Embodiment 63. The method of any one of Embodiments 56 to 62, wherein the neuromuscular condition is selected from Becker Muscular Dystrophy, Duchenne Muscular Dystrophy, Limb-Girdle Muscular Dystrophy and McArdle Disease.

Embodiment 64. The method of Embodiment 63, wherein the neuromuscular condition is selected Becker Muscular Dystrophy.

Embodiment 65. The method of Embodiment 63, wherein the neuromuscular condition is Duchenne Muscular Dystrophy.

Embodiment 66. The method of any one of Embodiments 56 to 65, wherein the subject is 18 years or younger.

Embodiment 67. The method of any one of Embodiments 56 to 66, wherein the administering does not negatively impact the cardiac function of the subject.

Embodiment 68. The method of any one of Embodiments 61 to 67, wherein one or more of characteristics (a)-(g) have improved following said administering.

Embodiment 69. A unit dose for oral administration, comprising a compound of Formula (I) 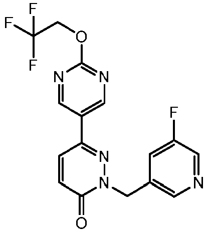 in an amount from about 1 mg to about 20 mg, and a pharmaceutically acceptable excipient.

Embodiment 70. The unit dose of Embodiment 69, comprising from about 1 mg to about 5 mg of the compound of Formula (I), and a pharmaceutically acceptable excipient.

Embodiment 71. The unit dose of Embodiment 70, comprising from about 1 mg to about 3 mg of the compound of Formula (I), and a pharmaceutically acceptable excipient.

Embodiment 72. The unit dose of Embodiment 70, comprising about 2.5 mg of the compound of Formula (I), and a pharmaceutically acceptable excipient.

Embodiment 73. The unit dose of any one of Embodiments 69 to 72, wherein the unit dose is formulated as a pill or a tablet.

Embodiment 74. A kit, comprising an oral pharmaceutical composition and instructions for administration to a subject in need thereof, wherein the oral pharmaceutical composition comprises a compound of Formula (I): 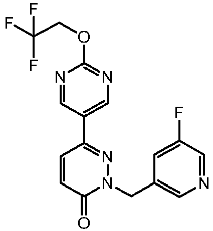 and a pharmaceutically acceptable excipient.

Embodiment 75. A method of treating a neuromuscular condition, comprising administering to a subject in need thereof a dose of 3 mg to 40 mg per day of a compound of Formula (I):

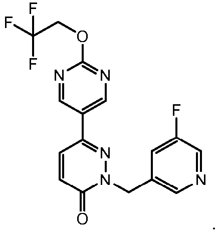

Embodiment 76. The method of Embodiment 75, comprising administering to the subject a dose of 15 mg to 40 mg per day of the compound of Formula (I).

Embodiment 77. The method of Embodiment 76, comprising administering to the subject a dose of 15 mg, 20 mg, 25 mg, or 30 mg per day of the compound of Formula (I).

Embodiment 78. The method of Embodiment 77, comprising administering to the subject a dose of 20 mg per day of the compound of Formula (I).

Embodiment 79. The method of Embodiment 75, comprising administering to the subject a dose of 2 mg to 15 mg per day of the compound of Formula (I).

Embodiment 80. The method of Embodiment 79, comprising administering to the subject a dose of 5 mg to 12.5 mg per day of the compound of Formula (I).

Embodiment 81. The method of Embodiment 80, comprising administering to the subject a dose of 2 mg, 2.5 mg, 5 mg, 7.5 mg, 10mg, or 12.5 mg per day of the compound of Formula (I).

Embodiment 82. The method of any one of Embodiments 75 to 81, wherein the subject is over 18 years old.

Embodiment 83. The method of any one of Embodiments 75 to 81, wherein the subject is 18 year or younger.

Embodiment 84. The method of any of Embodiments 75 to 83, wherein the neuromuscular condition is selected from Becker Muscular Dystrophy, Duchenne Muscular Dystrophy, Limb-Girdle Muscular Dystrophy and McArdle Disease.

Embodiment 85. The method of Embodiment 84, wherein the neuromuscular condition is Becker Muscular Dystrophy.

Embodiment 86. The method of Embodiment 84, wherein the neuromuscular condition is Duchenne Muscular Dystrophy.

Embodiment 87. The method of any one of Embodiments 75 to 86, wherein the administering comprises administering the compound of Formula (I) for 2 months or more, 3 months or more, 4 months or more, 5 months or more, 6 months or more, or 7 months or more.

Embodiment 88. The method of Embodiment 87, wherein the administering comprises administering the compound of Formula (I) for 4 months or more, 5 months or more, 6 months or more, or 7 months or more.

Embodiment 89. The method of Embodiment 88, wherein the administering comprises administering the compound of Formula (I) for 6 months or more.

Embodiment 90. A method of treating a neuromuscular condition, comprising administering to the subject a compound of Formula (I): 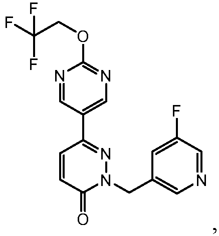 in an amount sufficient to maintain an AUC₂₄ of the subject of 1000 ng/mL or greater.

Embodiment 91. The method of Embodiment 90, comprising administering comprises administering the compound of Formula (I) for 2 months or more, 3 months or more, 4 months or more, 5 months or more, 6 months or more, or 7 months or more.

Embodiment 92. The method of Embodiment 91, wherein the administering comprises administering the muscle fast myosin (Type II) inhibitor for 4 months or more, 5 months or more, 6 months or more, or 7 months or more.

Embodiment 93. The method of any one of Embodiments 90 to 92, wherein the compound of Formula (I) is administered in an amount sufficient to maintain an AUC₂₄ of the subject of 1200 ng/mL or greater.

Embodiment 94. The method of Embodiment 93, wherein the compound of Formula (I) is administered in an amount sufficient to maintain an AUC₂₄ of the subject of 1400 ng/mL or greater.

Embodiment 95. The method of any one of Embodiments 90 to 94, wherein the neuromuscular condition is selected from Becker Muscular Dystrophy, Duchenne Muscular Dystrophy, Limb-Girdle Muscular Dystrophy and McArdle Disease.

Embodiment 96. The method of Embodiment 95, wherein the neuromuscular condition is Becker Muscular Dystrophy.

Embodiment 97. The method of Embodiment 95, wherein the neuromuscular condition is Duchenne Muscular Dystrophy.

Embodiment 98. The method of any one of Embodiments 90 to 97, wherein the subject is older than 18.

Embodiment 99. The method of any one of Embodiments 90 to 97, wherein the subject is 18 years or younger.

Embodiment 100. The method of any one of Embodiments 90 to 99, wherein the administering does not negatively impact the cardiac function of the subject.

Embodiment 101. The method of any one of Embodiments 90 to 100, wherein administering comprises administering the compound of Formula (I) once a day, twice a day, three times a day, once every two days, once every three days, once every four days, once every five days, once every six days or once a week.

Embodiment 102. The method of Embodiment 101, wherein administering comprises administering the compound of Formula I once a day.

Embodiment 103. The method of any one of Embodiments 90 to 102, wherein prior to said administering the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

Embodiment 104. A method of treating a neuromuscular condition, comprising administering to a subject a muscle fast myosin (Type II) inhibitor in an amount sufficient to maintain a myoglobin level or reduce a myoglobin level of the subject relative to a pre-treatment myoglobin level by 10% or more.

Embodiment 105. The method of Embodiment 104, comprising administering the muscle fast myosin (Type II) inhibitor in an amount sufficient to maintain the myoglobin level of the subject relative to the pre-treatment myoglobin level.

Embodiment 106. The method of Embodiment 104, comprising administering the muscle fast myosin (Type II) inhibitor in an amount sufficient to maintain the myoglobin level of the subject within 10% of the pre-treatment myoglobin level.

Embodiment 107. The method of Embodiment 104, comprising administering the muscle fast myosin (Type II) inhibitor in an amount sufficient to reduce the myoglobin level of the subject relative to a pre-treatment myoglobin by 5% or more, 10% or more, 15% or more, 20 % or more, or 25% or more.

Embodiment 108. The method of any one of Embodiments 104 to 107, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 109. The method of any one of Embodiments 104 to 108, wherein the myoglobin level of the subject is evaluated 2 months or more, 4 months or more, or 6 months or more following the first dose of said muscle fast myosin (Type II) inhibitor to the subject.

Embodiment 110. The method of any one of Embodiments 104 to 109, wherein the pre-treatment myoglobin level is evaluated within 1 month prior to the first dose of said muscle fast myosin (Type II) inhibitor to the subject.

Embodiment 111. A method of reducing a pain associated with a neuromuscular condition, comprising administering to a subject in need thereof a muscle fast myosin (Type II) inhibitor.

Embodiment 112. The method of Embodiment 111, wherein the pain is diffused.

Embodiment 113. The method of Embodiment 112, wherein the pain is on a spine and calves of the subject.

Embodiment 114. The method of any of Embodiments 111 to 113, wherein the neuromuscular condition is selected from Becker Muscular Dystrophy, Duchenne Muscular Dystrophy, Limb-Girdle Muscular Dystrophy and McArdle Disease.

Embodiment 115. The method of Embodiment 114, wherein the neuromuscular condition is Becker Muscular Dystrophy.

Embodiment 116. The method of Embodiment 114, wherein the neuromuscular condition is Duchenne Muscular Dystrophy.

Embodiment 117. The method of any of the preceding Embodiments, wherein said muscle fast myosin (Type II) inhibitor is administered for 1, 2, 3, 4, 5, 6, 7, 8, 9, 10, 11, or 12 months.

Embodiment 118. The method of Embodiment 117, wherein said muscle fast myosin (Type II) inhibitor is administered at a constant dosage throughout the 1, 2, 3, 4, 5, 6, 7, 8, 9, 10, 11, or 12 months.

Embodiment 119. The method of Embodiment 118, wherein said constant dosage is 10mg/day, 15mg/day, or 20mg/day.

Embodiment 120. The method of Embodiment 119, wherein said constant dosage is 10mg/day.

Embodiment 121. The method of Embodiment 117, wherein said muscle fast myosin (Type II) inhibitor is administered at a varying dosage throughout the 1, 2, 3, 4, 5, 6, 7, 8, 9, 10, 11, or 12 months

Embodiment 122. The method of Embodiment 121, wherein said muscle fast myosin (Type II) inhibitor is administered at a deescalating dosage throughout the 1, 2, 3, 4, 5, 6, 7, 8, 9, 10, 11, or 12 months.

Embodiment 123. The method of Embodiment 121, wherein said muscle fast myosin (Type II) inhibitor is administered at an escalating dosage throughout the 1, 2, 3, 4, 5, 6, 7, 8, 9, 10, 11, or 12 months.

Embodiment 124. The method of Embodiment 123, wherein said muscle fast myosin (Type II) inhibitor is administered at 10mg/day, followed by 15mg/day, and further followed by 20mg/day.

Embodiment 125. The method of Embodiment 124, wherein said muscle fast myosin (Type II) inhibitor is administered at 10mg/day for 1^{st} and 2^{nd} month, followed by 15mg/day from 3^{rd} month to 6^{th} month, and further followed by 20mg/day from 7^{th} month to 12^{th} month.

Embodiment 126. The method of Embodiment 121, wherein said muscle fast myosin (Type II) inhibitor is administered at a varying dosage to maintain a plasma concentration of said muscle fast myosin (Type II) inhibitor in the subject to be between 10ng/ml to 100ng/ml, 15-70 ng/ml, 20ng/ml to 70ng/ml, or 30ng/ml to 70ng/ml.

Embodiment 127. The method of Embodiment 121, wherein said muscle fast myosin (Type II) inhibitor is administered at a varying dosage to maintain a muscle concentration of said muscle fast myosin (Type II) inhibitor in the subject to be 1,000-4,000 ng/g.

Embodiment 128. The method of one of the preceding Embodiments, wherein said muscle fast myosin (Type II) inhibitor is enriched in muscles.

Embodiment 129. The method of one of the preceding Embodiments, wherein said muscle fast myosin (Type II) inhibitor comprises a compound of Formula (I):

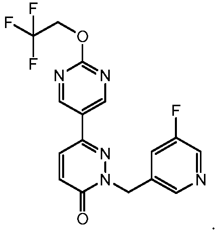

Embodiment 130. The unit dose of Embodiment 69, comprising from about 1 mg to about 15 mg of the compound of Formula (I), and a pharmaceutically acceptable excipient.

Embodiment 131. The unit dose of Embodiment 69, comprising from about 5 mg to about 15 mg of the compound of Formula (I), and a pharmaceutically acceptable excipient.

Embodiment 132. The unit dose of Embodiment 69, comprising from about 2.5 mg, about 5 mg, about 7.5 mg, about 10 mg, about 12.5 mg, about 15 mg, about 17.5 mg, or about 20 mg of the compound of Formula (I), and a pharmaceutically acceptable excipient.

Embodiment 133. The unit dose of Embodiment 69, comprising from about 2.5 mg of the compound of Formula (I), and a pharmaceutically acceptable excipient.

Embodiment 134. The unit dose of Embodiment 69, comprising from about 5 mg of the compound of Formula (I), and a pharmaceutically acceptable excipient.

Embodiment 135. The unit dose of Embodiment 69, comprising from about 10 mg of the compound of Formula (I), and a pharmaceutically acceptable excipient.

Embodiment 136. The method of Embodiment 75, comprising administering to the subject a dose of about 1 mg to about 15 mg per day of the compound of Formula (I).

Embodiment 137. The method of Embodiment 75, comprising administering to the subject a dose of about 5 mg to about 15 mg per day of the compound of Formula (I).

Embodiment 138. The method of Embodiment 75, comprising administering to the subject a dose of about 2.5 mg per day, about 5 mg per day, about 7.5 mg per day, about 10 mg per day, about 12.5 mg per day, about 15 mg per day, about 17.5 mg per day, or about 20 mg per day of the compound of Formula (I).

Embodiment 139. The method of Embodiment 75, comprising administering to the subject a dose of about 2.5 mg per day of the compound of Formula (I).

Embodiment 140. The method of Embodiment 75, comprising administering to the subject a dose of about 5 mg per day of the compound of Formula (I).

Embodiment 141. The method of Embodiment 75, comprising administering to the subject a dose of about 10 mg per day of the compound of Formula (I).

Embodiment 142. A method of treating a neuromuscular condition, comprising administering to a subject a muscle fast myosin (Type II) inhibitor in an amount sufficient to decrease protein concentration of one or more muscle injury biomarkers in the subject relative to a pre-treatment protein concentration of the one or more muscle injury biomarkers by 10% or more.

Embodiment 143. The method of Embodiment 142, comprising administering the muscle fast myosin (Type II) inhibitor in the amount sufficient to decrease the protein concentration of the one or more muscle injury biomarkers in the subject within 10% of the pre-treatment protein concentration of the one or more muscle injury biomarkers.

Embodiment 144. The method of Embodiment 142, comprising administering the muscle fast myosin (Type II) inhibitor in the amount sufficient to decrease the protein concentration of the one or more muscle injury biomarkers in the subject relative to the pre-treatment protein concentration of the one or more muscle injury biomarkers by 15% or more, 20 % or more, 25% or more, 30% or more, or 35% or more.

Embodiment 145. The method of any one of Embodiments 142 to 144, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 146. The method of Embodiment 145, wherein the protein concentration of the one or more muscle injury biomarkers of the subject is evaluated 2 months or more, 4 months or more, 6 months or more, 8 months or more, 10 months or more, or 12 months or more following the first dose of said muscle fast myosin (Type II) inhibitor to the subject.

Embodiment 147. The method of Embodiment 145 or 146, wherein the protein concentration of the one or more muscle injury biomarkers level is evaluated within 1 month prior to the first dose of said muscle fast myosin (Type II) inhibitor to the subject.

Embodiment 148. The method of any one of Embodiments 142 to 147, wherein the one or more muscle injury biomarkers comprise ACTN2, MYOM2, MYBPC1, PDLIM3, MYL3, TNNI2, MYL11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof.

Embodiment 149. A method of treating a neuromuscular condition, comprising administering to a subject a muscle fast myosin (Type II) inhibitor in an amount sufficient to decrease protein concentration of one or more pro-inflammatory proteins in the subject relative to a pre-treatment protein concentration of the one or more pro-inflammatory proteins by 10% or more.

Embodiment 150. The method of Embodiment 149, comprising administering the muscle fast myosin (Type II) inhibitor in the amount sufficient to decrease the protein concentration of the one or more pro-inflammatory proteins in the subject within 10% of the pre-treatment protein concentration of the one or more pro-inflammatory proteins.

Embodiment 151. The method of Embodiment 149, comprising administering the muscle fast myosin (Type II) inhibitor in the amount sufficient to decrease the protein concentration of the one or more pro-inflammatory proteins in the subject relative to the pre-treatment protein concentration of the one or more pro-inflammatory proteins by 15% or more, 20 % or more, 25% or more, 30% or more, or 35% or more.

Embodiment 152. The method of any one of Embodiments 149 to 151, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 153. The method of Embodiment 152, wherein the protein concentration of the one or more pro-inflammatory proteins of the subject is evaluated 2 months or more, 4 months or more, 6 months or more, 8 months or more, 10 months or more, or 12 months or more following the first dose of said muscle fast myosin (Type II) inhibitor to the subject.

Embodiment 154. The method of Embodiment 152 or 153, wherein the protein concentration of the one or more pro-inflammatory proteins is evaluated within 1 month prior to the first dose of said muscle fast myosin (Type II) inhibitor to the subject.

Embodiment 155. The method of any one of Embodiments 149 to 154, wherein the one or more pro-inflammatory proteins comprise CCL22, CCL5, CXCL10, FGG, IFN-γ, IL3, PPBP, PF4, or a combination thereof.

Embodiment 156. A method of treating a neuromuscular condition, comprising administering to a subject a muscle fast myosin (Type II) inhibitor in an amount sufficient to increase protein concentration of one or more anti-inflammatory proteins in the subject relative to a pre-treatment protein concentration of the one or more anti-inflammatory proteins by 10% or more.

Embodiment 157. The method of Embodiment 156, comprising administering the muscle fast myosin (Type II) inhibitor in the amount sufficient to increase the protein concentration of the one or more anti-inflammatory proteins in the subject within 10% of the pre-treatment protein concentration of the one or more anti-inflammatory proteins.

Embodiment 158. The method of Embodiment 156, comprising administering the muscle fast myosin (Type II) inhibitor in the amount sufficient to increase the protein concentration of the one or more anti-inflammatory proteins in the subject relative to the pre-treatment protein concentration of the one or more anti-inflammatory proteins by 15% or more, 20 % or more, 25% or more, 30% or more, or 35% or more.

Embodiment 159. The method of any one of Embodiments 156 to 158, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.

Embodiment 160. The method of Embodiment 159, wherein the protein concentration of the one or more anti-inflammatory proteins of the subject is evaluated 2 months or more, 4 months or more, 6 months or more, 8 months or more, 10 months or more, or 12 months or more following the first dose of said muscle fast myosin (Type II) inhibitor to the subject.

Embodiment 161. The method of Embodiment 159 or 160, wherein the protein concentration of the one or more anti-inflammatory proteins is evaluated within 1 month prior to the first dose of said muscle fast myosin (Type II) inhibitor to the subject.

Embodiment 162. The method of any one of Embodiments 156 to 161, wherein the one or more anti-inflammatory proteins comprise IL10, IL13, IL22, IL37, IL4, or a combination thereof.

Embodiment 163. The method of any one of Embodiments 142 to 162, wherein the muscle fast myosin (Type II) inhibitor is a compound of Formula (I):

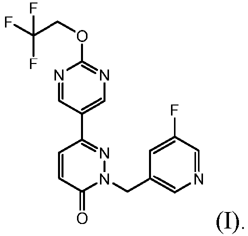

Embodiment 164. The method of any one of Embodiments 142 to 163, wherein administering comprises administering the muscle fast myosin (Type II) inhibitor once a day, twice a day, three times a day, once every two days, once every three days, once every four days, once every five days, once every six days or once a week.

Embodiment 165. The method of Embodiment 164, wherein administering comprises administering the muscle fast myosin (Type II) inhibitor once a day.

Embodiment 166. The method of any one of Embodiments 142 to 165, wherein the administering comprises administering the muscle fast myosin (Type II) inhibitor to the subject for 2 months or more, 3 months or more, 4 months or more, 5 months or more, 6 months or more, or 7 months or more.

Embodiment 167. The method of Embodiment 166, wherein the administering comprises administering the muscle fast myosin (Type II) inhibitor to the subject for 4 months or more, 5 months or more, 6 months or more, or 7 months or more.

Embodiment 168. The method of any one of Embodiments 142 to 167, wherein the neuromuscular condition is selected from Becker Muscular Dystrophy, Duchenne Muscular Dystrophy, Limb-Girdle Muscular Dystrophy and McArdle Disease.

Embodiment 169. The method of Embodiment 168, wherein the neuromuscular condition is Becker Muscular Dystrophy.

Embodiment 170. The method of Embodiment 168, wherein the neuromuscular condition is Duchenne Muscular Dystrophy.

Embodiment 171. The method of any one of Embodiments 142 to 170, wherein the subject is 18 years or younger.

Embodiment 172. The method of any one of Embodiments 142 to 170, wherein the subject is over 18 years old.

Embodiment 173. The method of any one of Embodiments 142 to 172, wherein prior to said administering the method further comprises selecting a subject for treatment with one or more of characteristics (a)-(g): (a) a creatine kinase activity level is 1000 U/L or greater, 1200 U/L or greater, 1300 U/L or greater; (b) a TNNI2 concentration of 20 ng/mL or greater, 30 ng/mL or great or 40 ng/mL or greater; (c) a dystrophin gene mutation; (d) a pre-treatment NSAA yearly change of -0.1 to -10, or -0.5 to -3; (e) a pre-treatment serum creatinine of 1 mg/dL or less, 0.8 mg/dL or less, 0.6 mg/dL or less; (f) a DXA % lean mass of <75%, less than 70%, less than 65%, less than 60%; and (g) a self-reported pain score associated with muscular dystrophy of 1.5 or greater, or 2 or greater.

### EXAMPLES

The invention now being generally described, it will be more readily understood by reference to the following examples which are included merely for purposes of illustration of certain aspects and embodiments of the present invention, and are not intended to limit the invention in any way.

### Example 1 -Human Clinical Trials

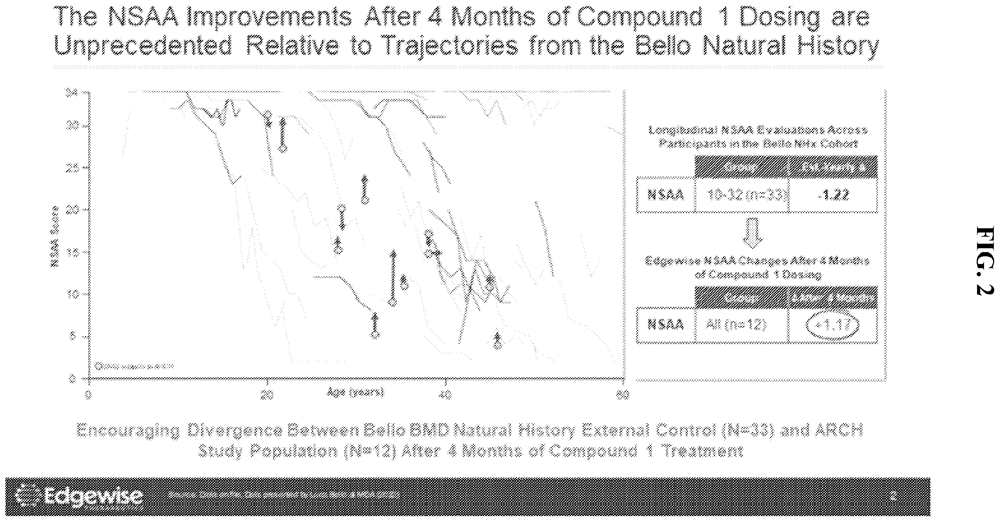

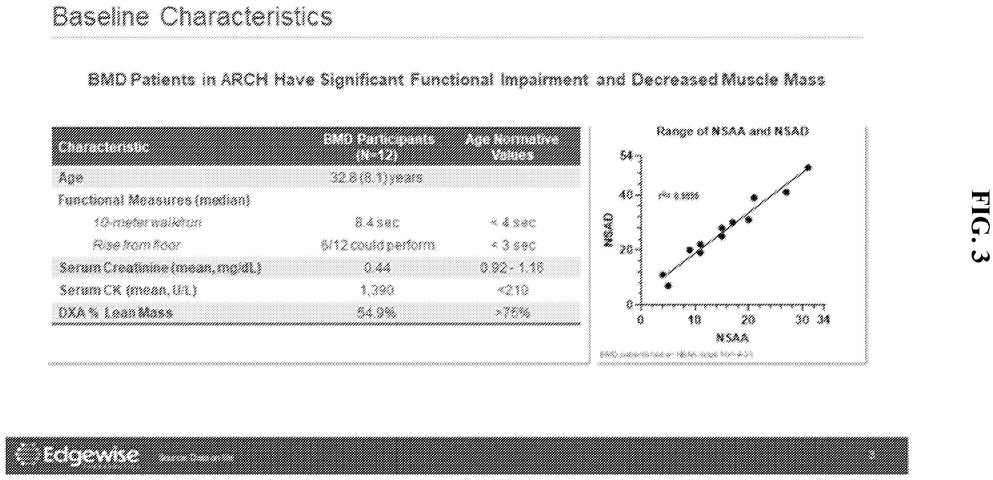

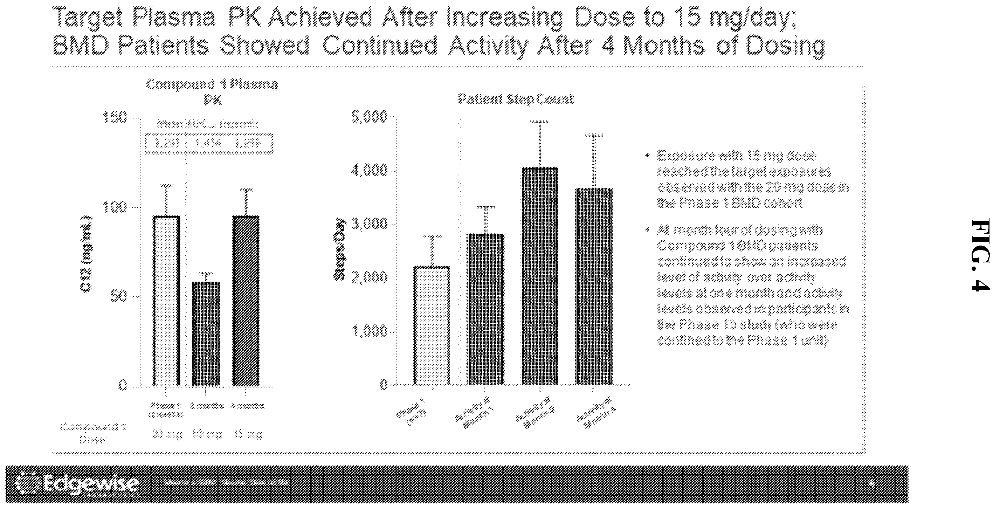

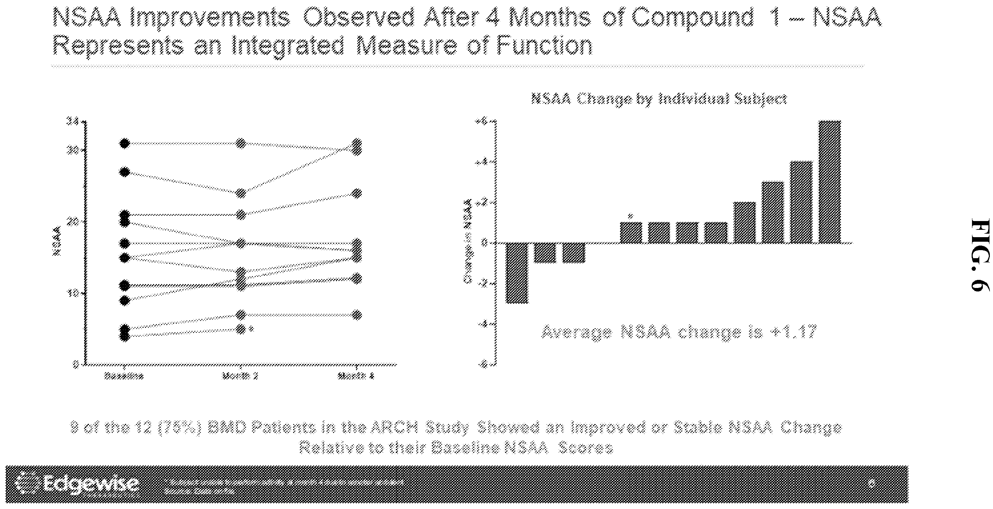

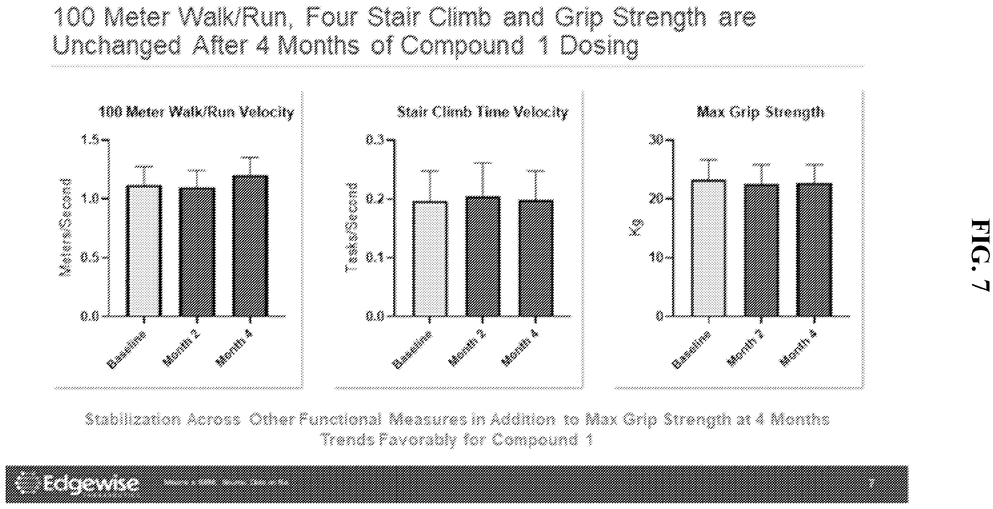

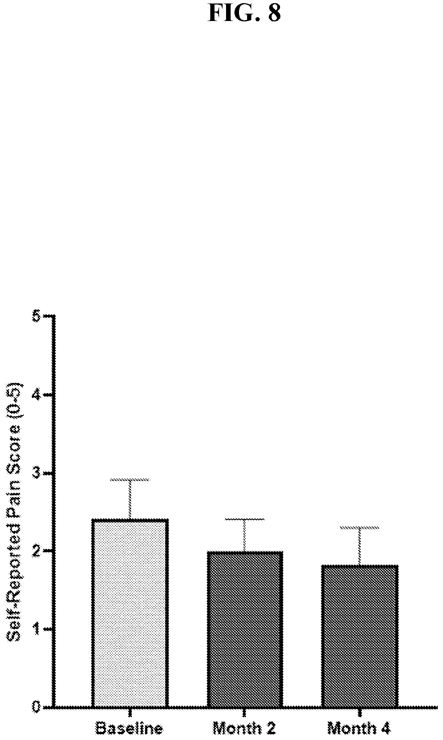

FIGs. 2 to 8 depict positive 4-month interim results from the ongoing open label, single-center study assessing the safety, tolerability, and impact on muscle damage biomarkers, and pharmacokinetics (PK) of Compound 1 in adults with BMD. Twelve adults with BMD enrolled in the ARCH study, were dose escalated to daily 15 mg oral doses of Compound 1 at night after having initially received a 10 mg dose during the first 2 months of the study. Compound 1 was well-tolerated in all participants with no discontinuations or dose reductions. The most common adverse events observed to date were dizziness, drowsiness, and headache. All eligible patients have subsequently been dose escalated to 20 mg daily as per protocol.

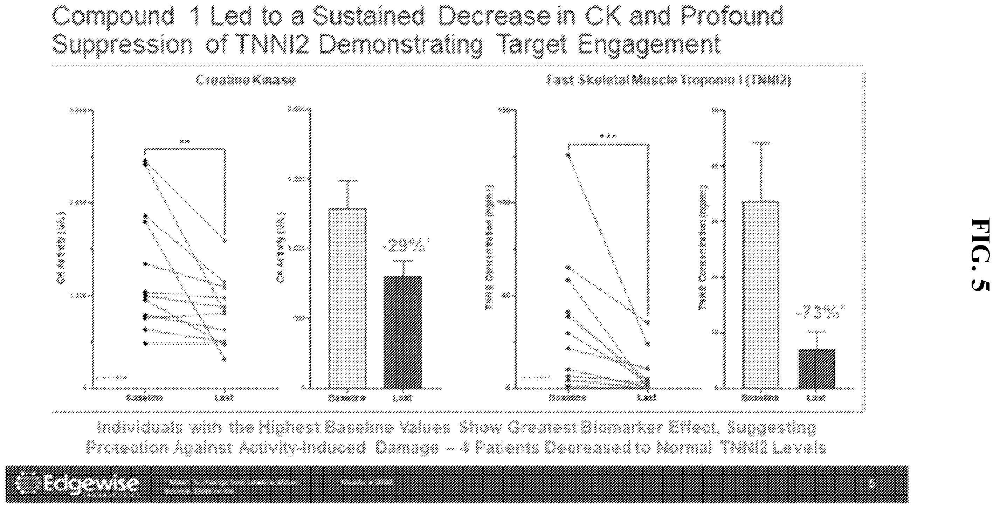

Treatment with Compound 1 led to a significant decrease in key biomarkers of muscle damage when assessed by laboratory assays. Importantly, CK and fast skeletal muscle troponin I were reduced by an average of 29% and 73%, respectively, after 4 months (FIG. 5). While CK reductions were sustained and in line with observations made at 2 months, fast skeletal muscle troponin I levels continued to drop with continued exposure to Compound 1 with four patients achieving levels observed in unaffected adults. Similar to observations made after 2 months, both CK and fast skeletal muscle troponin I were significantly decreased in the context of typical everyday activity levels as measured with a pedometer.

After 4 months of Compound 1 dosing, NSAA increased by an average of 1.17 points compared to pre-therapy baseline. Remarkably, nine of the twelve participants showed either a functional improvement or exhibited no decline on NSAA relative to their baselines (FIG. 2). The NSAA improvements observed after only 4 months of Compound 1 dosing differ from trajectories observed in the natural history study reported by Bello et al. (2016), one of the most comprehensively characterized BMD cohorts, in which the yearly decline was 1.22 NSAA points. These observations were further corroborated by an independent study from van de Velde et al. (2021), which showed a decline of 2.5 NSAA points over 2 years.

### Example 2 - Continued Human Clinical Trials

FIGs. 10 to 13 depict promising 12-month results from the ongoing open label, single-center study assessing the safety, tolerability, and impact on muscle damage biomarkers, and pharmacokinetics (PK) of Compound 1 in adults with BMD. The different dosing regimens depicted in FIGs. 10A-10C, FIG. 11B, and FIGs. 12A-12F were carried out in three cohorts. One cohort has been dosed with 20 mg of Compound 1 for 12 months. A second cohort has been dosed with 15 mg of Compound 1 for 6 months. A third cohort has been dosed with 10 mg of Compound 1 for 2 months. Compound 1 was well tolerated at all doses without any severe adverse events (AEs). Therefore, no treatment plans were discontinued due to AEs.

Several biomarkers of muscle damage were tested to evaluate the efficacy of Compound 1 with different dosing regimens. FIGs. 10A-10C illustrate Compound 1 resulted in sustained decrease of creatine kinase (CK), fast skeletal muscle troponin I (TNNI2), and myoglobin. Particularly, the 10mg dose showed near maximal decreases. When analyzing the changes of biomarkers in individual participants from the 12-month group, individuals with the highest baseline values of CK or TNNI2 showed the greatest reduction of biomarker by Compound 1, suggesting Compound 1 had a protection against activity-induced damages. *See* FIGs. 10D-10G.

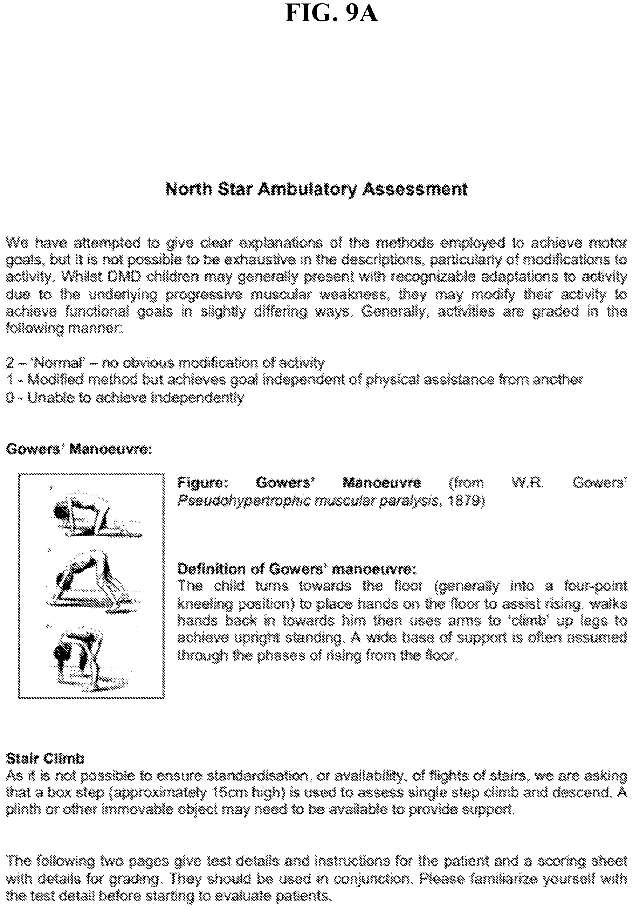

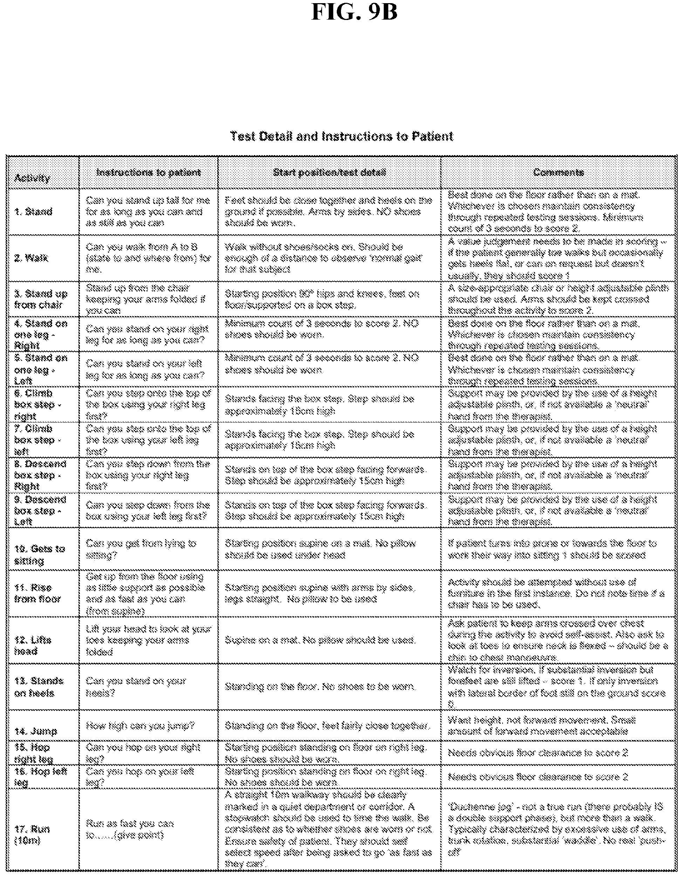

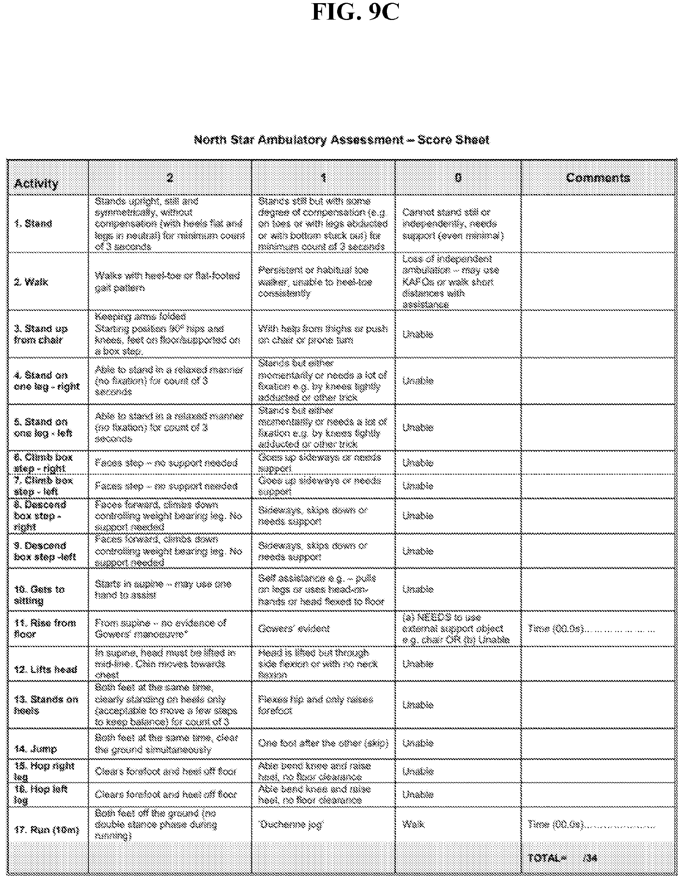

NSAA scores were also quantified based on FIGs. 9A-9C. Compound 1 stabilized NSAA scores by rescuing the natural history trend by about 1.6 points, and led to a trend towards an improvement in the scores. *See* FIG. 11A. Individual NSAA responses from the 12-month group were reflected in FIG. 11C, which showed that about 75% of the participants from the 12-month group exhibited the same or better NSAA scores. In comparison, the expected natural history decline in untreated patients is around -1.2 to -1.3 points.

In parallel, the plasma concentrations of Compound 1 were also monitored across different dosing regimens. *See* FIG. 11B. For group with higher dosage (e.g., the 20mg group), there were patients with high exposures or low exposures. It was unexpected that the patients with high exposures reached to a range of plasma concentration after dosing with 20mg/day, which was more than 4-fold higher than the target exposures predicted from preclinical models. It is also worth noting that Compound 1 was enriched in muscle. The target muscle concentration of Compound 1 from patients with lower plasma concentration were 1,000-4000 ng/g from preclinical experiments. Using muscle biopsy data from a Phase 1 multiple ascending dose (MAD) study, this equates to approximately 15-70 ng/ml in BMD. In other words, Compound 1 was enriched by about 60 fold in muscle compared to plasma.

Furthermore, the participants from the 20mg group with the lower exposures (highlighted in a bottom circle in FIG. 11D) had stable or the best improving NSAA scores *(see* bars with darker shades in FIG. 11C). In contrast, the participants with less optimal outcomes (e.g., negative changes in NSAA scores in FIG. 11C) were under high exposures of Compound 1 (highlighted in a top circle in FIG. 11D). Therefore, a lower exposure of Compound 1 (e.g., 10mg) may have a more desired functional benefit.

Furthermore, functional tests were carried out to evaluate the functional improvements of muscle in the participants resulted from Compound 1. For example, no statistically significant deterioration was observed in 10m velocity (*see* FIG. 12A), 100 meter velocity (*see* FIG. 12B), or 4-4-stair climb time velocity (*see* FIG. 12C). Different parameters of muscle strength were also quantified and evaluated in FIGs. 12D-12F. Furthermore, it was also observed that some participants from the 20mg group showed muscle strength weakness (*see e.g.,* FIGs. 12C-12F).

In combination with the data shown in FIGs. 10A-10C and 11B-11D, the reduction in related biomarkers indicating an improvement of activity-induced muscle damage and superior NSAA scores could be achieved at 10 mg/day. The dose of 20mg/day resulted in efficacy, however some weakening of the muscles also occurred. Therefore, Compound 1 with an optimal dosage control (e.g., 10mg/day) could result in a well-balanced outcome of muscle preservation and avoiding some muscle weakness.

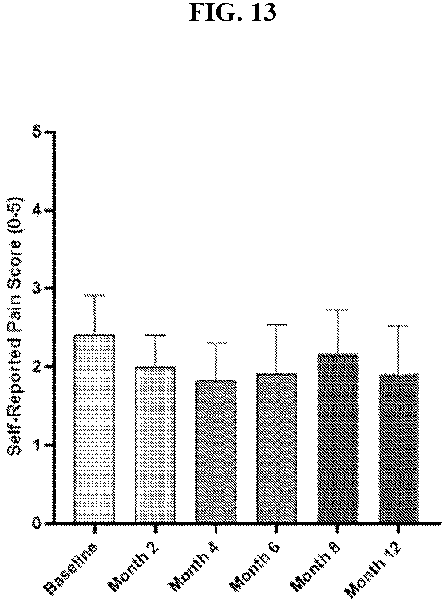

BMD individuals usually report more diffuse pain on spine and calves, *See* Jacques MF et al., Plos ONE (2019). Self-reported pain evaluation was collected and quantified in FIG. 13. A positive trend in the self-reported pain scores was observed after 12 months of Compound 1 treatment. Therefore, Compound 1 has shown to reduce pain associated with BMD.

### Example 3 - Characterization of Short- and Long-Term proteomic response to the Fast Skeletal Myosin Inhibitor in Becker Muscular Dystrophy (BMD)

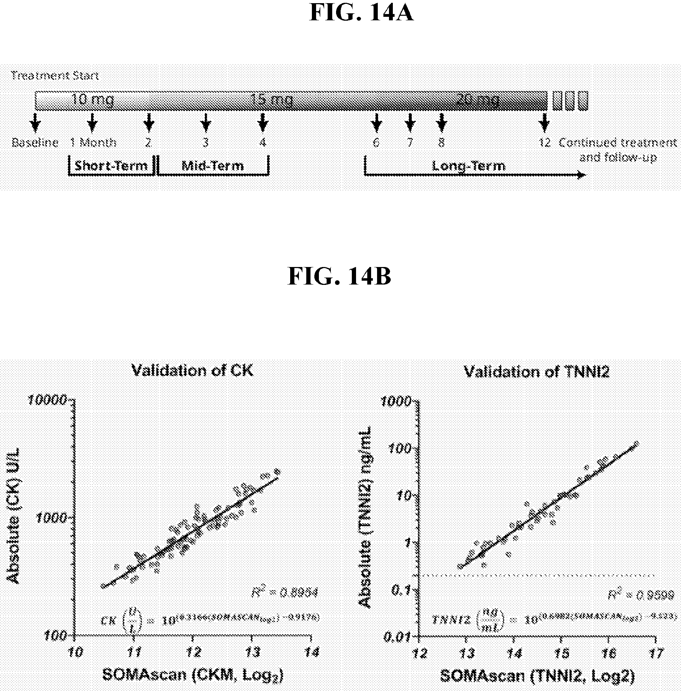

Changes in muscle injury biomarkers such as creatine kinase (CK) or fast skeletal muscle troponin I (TNNI2) were assessed in clinical trial participants. The abundance of the biomarkers were measured by plasma proteomics (SOMAscan) in short- (1-2 months), mid- (3-4 months), or long-term (6-12 months) treatment. FIG. 14A illustrates the design of this study. 12 BMD participants, 7 of whom participated in the Phase I MAD study (Example 2), were enrolled and initially treated with 10 mg of Compound 1 daily, with dose-escalation from 10 mg to 20 mg daily as shown. Blood draws were taken at pre-dose baseline, then at regular intervals thereafter for analysis by SOMAscan, as well as quantification of CK activity or TNNI2 concentration by an activity assay or ELISA. SOMAscan is a multistep modified aptamer-based assay for high0throughpu, sensitive, and unbiased biomarker measurement. Proteins in the samples were selectively bound with fluorescent aptamers, which were then measured on a chip array to yield values proportion to absolute concentrations.

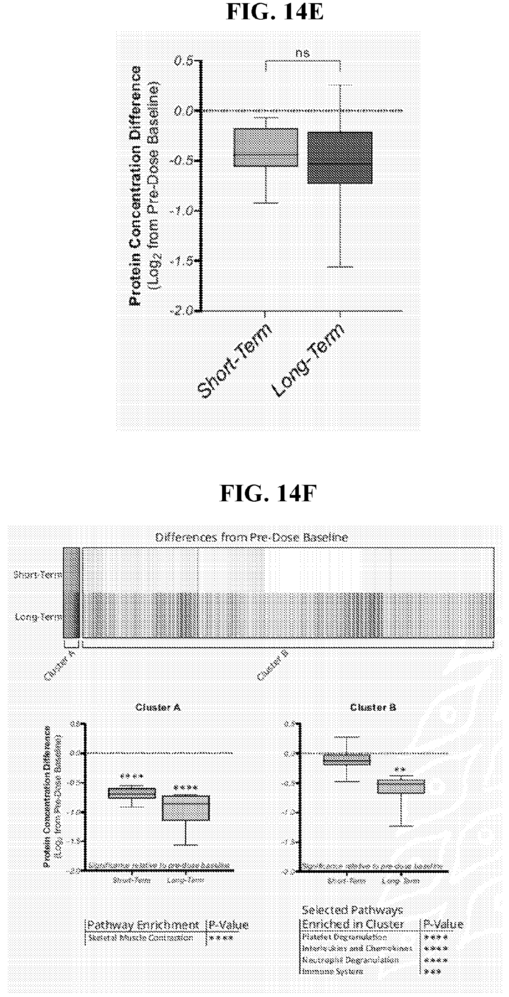

In this study, SOMAscan was correlated to CK and TNNI2 to allow for direct conversion to absolute concentrations as well as used to identify large-scale proteomic differences between short- and long-term treatment with Compound 1. FIG. 14B illustrates validation of CK and TNNI2, where a strong correlation between SOMAscan and absolute measurements of CK (left) and TNNI2 (right) was observed. FIG. 14C illustrates SOMAscan-quantified CK and TNNI2 in blood. Circulating CK and TNNI2 were significantly increased over values seen in healthy individuals at baseline and were reduced by short-term treatment. TNNI2 was reduced to nearly healthy levels by longer-term treatment timepoints. Indicated significance was for all values at that treatment point relative to pre-dose baseline. FIG. 14D and FIG. 14E illustrate the effect of Compound 1 on muscle injury proteins. SOMAscan was used to analyze the effects of treatment on a set of proteins identified to correlate with bona-fide contraction-induced skeletal muscle injury (FIG. 14D). These proteins, like CK and TNNI2, were decreased from pre-dose baseline by short-term treatment and exhibited stable levels or further decreases over long-term treatments. As a population, there was no difference in the response of muscle injury proteins with short-term and long-term treatment (FIG. 14E), indicating that muscle effects of treatment are maximized by 10 mg Compound 1 treatment for 1-2 months.

FIG. 14F illustrates proteomics of long-term Compound 1 treatment. Proteins that were: decreased by long-term treatment; or differentiated based on long-term treatment compared to short-term treatment exhibited two distinct clusters of changes. Cluster A was significantly decreased from pre-treatment baseline after both short- and long-term treatment and was characterized by several skeletal muscle proteins. Conversely, proteins in Cluster B were only responsive after longer period of Compound 1 treatment. These proteins primarily represented immune and inflammatory pathways.

FIG. 14G and FIG. 14H illustrate favorable shift in inflammatory biomarkers with long-term treatment. A favorable shift was observed in the inflammatory profile of study participants after long-term Compound 1 treatment. Several proinflammatory cytokines and chemokines were very significantly reduced after 6-12 months as indicated by decrease in gray scale intensity (top panel of FIG. 14G), while several anti-inflammatory interleukins were increased after long-term treatment as indicated by increase in gray scake intensity (bottom panel oof FIG. 14G). The observed protein concentration changes after 6-12 months were consistent across study participants (FIG. 14H).

As shown in this study, SOMAscan values were strongly correlated to absolute measurements of CK and TNNI2. CK, TNNI2, and a broader set of muscle injury proteins were elevated in pre-dose baseline and decreased soon after initiation of treatment and remained markedly decreased with long-term treatment. The short-term proteomics of Compound 1 treatment was dominated by skeletal muscle proteins that reflect muscle damage. Additionally, long-term treatment also exhibited a consistent shift in proteins associated with inflammation, wherein pro-inflammatory factors were reduced while anti-inflammatory cytokines were increased. Although this study varied both time and exposure, these proteins were expected to require longer time spans to see significant change and could reflect the normalization of muscle upon sustained reduction of muscle damage. Proteomic profiles of treatment with Compound 1 showed a rapid and sustained decrease in marker of muscle injury with a longer-term change in the inflammatory milieu, and the proteomic profile was shifted toward that of healthy individuals.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### THE FOLLOWING ARE ADDITIONAL ASPECTS OF THE PRESENT INVENTION:

1. A method of treating a neuromuscular condition, comprising administering to a subject in need thereof a dose of 1 mg to 40 mg per day of a compound of Formula (I): 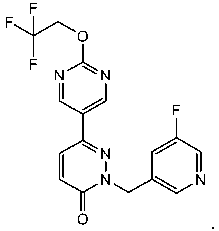
2. The method of aspect 1, comprising administering to the subject a dose of 15 mg to 40 mg per day of the compound of Formula (I).
3. The method of aspect 2, comprising administering to the subject a dose of 15 mg, 20 mg, 25 mg, or 30 mg per day of the compound of Formula (I).
4. The method of aspect 3, comprising administering to the subject a dose of 20 mg per day of the compound of Formula (I).
5. The method of aspect 1, comprising administering to the subject a dose of 1 mg to 20 mg per day of the compound of Formula (I).
6. The method of aspect 5, comprising administering to the subject a dose of 1 mg, 2 mg, 2.5 mg, 5 mg, 7.5 mg, 10 mg, 12.5 mg, 15 mg, or 20 mg per day of the compound of Formula (I).
7. The method of aspect 6, comprising administering to the subject a dose of 1 mg per day of the compound of Formula (I).
8. The method of aspect 6, comprising administering to the subject a dose of 2 mg per day of the compound of Formula (I).
9. The method of aspect 6, comprising administering to the subject a dose of 2.5 mg per day of the compound of Formula (I).
10. The method of aspect 6, comprising administering to the subject a dose of 5 mg per day of the compound of Formula (I).
11. The method of aspect 6, comprising administering to the subject a dose of 7.5 mg per day of the compound of Formula (I).
12. The method of aspect 6, comprising administering to the subject a dose of 10 mg per day of the compound of Formula (I).
13. The method of aspect 6, comprising administering to the subject a dose of 15 mg per day of the compound of Formula (I).
14. The method of aspect 6, comprising administering to the subject a dose of 20 mg per day of the compound of Formula (I).
15. The method of any one of aspects 1 to 14, wherein the subject is over 18 years old.
16. The method of aspect 15, comprising administering to the subject a dose of 10 mg per day of the compound of Formula (I).
17. The method of aspect 15, comprising administering to the subject a dose of 15 mg per day of the compound of Formula (I).
18. The method of aspect 15, comprising administering to the subject a dose of 20 mg per day of the compound of Formula (I).
19. The method of any one of aspects 1 to 7, wherein the subject is 18 years or younger.
20. The method of aspect 19, comprising administering to the subject a dose of 10 mg per day of the compound of Formula (I).
21. The method of aspect 19, comprising administering to the subject a dose of 7.5 mg per day of the compound of Formula (I).
22. The method of aspect 19, comprising administering to the subject a dose of 5 mg per day of the compound of Formula (I).
23. The method of aspect 19, comprising administering to the subject a dose of 2.5 mg per day of the compound of Formula (I).
24. The method of aspect 19, comprising administering to the subject a dose of 2 mg per day of the compound of Formula (I).
25. The method of aspect 19, comprising administering to the subject a dose of 1 mg per day of the compound of Formula (I).
26. The method of any one of aspects 1 to 25, wherein said administering comprises administering of a first dose of said muscle fast myosin (Type II) inhibitor and one or more subsequent doses of said muscle fast myosin (Type II) inhibitor, and optionally wherein the one or more subsequent doses are higher than the first dose.
27. The method of any one of aspects 1-26, wherein said administering maintains a North Star Ambulatory Assessment (NSAA) score or increases the NSAA score of the subject relative to a pre-treatment NSAA score.
28. The method of any one of aspects 1-27, wherein said administering maintains or improves one or more of the following functional measures of the subject: max elbow flexion strength relative to a pre-treatment max elbow flexion strength; max knee extension strength relative to a pre-treatment max knee extension strength;10 meter walk/run velocity relative to a pre-treatment 10 meter walk/run velocity; 100 meter timed test velocity relative to a pre-treatment 100 meter timed test velocity; 4-stair climb time velocity relative to a pre-treatment 4-stair climb time velocity; and max-grip strength relative to a pre-treatment max-grip strength value.
29. The method of any one of aspects 1-28, wherein said administering maintains a creatine kinase activity level or reduces the creatine kinase activity level of the subject relative to a pre-treatment creatine kinase activity level by 10% or more.
30. The method of any one of aspects 1-28, wherein said administering maintains a creatine kinase activity level or increases the creatine kinase activity level of the subject relative to a pre-treatment creatine kinase activity level by 10% or more.
31. The method of any one of aspects 1-30, wherein said administering maintains a Dual Energy x-Ray absorptiometry (DXA) % lean mass of the subject or increase the DXA % lean mass of the subject relative to a pre-treatment DXA % lean mass by 5% or more.
32. The method of any one of aspects 1-31, wherein said administering maintains a Fast Skeletal Muscle Troponin I (TNNI2) concentration of the subject or reduce the TNNI2 concentration of the subject relative to a pre-treatment TNNI2 concentration by 10% or more, and optionally wherein the TNNI2 concentration is inferred from a SOMAscan level.
33. The method of any one of aspects 1-32, wherein said administering decreases protein concentration of one or more muscle injury biomarkers in the subject relative to a pre-treatment protein concentration of the one or more muscle injury biomarkers by 10% or more.
34. The method of aspect 33, wherein the one or more muscle injury biomarkers comprise ACTN2, MYOM2, MYBPC1, PDLIM3, MYL3, TNNI2, MYL11, CKB, CKM, APOBEC2, ENO3, CA3, KLHL41, ADSS1, CAPN3, HSPB6, TNNT2, MYBPC2, GPD1(a), MUSTN1, FBP2, DUSP29, MYBPH, GPD1(b), THAP4, CHCD10, or a combination thereof.
35. The method of any one of aspects 1-34, wherein said administering decreases protein concentration of one or more pro-inflammatory proteins in the subject relative to a pre-treatment protein concentration of the one or more pro-inflammatory proteins by 10% or more.
36. The method of aspect 35, wherein the one or more pro-inflammatory proteins comprise CCL22, CCL5, CXCL10, FGG, IFN-γ, IL3, PPBP, PF4, or a combination thereof.
37. The method of any one of aspects 1-34, wherein said administering increases protein concentration of one or more anti-inflammatory proteins in the subject relative to a pre-treatment protein concentration of the one or more anti-inflammatory proteins by 10% or more.
38. The method of aspect 37, wherein the one or more anti-inflammatory proteins comprise IL10, IL13, IL22, IL37, IL4, or a combination thereof.
39. The method of any of aspects 1 to 38, wherein the neuromuscular condition is selected from Becker Muscular Dystrophy, Duchenne Muscular Dystrophy, Limb-Girdle Muscular Dystrophy and McArdle Disease.
40. The method of aspect 39, wherein the neuromuscular condition is Becker Muscular Dystrophy.
41. The method of aspect 39, wherein the neuromuscular condition is Duchenne Muscular Dystrophy.
42. The method of any one of aspects 1 to 41, wherein the administering comprises administering the compound of Formula (I) for 2 months or more, 3 months or more, 4 months or more, 5 months or more, 6 months or more, or 7 months or more.
43. The method of aspect 42, wherein the administering comprises administering the compound of Formula (I) for 4 months or more, 5 months or more, 6 months or more, or 7 months or more.
44. The method of aspect 42, wherein the administering comprises administering the compound of Formula (I) for 6 months or more.
45. The method of any one of aspects 1-44, wherein prior to said administering the method further comprises selecting a subject for treatment with one or more of characteristics (a)-(g):
   (a) a creatine kinase activity level is 1000 U/L or greater, 1200 U/L or greater, 1300 U/L or greater;
   (b) a TNNI2 concentration of 20 ng/mL or greater, 30 ng/mL or great or 40 ng/mL or greater;
   (c) a dystrophin gene mutation;
   (d) a pre-treatment NSAA yearly change of -0.1 to -10, or -0.5 to -3;
   (e) a pre-treatment serum creatinine of 1 mg/dL or less, 0.8 mg/dL or less, 0.6 mg/dL or less;
   (f) a DXA % lean mass of <75%, less than 70%, less than 65%, less than 60%; and
   (g) a self-reported pain score associated with muscular dystrophy of 1.5 or greater, or 2 or greater.

## Claims

1. A muscle fast myosin (Type II) inhibitor for use in a therapeutic method of inhibiting muscle fast myosin (Type II) in a subject in need thereof, the method comprising selecting a subject with one or more of characteristics (a)-(g):
(a) a creatine kinase activity level is 1000 U/L or greater, 1200 U/L or greater, or 1300 U/L or greater;
(b) a TNNI2 concentration of 20 ng/mL or greater, 30 ng/mL or greater, or 40 ng/mL or greater;
(c) a dystrophin gene mutation;
(d) a pre-treatment NSAA yearly change of -0.1 to -10, -0.5 to -3, or -1 to -4;
(e) a pre-treatment serum creatinine of 1 mg/dL or less, 0.8 mg/dL or less, or 0.6 mg/dL or less;
(f) a DXA % lean mass of <75%, less than 70%, less than 65%, less than 60%; and
(g) a self-reported pain score associated with muscular dystrophy of 1.5 or greater, or 2 or greater; and
administering the muscle fast myosin (Type II) inhibitor to the subject.

2. The muscle fast myosin (Type II) inhibitor for use of claim 1, wherein the dystrophin gene mutation is selected from del 45-48, del 45-47, del 48, del x-51, del 45-55, and del 48-49.

3. The muscle fast myosin (Type II) inhibitor for use of claim 1 or 2, wherein the muscle fast myosin (Type II) inhibitor is a compound of Formula (I):

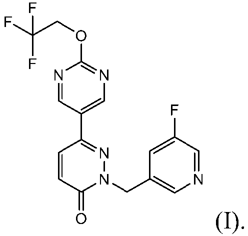

4. The muscle fast myosin (Type II) inhibitor for use of any one of claims 1 to 3, wherein administering comprises administering the muscle fast myosin (Type II) inhibitor once a day, twice a day, three times a day, once every two days, once every three days, once every four days, once every five days, once every six days or once a week.

5. The muscle fast myosin (Type II) inhibitor for use of claim 4, wherein administering comprises administering the muscle fast myosin (Type II) inhibitor once a day.

6. The muscle fast myosin (Type II) inhibitor for use of any one of claims 3 to 5, wherein the administering comprises administering to the subject a dose of 1 mg to 40 mg per day of the compound of Formula (I).

7. The muscle fast myosin (Type II) inhibitor for use of any one of claims 3 to 6, wherein the administering comprises administering to the subject in need thereof a dose of 1 mg to 20 mg per day of the compound of Formula (I); optionally
wherein the administering comprises administering to the subject a dose of 1 mg, 2 mg, 2.5 mg, 5 mg, 7.5 mg, 10 mg, 12.5 mg, 15 mg, or 20 mg per day of the compound of Formula (I).

8. The muscle fast myosin (Type II) inhibitor for use of any one of claims 3 to 7, wherein the administering comprises administering to the subject a dose of 5 mg per day of the compound of Formula (I).

9. The muscle fast myosin (Type II) inhibitor for use of any one of claims 3 to 7, wherein the administering comprises administering to the subject a dose of 10 mg per day of the compound of Formula (I).

10. The muscle fast myosin (Type II) inhibitor for use of any one of claims 3 to 7, wherein the administering comprises administering to the subject a dose of 15 mg per day of the compound of Formula (I).

11. The muscle fast myosin (Type II) inhibitor for use of any one of claims 1 to 10, wherein the method is for treating a neuromuscular condition, and the neuromuscular condition is selected from Becker Muscular Dystrophy, Duchenne Muscular Dystrophy, Limb-Girdle Muscular Dystrophy and McArdle Disease.

12. The muscle fast myosin (Type II) inhibitor for use of claim 11, wherein the neuromuscular condition is Becker Muscular Dystrophy.

13. The muscle fast myosin (Type II) inhibitor for use of claim 11, wherein the neuromuscular condition is Duchenne Muscular Dystrophy.

14. The muscle fast myosin (Type II) inhibitor for use of any one of claims 1 to 13, wherein the subject is 18 years or younger.

15. The muscle fast myosin (Type II) inhibitor for use of any one of claims 1 to 14, wherein the administering does not negatively impact the cardiac function of the subject.
